# EUROPEAN PATENT APPLICATION

(11) **EP 4 043 452 A1**
(43) Date of publication of application: **17.08.2022**
(21) Application number: 21190665.6
(22) Date of filing: 10.08.2021
(51) Int. Cl.: C07D 401/12, C07D 401/14, A61K 31/4709, A61P 35/00

(54) **BIVALENT FIBROBLAST ACTIVATION PROTEIN LIGANDS FOR TARGETED DELIVERY APPLICATIONS**

(30) Priority: 12.02.2021 WO PCT/EP2021/053494
(71) Applicant: Philochem AG, 8112 Otelfingen (CH)
(72) Inventor: CAZZAMALLI, Samuele, 8112 Otelfingen (CH); GALBIATI, Andrea, 8112 Otelfingen (CH); MILLUL, Jacopo, 8112 Otelfingen (CH); ZANA, Aureliano, 8112 Otelfingen (CH)
(74) Representative: Hoffmann Eitle

(57) **Abstract**

The present invention relates to ligands of Fibroblast Activation Protein (FAP) for the active delivery of various payloads (e.g. cytotoxic drugs, radionuclides, fluorophores, proteins and immunomodulators) at the site of disease. In particular, the present invention relates to the development of bivalent FAP ligands for targeting applications, in particular diagnostic methods and/or methods for therapy or surgery in relation to a disease or disorder, such as cancer, inflammation or another disease characterized by overexpression of FAP.

## Description

### INTRODUCTION

### Field

The present invention relates to ligands of Fibroblast Activation Protein (FAP) for the active delivery of various payloads (e.g. cytotoxic drugs, radionuclides, fluorophores, proteins and immunomodulators) at the site of disease. In particular, the present invention relates to the development of bivalent FAP ligands for targeting applications, in particular diagnostic methods and/or methods for therapy or surgery in relation to a disease or disorder, such as cancer, inflammation or another disease characterized by overexpression of FAP.

### Background of the Invention

Chemotherapy is still widely applied for the treatment of cancer patients and of other diseases. Conventional anti-cancer chemotherapeutic agents act on basic mechanisms of cell survival and cannot distinguish between healthy cells and malignant cells. Moreover, those drugs do not accumulate efficiently to the site of the disease upon systemic administration. Unspecific mechanism of actions and inefficient localization at the tumour site account for unsustainable side-effects and poor therapeutic efficacy of conventional chemotherapy.

The development of targeted drugs, able to selectively localize at the site of the disease after systemic administration, is highly desirable. A strategy to generate such drugs is represented by the chemical conjugation of a therapeutic payload, like cytotoxic drugs or radionuclides, to a ligand specific to a marker of a disease. Disease-specific monoclonal antibodies, peptides and small ligands have been considered as ligands of choice for the development of targeted drug products. The use of small ligands for targeting applications has several advantages compared to bigger molecules like peptides and antibodies: more rapid and efficient tumour penetration, lower immunogenicity and lower manufacturing costs.

Small organic ligands specific to prostate-specific membrane antigen, folate receptor and carbonic anhydrase IX have shown excellent biodistribution profiles in preclinical models of cancer and in patients. These ligands have been conjugated to cytotoxic drugs and to radionuclides to generate small molecule-drug conjugate and small molecule-radio conjugate products (SMDCs and SMRCs) for the treatment of cancer. 177-Lutetium-PSMA-617 represents an example of a late stage SMRC which is now being investigated in a phase III trial for the treatment of metastatic castrate-resistant prostate cancer (mCRPC) patients (VISION trial).

Fibroblast activation protein (FAP) is a membrane-bound gelatinase which promotes tumour growth and progression and is overexpressed in cancer-associated fibroblasts. FAP represents an ideal target for the development of targeted SMDCs and SMRCs due to its low expression in normal organs.

WO2019154886 and WO2019154859 describe heterocyclic compounds as fibroblast activation protein-alpha inhibitors used to treat different cancer types. WO2019118932 describes substituted N-containing cyclic compounds as fibroblast activation protein alpha inhibitors used to treat different pathological conditions. WO2019083990 describes imaging and radiotherapeutic targeting fibroblast-activation protein-alpha (FAP-alpha) compounds as FAP-alpha inhibitors used for imaging disease associated with FAP-alpha and to treat proliferative diseases, and notes that the 4-isoquinolinoyl and 8-quinolinoyl derivatives described therein are characterized by very low FAP-affinity. WO2013107820 describes substituted pyrrolidine derivatives used in the treatment of proliferative disorders such as cancers and diseases indicated by tissue remodelling or chronic inflammation such as osteoarthritis. WO2005087235 describes pyrrolidine derivatives as dipeptidyl peptidase IV inhibitors to treat Type II diabetes. WO2018111989 describes conjugates comprising fibroblast activation protein (FAP) inhibitor, bivalent linker and e.g. near infrared (NIR) dye, useful for removing cancer-associated fibroblasts, imaging population of cells in vitro, and treating cancer. Tsutsumi et al. (J Med Chem 1994) describe the preparation and in vitro prolyl endopeptidase (PEP) inhibitory activity of a series of a-keto heterocyclic compounds. Hu et al. (Bioorg Med Chem Lett 2005) describe the structure-activity relationship of various N-alkyl Gly-boro-Pro derivatives against FAP and other two dipeptidyl peptidases. Edosada et al. (J Biol Chem 2006) describe the dipeptide substrate specificity of FAP and the development of a Ac-Gly-BoroPro FAP-selective inhibitor. Gilmore et al. (Biochem Biophys Res Commun 2006) describe the design, synthesis, and kinetic testing of a series of dipeptide proline diphenyl phosphonates, against DPP-IV and FAP. Tran et al. (Bioorg Med Chem Lett 2007) describe the structure-activity relationship of various N-acyl-Gly-, N-acyl-Sar-, and N-blocked-boroPro derivatives against FAP. Tsai et al. (J Med Chem 2010) describe structure-activity relationship studies that resulted in a number of FAP inhibitors with excellent selectivity over DPP-IV, DPP-II, DPP8, and DPP9. Ryabtsova et al. (Bioorg Med Chem Lett 2012) describe the synthesis and the evaluation of FAP inhibition properties of a series of N-acylated glycyl-(2-cyano)pyrrolidines. Poplawski et al. (J Med Chem 2013) describe N-(pyridine-4-carbonyl)-D-Ala-boroPro as a potent and selective FAP inhibitor. Jansen et al. (ACS Med Chem Lett 2013) describe FAP inhibitors based on the N-(4-quinolinoyl)-Gly-(2-cyanopyrrolidine) scaffold. Jansen et al. (Med Chem Commun 2014) the structure-activity relationship of FAP inhibitors based on the linagliptin scaffold. Jansen et al. (Med Chem Commun 2014) describe xanthine-based FAP inhibitors with low micromolar potency. Jansen et al. (J Med Chem 2014) describe the structure-activity relationship of FAP inhibitors based on the N-4-quinolinoyl-Gly-(2S)-cyanoPro scaffold. Jackson et al. (Neoplasia 2015) describe the development of a pseudopeptide inhibitor of FAP. Meletta et al. (Molecules 2015) describe the use of a boronic-acid based FAP inhibitor as non-invasive imaging tracers of atherosclerotic plaques. Dvořáková et al. (J Med Chem 2017) describe the preparation of a polymer conjugate containing a FAP-specific inhibitor for targeting applications. Loktev et al. (J Nucl Med 2018) describe the development of an iodinated and a DOTA-coupled radiotracer based on a FAP-specific enzyme inhibitor. Lindner et al. (J Nucl Med 2018) describe the modification and optimization of FAP inhibitors for use as theranostic tracers. Giesel et al. (J Nucl Med 2019) describe the clinical imaging performance of quinoline-based PET tracers that act as FAP inhibitors.

### PROBLEMS TO BE SOLVED BY THE INVENTION

The present invention aims at the problem of providing improved binders (ligands) of fibroblast activation protein (FAP) suitable for targeting applications. The binders should be suitable for inhibition of FAP and/or targeted delivery of a payload, such as a therapeutic or diagnostic agent, to a site afflicted by or at risk of disease or disorder characterized by overexpression of FAP. Preferably, the binder should provide a superior therapeutic index in terms of tumour to non-tumour (T/NT) ratio when administered in vivo, and/or be obtainable by an efficient synthetic route.

### SUMMARY OF THE INVENTION

The present inventors have found novel bivalent organic ligands of fibroblast activation protein (FAP) ("Bi-ESV6") suitable for targeting applications. The compounds according to the present invention (also referred to as ligands or binders) comprise two small binding moieties A having the following structure:

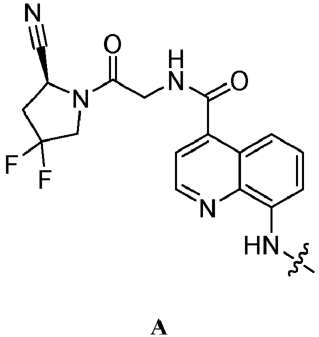

A compound according to the present invention may be represented by following general Formula I, 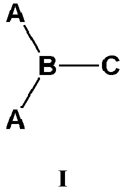 its individual diastereoisomers, its hydrates, its solvates, its crystal forms, its individual tautomers or a pharmaceutically acceptable salt thereof, wherein **A** is a binding moiety; **B** is a covalent bond or a moiety comprising a chain of atoms that covalently attaches the moieties **A** and **C;** and **C** is a payload moiety.

The present invention further provides a pharmaceutical composition comprising said compound and a pharmaceutically acceptable excipient.

The present invention further provides said compound or pharmaceutical composition for use in a method for treatment of the human or animal body by surgery or therapy or a diagnostic method practised on the human or animal body; as well as a method for treatment of the human or animal body by surgery or therapy or a diagnostic method practised on the human or animal body comprising administering a therapeutically or diagnostically effective amount of said compound or pharmaceutical composition to a subject in need thereof.

The present invention further provides said compound or pharmaceutical composition for use in a method for therapy or prophylaxis of a subject suffering from or having risk for a disease or disorder; as well as a method for treatment therapy or prophylaxis of a disease or disorder comprising administering a therapeutically or diagnostically effective amount of said compound or pharmaceutical composition to a subject suffering from or having risk for said disease or disorder.

The present invention further provides said compound or pharmaceutical composition for use in a method for guided surgery practised on a subject suffering from or having risk for a disease or disorder; as well as a method for guided surgery comprising administering a therapeutically or diagnostically effective amount of said compound or pharmaceutical composition to a subject suffering from or having risk for a disease or disorder.

The present invention further provides said compound or pharmaceutical composition for use in a method for diagnosis of a disease or disorder, the method being practised on the human or animal body and involving a nuclear medicine imaging technique, such as Positron Emission Tomography (PET); as well as a method for diagnosis of a disease or disorder, the method being practised on the human or animal body and involving a nuclear medicine imaging technique, such as Positron Emission Tomography (PET), and comprising administering a therapeutically or diagnostically effective amount of said compound or pharmaceutical composition to a subject in need thereof.

The present invention further provides said compound or pharmaceutical composition for use in a method for targeted delivery of a therapeutic or diagnostic agent to a subject suffering from or having risk for a disease or disorder; as well as a method for targeted delivery of a therapeutically or diagnostically effective amount of said compound or pharmaceutical composition to a subject suffering from or having risk for a disease or disorder.

Preferably, the aforementioned disease or disorder is characterized by overexpression of FAP and is independently selected from cancer, inflammation, atherosclerosis, fibrosis, tissue remodelling and keloid disorder, preferably wherein the cancer is selected from the group consisting of breast cancer, pancreatic cancer, small intestine cancer, colon cancer, multi-drug resistant colon cancer, rectal cancer, colorectal cancer, metastatic colorectal cancer, lung cancer, non-small cell lung cancer, head and neck cancer, ovarian cancer, hepatocellular cancer, oesophageal cancer, hypopharynx cancer, nasopharynx cancer, larynx cancer, myeloma cells, bladder cancer, cholangiocarcinoma, clear cell renal carcinoma, neuroendocrine tumour, oncogenic osteomalacia, sarcoma, CUP (carcinoma of unknown primary), thymus cancer, desmoid tumours, glioma, astrocytoma, cervix cancer, skin cancer, kidney cancer and prostate cancer. More preferably, the disease or disorder is selected from melanoma and renal cell carcinoma.

### BRIEF DESCRIPTION OF THE DRAWINGS

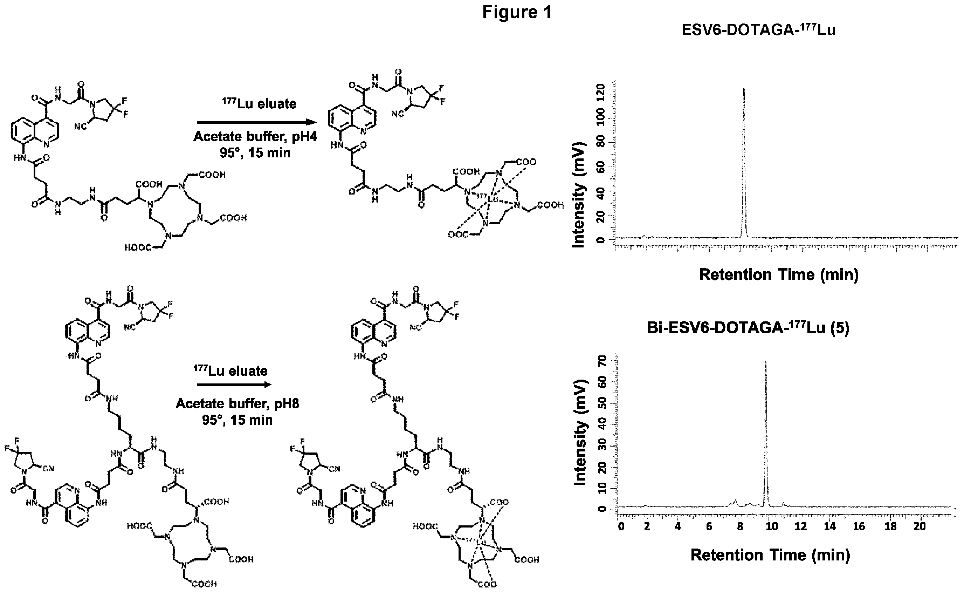

**Figure 1****:** HPLC profiles of ¹⁷⁷Lutetium-labeled preparations of ESV6-DOTAGA and Bi-ESV6-DOTAGA indicate a high degree of purity of the radioconjugate.

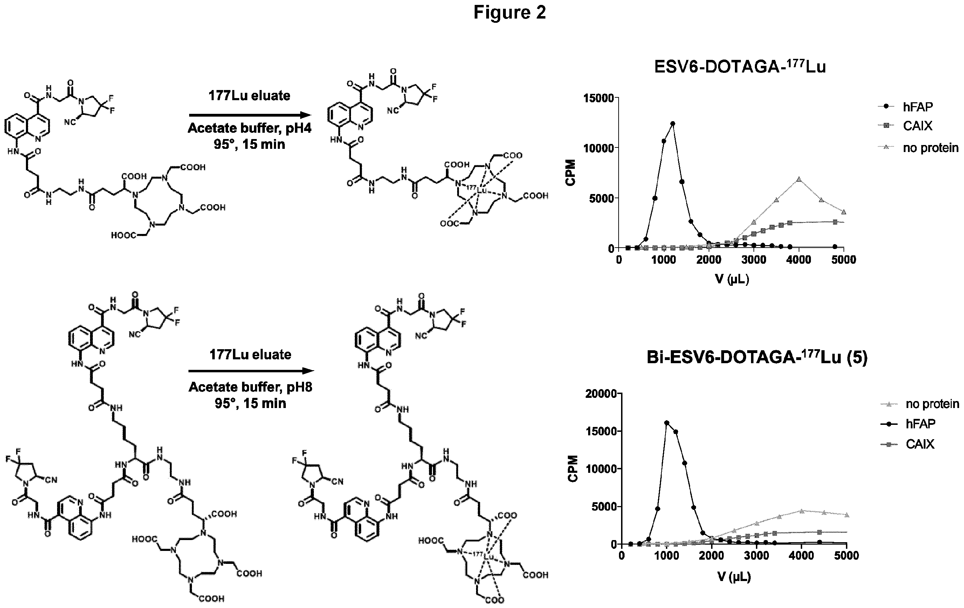

**Figure 2****:** Co-elution experiments performed with ¹⁷⁷Lu-ESV6-DOTAGA and ¹⁷⁷Lu-Bi-ESV6-DOTAGA on hFAP, hCAIX and without protein. Both compounds form a stable complex with hFAP and were eluted in the first 2 mL, as expected. When the compounds were incubated with the irrelevant protein CAIX or without any protein, the peak of radioactivity was detected after more than 3000 µL of elutate. ¹⁷⁷Lu -ESV6-DOTAGA and ¹⁷⁷Lu-Bi-ESV6-DOTAGA form a stable complex with recombinant human FAP.

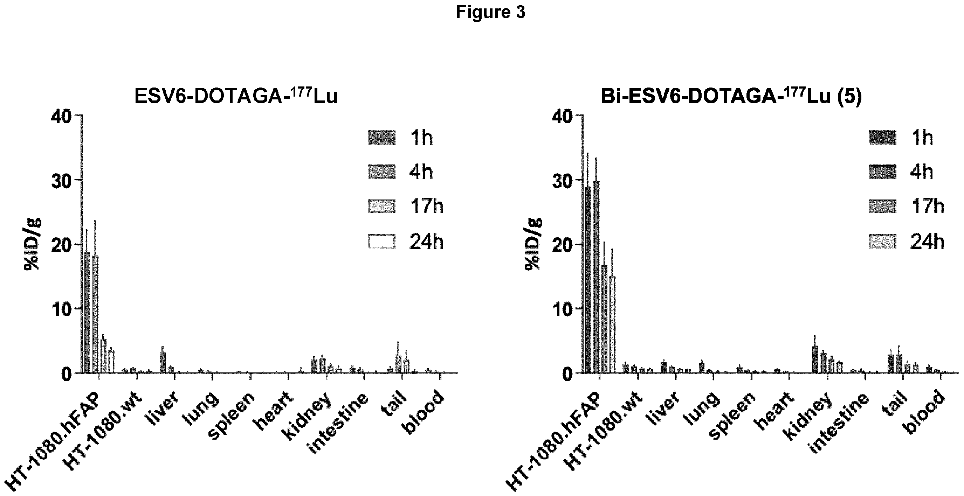

**Figure 3****:** Injected dose per gram of tissue (ID%/g) at 1h, 4h, 17h and 24h indicate a very high uptake in FAP-expressing tumour in mice treated with ¹⁷⁷Lu-Bi-ESV6-DOTAGA and a high uptake in mice treated with ¹⁷⁷Lu-ESV6-DOTAGA. Negligible uptake in non FAP-expressing tumour (HT-1080.wt) is registered for both radio-conjugates indicating their high degree of specificity for FAP. Negligible uptake in normal organs is registered for both radio-conjugates indicating their high degree of tolerability. The kidney uptake for ¹⁷⁷Lu-Bi-ESV6-DOTAGA is transient and becomes negligible 24 hours after injection.

### DETAILED DESCRIPTION OF THE INVENTION

The present inventors have identified small molecule binders of fibroblast activation protein (FAP) which are suitable for targeting applications. The binders according to the invention provide high inhibition of FAP, high affinity for FAP and/or are suitable for targeted delivery of a payload, such as a therapeutic or diagnostic agent, to a site afflicted by or at risk of disease or disorder characterized by overexpression of FAP. The binders according to the present invention form a stable complex with FAP, display an increased affinity, increased inhibitory activity, a slower rate of dissociation from the complex, and/or prolonged residence at a disease site. The binders according to the invention further can have an increased tumour-to-liver, tumour-to-kidney and/or tumour-to-intestine uptake ratio; a more potent anti-tumour effect (e.g., measured by mean tumour volume increase), and/or lower toxicity (e.g., as assessed by the evaluation of changes (%) in body weight).

In particular, binders according to the invention surprisingly can exhibit a very high, specific uptake in FAP-expressing tumours in combination with low uptake in normal organs. That is, the binders can provide advantageous therapeutic index in terms of tumour to non-tumour (T/NT) ratio when administered in vivo.

The binders according to the invention further can have a high or improved affinity for human and murine fibroblast activation protein and/or cross-reactivity to the murine antigen. The binders according to the invention preferably attain FAP-specific cellular binding; FAP-selective accumulation on the cell membrane; FAP-selective accumulation inside the cytosol. The binders according to the invention can further preferably, rapidly and homogeneously localize at the tumour site in vivo with a high tumour-to-organs selectivity, in particular for melanoma and/or renal cell carcinoma. Binders according to the invention comprising a radioactive payload (e.g., ¹⁷⁷Lu) preferably attain dose-dependent response, with target saturation reached between 250 nmol/Kg and 500 nmol/Kg reached and/or maintained at up to 12 h, more preferably 1 to 9 h, further more preferably 3 to 6 h after intravenous administration.

Additionally, binders according to the invention can be advantageously obtained by efficient synthetic routes as described herein.

As explained above, the present invention provides a compound, its individual diastereoisomers, its hydrates, its solvates, its crystal forms, its individual tautomers or a pharmaceutically acceptable salt thereof, wherein the compound comprises two moieties **A,** each having the following structure:

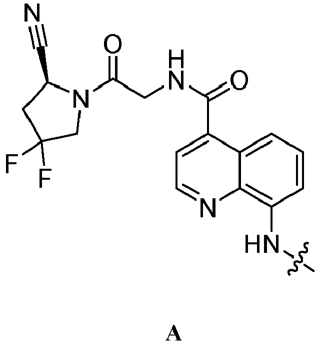

A compound according to the present invention may be represented by Formula **I**:

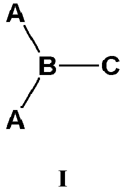

Therein, **B** is a covalent bond or a moiety comprising a chain of atoms covalently attaching moieties **A** to **C;** and **C** may be an atom, a molecule or a particle, and/or is a therapeutic or diagnostic agent.

### Moiety A

Without wishing to be bound by any theory, it is contemplated that these surprising technical effects are associated with the particular structure of the small binding moieties **A** wherein the quinoline ring is substituted at the 8-position by a nitrogen-containing group, such as an amino or amido group:

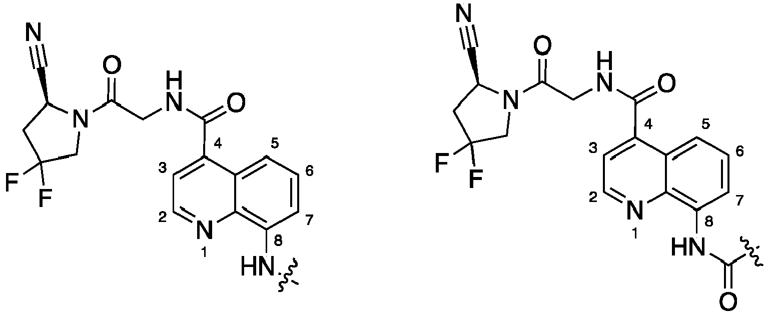

It has been previously shown that the higher target protein affinity of a compound results in longer tumour residence *in vivo* (Wichert et al., Nature Chemistry 7, 241-249 (2015**)).** The compounds of the present invention have an increased affinity, slower dissociation rate with respect to FAP as compared to prior art compounds, and therefore are also considered to as having a prolonged residence at the disease site at a therapeutically or diagnostically relevant level, preferably beyond 1 h, more preferably beyond 6 h post injection. Preferably, the highest enrichment is achieved after 5 min, 10 min, 20 min, 30 min, 45 min, 1 h, 2 h, 3 h, 4 h, 5 h or 6 h; and/or enrichment in the disease site is maintained at a therapeutically or diagnostically relevant level, over a period of or at least for 5 min, 10 min, 20 min, 30 min, 45 min, 1 h, 2 h, 3 h, 4 h, 5 h or 6 h, more preferably beyond 6 h post injection.

Preferably, each binding moiety **A** has the following structure **A¹**; more preferably the following structure **A²,** wherein m is 0, 1, 2, 3, 4 or 5, preferably 1:

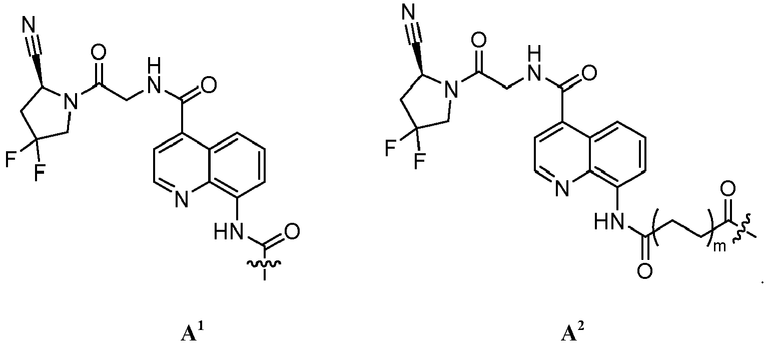

### Moiety B

Moiety **B** is a covalent bond or a moiety comprising a chain of atoms that covalently attaches **A** to the payload **C**, *e.g.,* through one or more covalent bond(s). The moiety **B** may be cleavable or non-cleavable, multifunctional moiety which can be used to link one or more payload and/or binder moieties to form the targeted conjugate of the invention.

Specifically, moiety **B** is a multifunctional moiety linking one or more moieties C and/or moieties **A.** The structure of the compound comprises 2 moieties **A** per molecule. The structure of the compound may comprise more than one moieties **C**, preferably 2, 3, 4, 5, 6, 7, 8, 9 or 10 moieties **C** per molecule. Preferably, the structure of the compound comprises 2 moieties **A** and 1 moiety **C** per molecule.

When cleavable linker units are present within moiety **B,** release mechanisms can be identical to those specific to antibodies linked to cytotoxic payloads. Indeed, the nature of the binding moieties is independent in that respect. Therefore, there is envisaged pH-dependent [Leamon, C.P. et al (2006) Bioconjugate Chem., 17, 1226; Casi, G. et al (2012) J. Am. Chem. Soc., 134, 5887], reductive [Bernardes, G.J. et al (2012) Angew. Chem. Int. Ed. Engl., 51. 941; Yang, J. et al (2006) Proc. Natl. Acad. Sci. USA, 103, 13872] and enzymatic release [Doronina S.O. et al (2008) Bioconjugate Chem, 19, 1960; Sutherland, M.S.K. (2006) J. Biol. Chem, 281, 10540]. In a specific setting, when functional groups are present on either the binding moiety or payloads (e.g. thiols, alcohols) a linkerless connection can be established thus releasing intact payloads, which simplifies substantially pharmacokinetic analysis.

Moiety B can comprise or consist of a unit shown in Table 1 below wherein the substituents R and Rⁿ shown in the formulae may suitably be independently selected from H, halogen, substituted or unsubstituted (hetero)alkyl, (hetero)alkenyl, (hetero)alkynyl, (hetero)aryl, (hetero)arylalkyl, (hetero)cycloalkyl, (hetero)cycloalkylaryl, heterocyclylalkyl, a peptide, an oligosaccharide or a steroid group. Preferably, each of R, R₁, R₂ and R₃ is independently selected from H, OH, SH, NH₂, halogen, cyano, carboxy, alkyl, cycloalkyl, aryl and heteroaryl, each of which is substituted or unsubstituted. Suitably R and Rⁿ are independently selected from H, or C1-C7 alkyl or heteroalkyl. More suitably, R and Rⁿ are independently selected from H, methyl or ethyl.

**Table 1**

| Linker | Structure | Release mechanism |
|---|---|---|
| Amide | 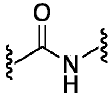 | Proteolysis |
| | 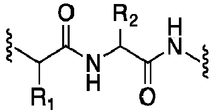 | |
| Ester | 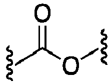 | Hydrolysis |
| Carbamate | 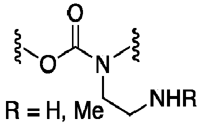 | Hydrolysis |
| Hydrazone | 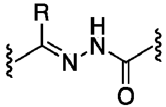 | Hydrolysis |
| Thiazolidine | 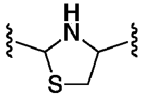 | Hydrolysis |
| Methylene alkoxy carbamate | 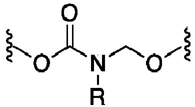 | Hydrolysis |
| Disulfide | 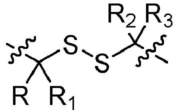 | Reduction |

Moiety **B,** unit(s) **B_{L}** and/or unit(s) **B_{S}** may suitably comprise as a cleavable bond a disulfide linkage since these linkages are stable to hydrolysis, while giving suitable drug release kinetics at the target *in vivo,* and can provide traceless cleavage of drug moieties including a thiol group.

Moiety **B,** unit(s) **B_{L}** and/or unit(s) **B_{S}** may be polar or charged in order to improve water solubility of the conjugate. For example, the linker may comprise from about 1 to about 20, suitably from about 2 to about 10, residues of one or more known water-soluble oligomers such as peptides, oligosaccharides, glycosaminoglycans, polyacrylic acid or salts thereof, polyethylene glycol, polyhydroxyethyl (meth) acrylates, polysulfonates, etc. Suitably, the linker may comprise a polar or charged peptide moiety comprising e.g. from 2 to 10 amino acid residues. Amino acids may refer to any natural or non-natural amino acid. The peptide linker suitably includes a free thiol group, preferably a N-terminal cysteine, for forming the said cleavable disulfide linkage with a thiol group on the drug moiety. Any peptide containing L- or D-aminoacids can be suitable; particularly suitable peptide linkers of this type are Asp-Arg-Asp-Cys and/or Asp-Lys-Asp-Cys.

In these and other embodiments, moiety **B,** unit(s) **B_{L}** and/or unit(s) **B_{S}** may comprise a cleavable or non-cleavable peptide unit that is specifically tailored so that it will be selectively enzymatically cleaved from the drug moiety by one or more proteases on the cell surface or the extracellular regions of the target tissue. The amino acid residue chain length of the peptide unit suitably ranges from that of a single amino acid to about eight amino acid residues. Numerous specific cleavable peptide sequences suitable for use in the present invention can be designed and optimized in their selectivity for enzymatic cleavage by a particular tumour-associated enzyme e.g. a protease. Cleavable peptides for use in the present invention include those which are optimized toward the proteases MMP-1, 2 or 3, or cathepsin B, C or D. Especially suitable are peptides cleavable by Cathepsin B. Cathepsin B is a ubiquitous cysteine protease. It is an intracellular enzyme, except in pathological conditions, such as metastatic tumours or rheumatoid arthritis. An example for a peptide cleavable by Cathepsin B is containing the sequence Val-Cit.

In any of the above embodiments, the moiety **B** and in particular, unit(s) **B_{L}** suitably further comprise(s) self-immolative moiety can or cannot be present after the linker. The self-immolative linkers are also known as electronic cascade linkers. These linkers undergo elimination and fragmentation upon enzymatic cleavage of the peptide to release the drug in active, preferably free form. The conjugate is stable extracellularly in the absence of an enzyme capable of cleaving the linker. However, upon exposure to a suitable enzyme, the linker is cleaved initiating a spontaneous self-immolative reaction resulting in the cleavage of the bond covalently linking the self-immolative moiety to the drug, to thereby effect release of the drug in its underivatized or pharmacologically active form. In these embodiments, the self-immolative linker is coupled to the binding moiety through an enzymatically cleavable peptide sequence that provides a substrate for an enzyme to cleave the amide bond to initiate the self-immolative reaction. Suitably, the drug moiety is connected to the self-immolative moiety of the linker via a chemically reactive functional group pending from the drug such as a primary or secondary amine, hydroxyl, sulfhydryl or carboxyl group.

Examples of self-immolative linkers are PABC or PAB (para-aminobenzyloxycarbonyl), attaching the drug moiety to the binding moiety in the conjugate (Carl et al (1981) J. Med. Chem. 24: 479-480; Chakravarty et al (1983) J. Med. Chem. 26: 638-644). The amide bond linking the carboxy terminus of a peptide unit and the para-aminobenzyl of PAB may be a substrate and cleavable by certain proteases. The aromatic amine becomes electron-donating and initiates an electronic cascade that leads to the expulsion of the leaving group, which releases the free drug after elimination of carbon dioxide (de Groot, et al (2001) Journal of Organic Chemistry 66 (26): 8815-8830). Further self-immolating linkers are described in WO2005/082023.

In yet other embodiments, the linker comprises a glucuronyl group that is cleavable by glucoronidase present on the cell surface or the extracellular region of the target tissue. It has been shown that lysosomal beta-glucuronidase is liberated extracellularly in high local concentrations in necrotic areas in human cancers, and that this provides a route to targeted chemotherapy (Bosslet, K. et al. Cancer Res. 58, 1195-1201 (1998)).

In any of the above embodiments, the moiety B suitably further comprises a spacer unit. A spacer unit can be the unit **B_{S}**, which may be linked to the binding moiety A, for example via an amide, amine or thioether bond. The spacer unit is of a length that enables e.g. the cleavable peptide sequence to be contacted by the cleaving enzyme (e. g. cathepsin B) and suitably also the hydrolysis of the amide bond coupling the cleavable peptide to the self-immolative moiety X. Spacer units may for example comprise a divalent radical such as alkylene, arylene, a heteroarylene, repeating units of alkyloxy (e.g. polyethylenoxy, PEG, polymethyleneoxy) and alkylamino (e.g. polyethyleneamino), or diacid ester and amides including succinate, succinamide, diglycolate, malonate, and caproamide.

In any of the embodiments described therein, ^{∗} represents a point of attachment to moiety **A** or a point of attachment for which the shortest path to moiety **A** comprises less atoms than that for •, as the case may be; and • represents a point of attachment a point of attachment to moiety **C** or a point of attachment to moiety **C** for which the shortest path to moiety **C** comprises less atoms than that for ^{∗}, as the case may be. The same applies also for cases where a reactive moiety **L** is present rather than payload moiety **C.** The following notations and all have the meaning of a point of attachment of a certain group or atom (e.g., R) to a further moiety:

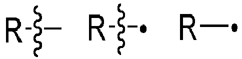

If the structure of relevance is a peptide mono- or oligomer, each ^{∗} represents a point of attachment for which the shortest path to moiety **A** comprises less atoms than that for •; and each • represents a point of attachment for which the shortest path to moiety **C** comprises less atoms than that for ^{∗}, with the proviso that when n is > 1 and a respective point of attachment is indicated on any one of R^{a}, R^{b} and R^{c}, then it can be independently present in one or more of the peptide monomeric units, preferably in one peptide monomeric unit most distant from the other point of attachment indicated in the respective structure.

In any of the embodiments described herein, the terms "peptide", "dipeptide", "tripeptide", "tetrapeptide" etc. refer to peptide mono- or oligomers having a backbone formed by proteinogenic and/or a non-proteinogenic amino acids. As used herein, the terms "aminoacyl" or "aminoacid" generally refer to any proteinogenic or a non-proteinogenic amino acid. Preferably, in any of the embodiments disclosed therein, the side-chain residues of a proteinogenic or a non-proteinogenic amino acid are represented by any of R^{a}, R^{b} and R^{c}, each of which is selected from the following list: 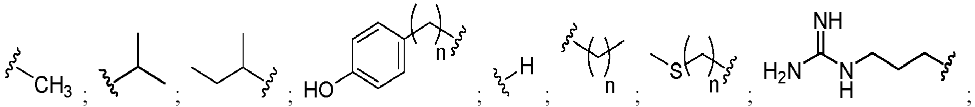 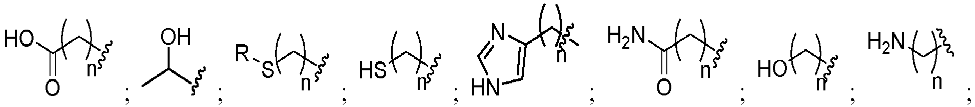 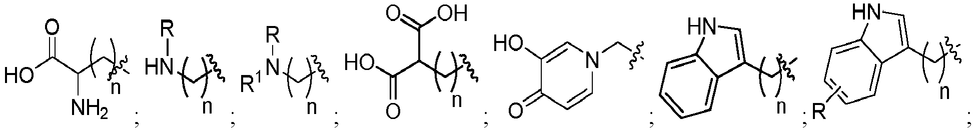 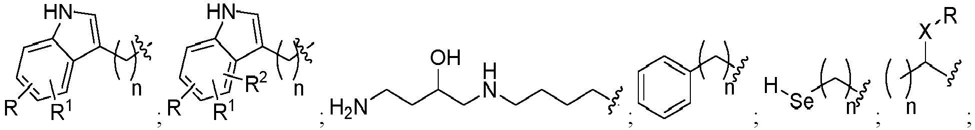 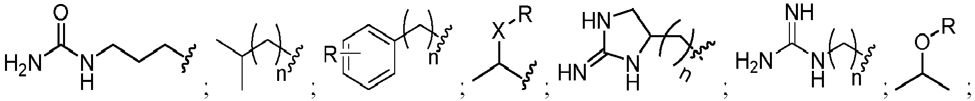 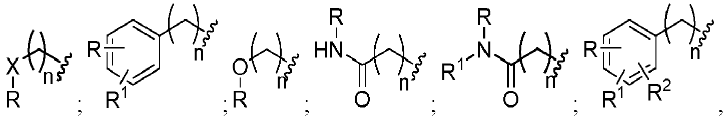
wherein each of R, R¹, R² and R³ is independently selected from H, OH, SH, NH₂, halogen, cyano, carboxy, alkyl, cycloalkyl, aryl and heteroaryl, each of which is substituted or unsubstituted;
each X is independently selected from NH, NR, S, O and CH₂, preferably NH; and
each *n* and *m* is independently an integer preferably selected from 0, 1, 2, 3, 4, 5, 6, 7, 8, 9, 10, 11, 12, 13, 14, 15, 16, 17, 18, 19 and 20.

Preferably, in any of the embodiments disclosed therein, side-chain residues of a proteinogenic or a non-proteinogenic amino acid are represented by any of R^{a}, R^{b} and R^{c},
each of which may be part of a 3-, 4-, 5-, 6- or 7-membered ring. For instance, the side chain alpha, beta and/or gamma position of said proteinogenic or non-proteinogenic amino acid can be part of a cyclic structure selected from an azetidine ring, pyrrolidine ring and a piperidine ring, such as in the following aminoacids (proline and hydroxyproline): 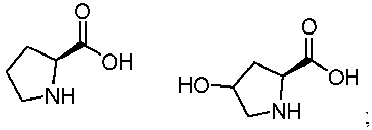 or
each of which may independently be part of an unsaturated structure (i.e. wherein the H atom geminal to the respective group R^{a}, R^{b} and R^{c} is absent), e.g.:

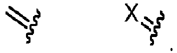

As used herein, the following notation of peptide sequences refers to a sequence from N to C terminus, and attachment of group through a horizontal bond (here: moiety C) means covalent attachment to the peptide backbone via amide bond to the respective terminal amino acid (here: AA₃):

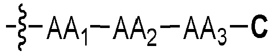

As used herein, the following notation of peptide sequences refers to a sequence from N to C terminus, and attachment of group through a vertical bond (here: moiety C) means covalent attachment via the sidechain of the respective amino acid (here: AA₃):

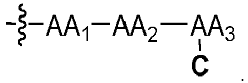

Further preferable non-proteinogenic amino acids can be selected from the following list:

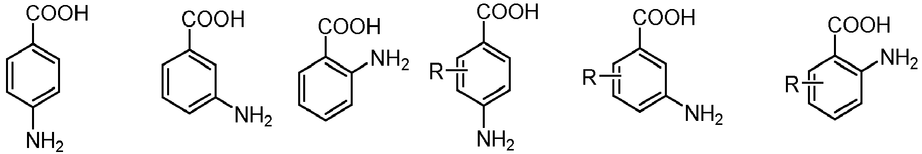

Particularly preferred embodiments for the moiety **B** as well as the compound according to the present invention are shown in the appended claims.

Preferably, **B** is represented by any of the following general Formulae **II―V**, wherein: 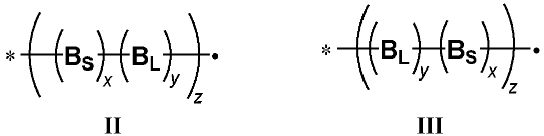 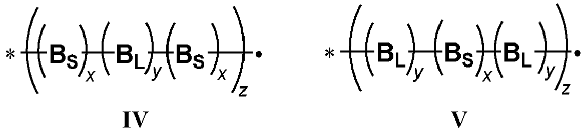
each *x* is an integer independently selected from the range of 0 to 100, preferably 0 to 50, more preferably 0 to 30, yet more preferably selected from 0, 1, 2, 3, 4, 5, 6, 7, 8, 9, 10, 11, 12, 13, 14, 15, 16, 17, 18, 19 and 20;
each *y* is an integer independently selected from the range of 0 to 30, preferably selected from 0, 1, 2, 3, 4, 5, 6, 7, 8, 9, 10, 11, 12, 13, 14, 15, 16, 17, 18, 19 and 20;
each z is an integer independently selected from the range of 0 to 5, preferably selected from selected from 0, 1, 2, 3 and 4;
**B** is a multifunctional moiety linking moiety **C** and the two moieties **A;**
^{∗} represents a point of attachment to a moiety **A;** and
• represents a point of attachment to moiety **C.**

More preferably, the compound comprises moiety **B** represented by any of the following general Formulae **IIa―Va**: 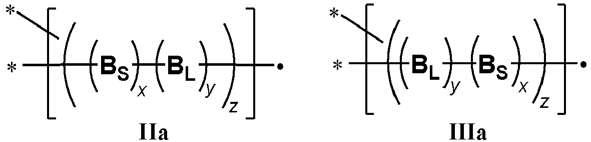 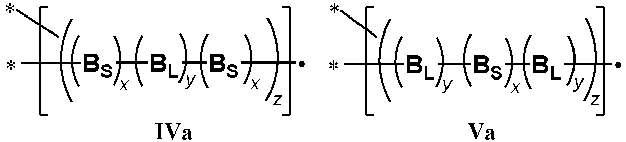
wherein *x, y* and z are as previously defined;
each ^{∗} represents a point of attachment to a moiety **A;** and
• represents a point of attachment to moiety **C.**

**B_{S}** and/or **B_{L}** can be a group comprising or consisting of a structural unit independently selected from the group consisting of alkylene, cycloalkylene, arylalkylene, heteroarylalkylene, heteroalkylene, heterocycloalkylene, alkenylene, cycloalkenylene, arylalkenylene, heteroarylalkenylene, heteroalkenylene, heterocycloalenkylene, alkynylene, heteroalkynylene, arylene, heteroarylene, aminoacyl, oxyalkylene, aminoalkylene, diacid ester, dialkylsiloxane, amide, thioamide, thioether, thioester, ester, carbamate, hydrazone, thiazolidine, methylene alkoxy carbamate, disulfide, vinylene, imine, imidamide, phosphoramide, saccharide, phosphate ester, phosphoramide, carbamate, dipeptide, tripeptide, tetrapeptide, each of which is substituted or unsubstituted.

**B_{S}** and/or **B_{L}** can be a group comprising or consisting of a structural unit independently selected from the group consisting of: 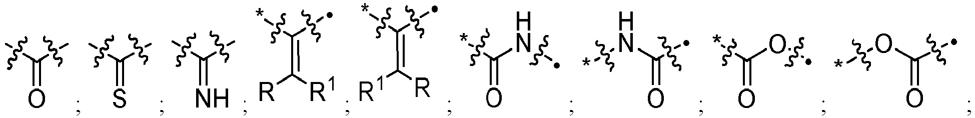 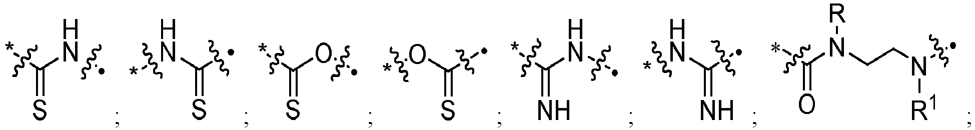 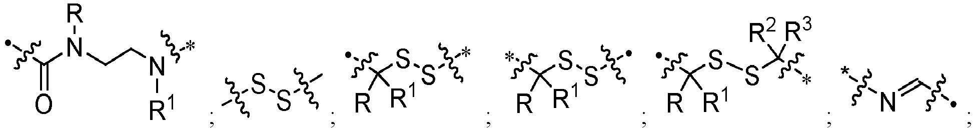 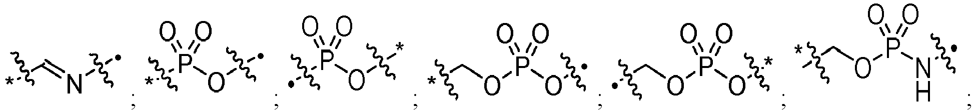 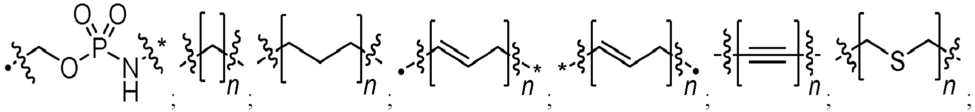 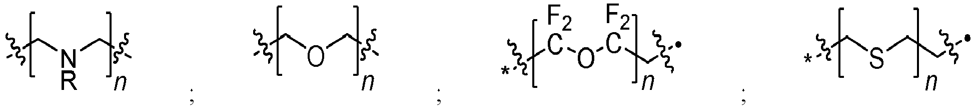 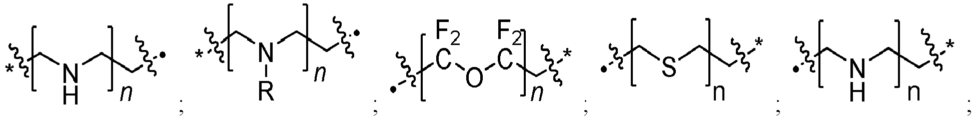 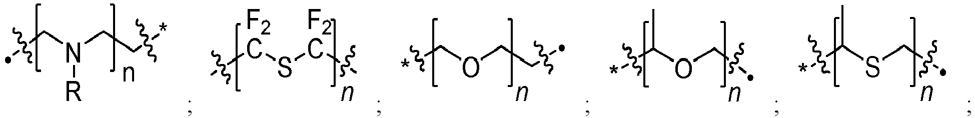 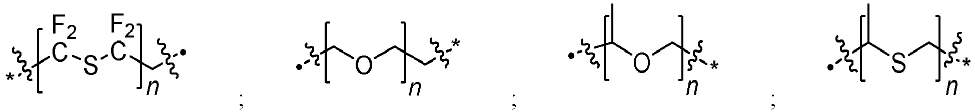 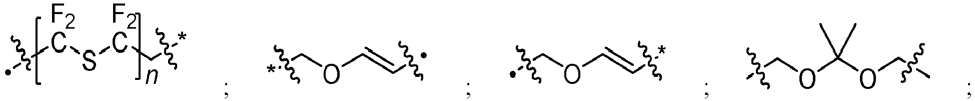 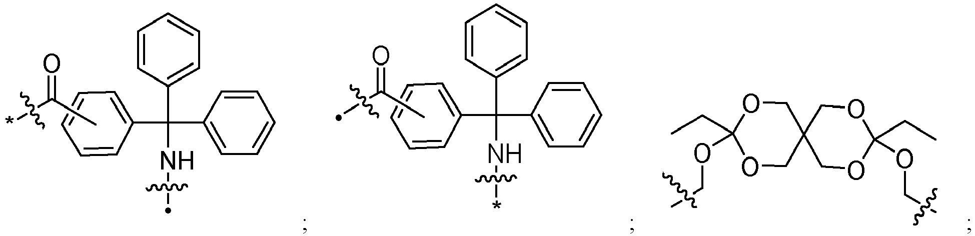 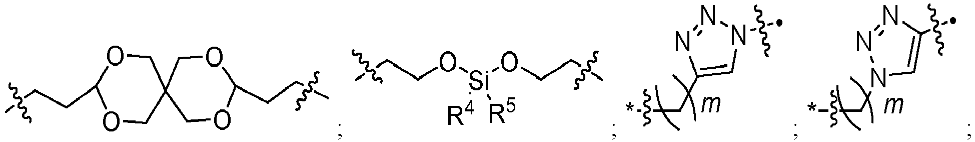 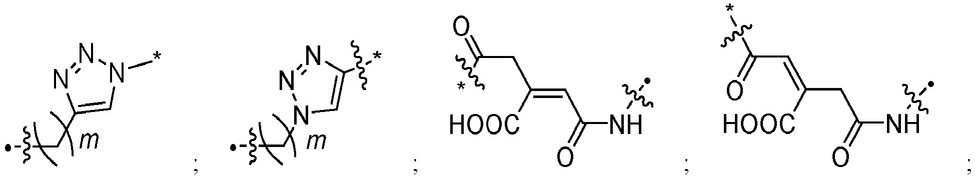 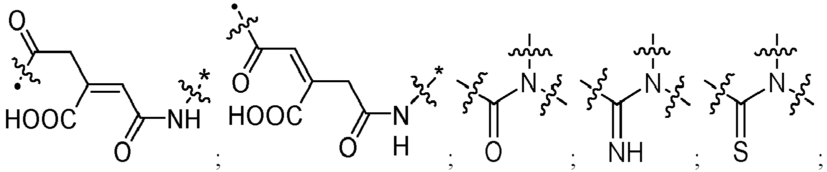 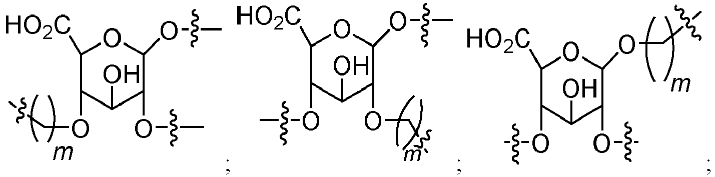 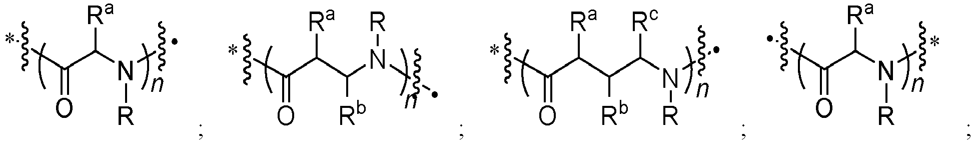 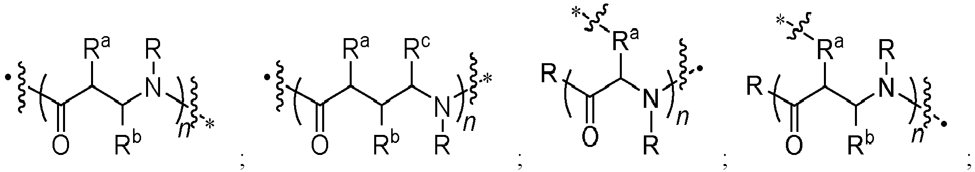 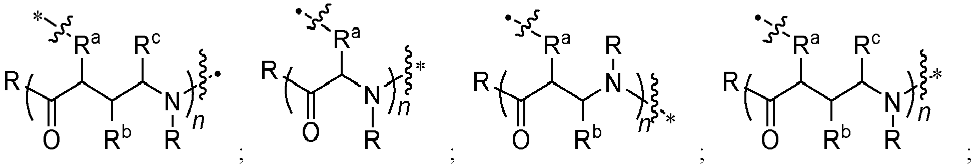 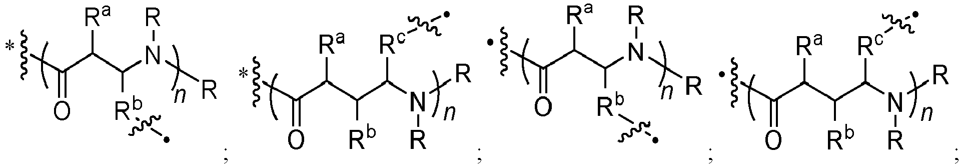 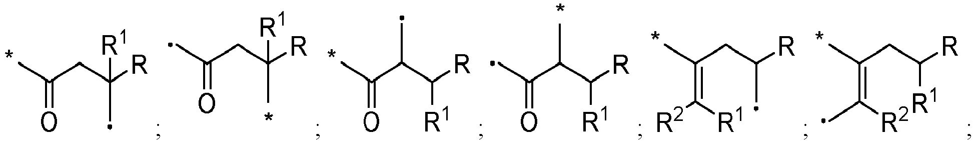 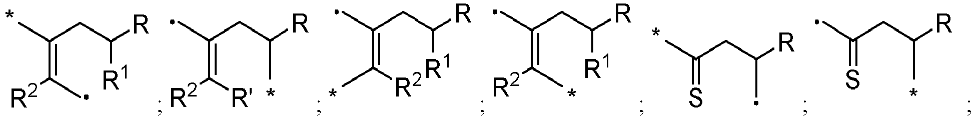 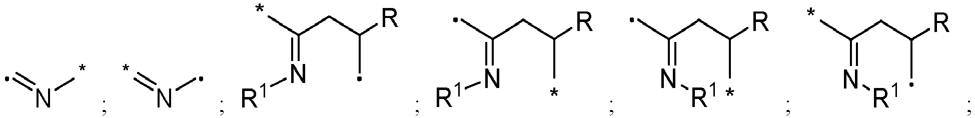 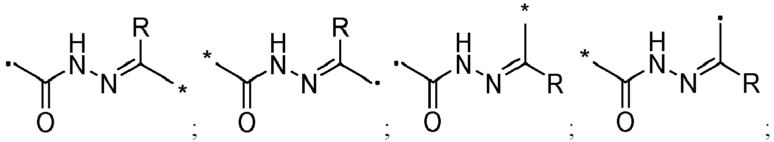 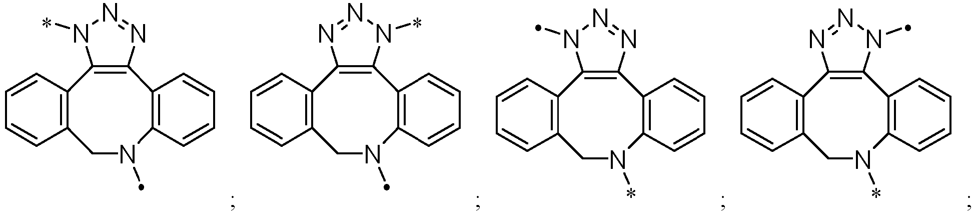 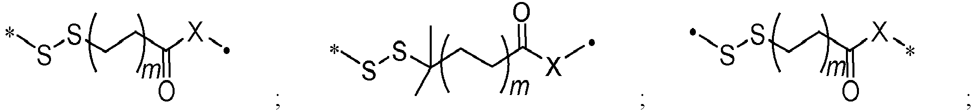 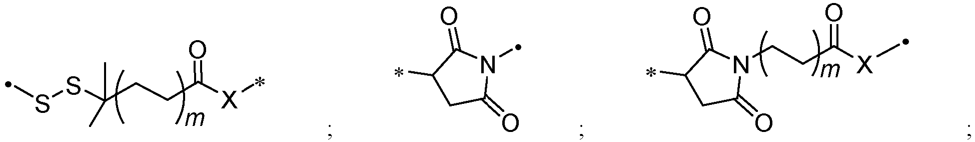 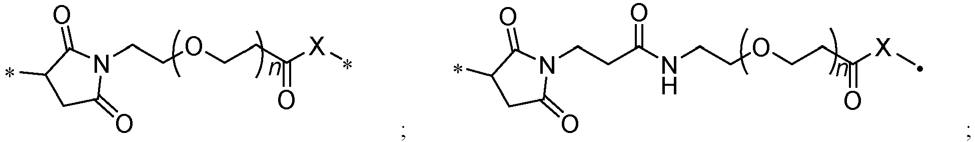 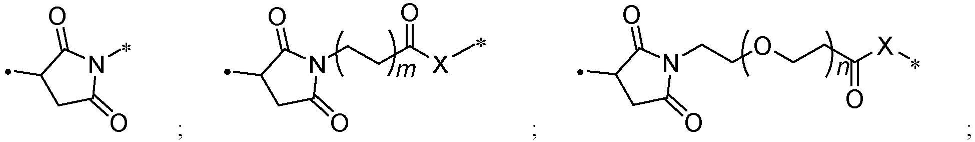 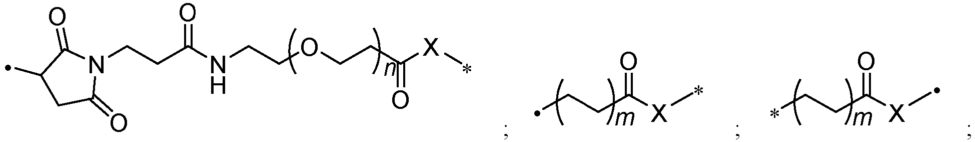 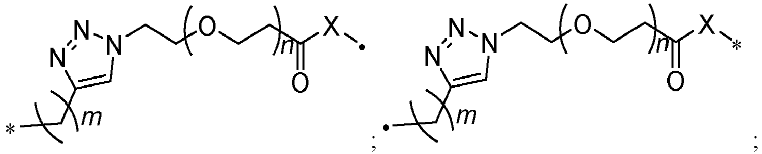
wherein each of R, R¹, R² and R³ is independently selected from H, OH, SH, NH₂, halogen, cyano, carboxy, alkyl, cycloalkyl, aryl and heteroaryl, each of which is substituted or unsubstituted;
each of R⁴ and R⁵ is independently selected from alkyl, cycloalkyl, aryl and heteroaryl, each of which is substituted or unsubstituted;
each of R^{a}, R^{b} and R^{c} is independently selected from side-chain residues of a proteinogenic or a non-proteinogenic amino acid, each of which can be further substituted;
each X is independently selected from NH, NR, S, O and CH₂, preferably NH;
each *of n* and *m* is independently an integer from 0 to 100, preferably 0 to 50, more preferably 0 to 30, yet more preferably selected from 0, 1, 2, 3, 4, 5, 6, 7, 8, 9, 10, 11, 12, 13, 14, 15, 16, 17, 18, 19 and 20; and
wherein each ^{∗} represents a point of attachment for which the shortest path to a moiety **A** comprises less atoms than that for •; and each • represents a point of attachment for which the shortest path to moiety **C** comprises less atoms than that for ^{∗}.

One or more **B_{L}** can independently comprise or consist of one or more of the following structural units: 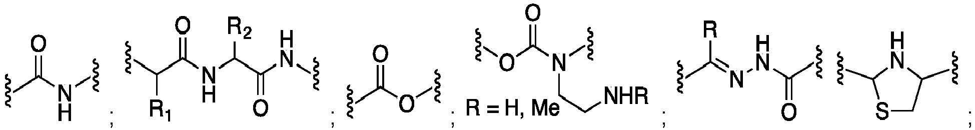 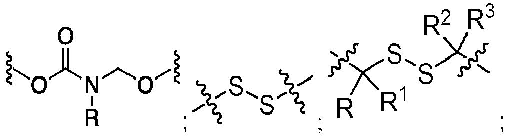 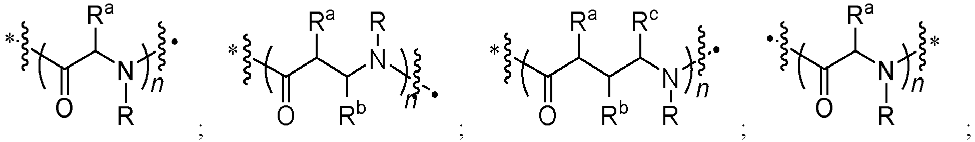 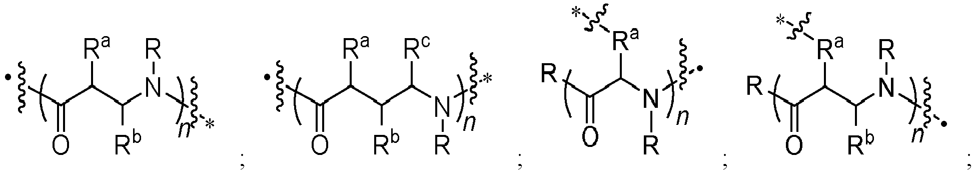 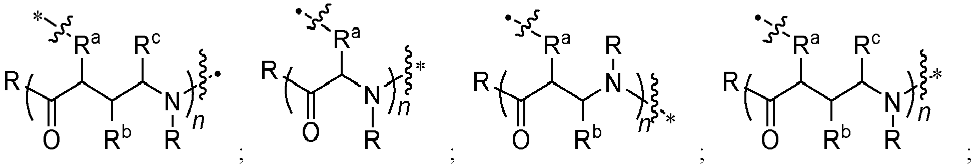 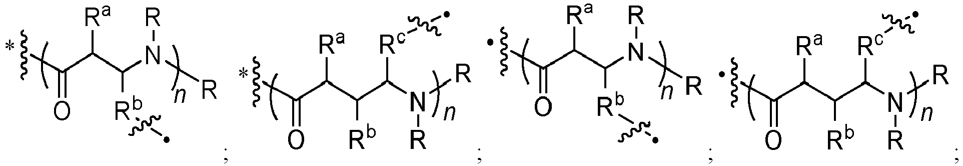
wherein in each of the above structures, n is 1, 2, 3 or 4; and
each ^{∗} represents a point of attachment for which the shortest path to a moiety **A** comprises less atoms than that for •; and each • represents a point of attachment for which the shortest path to moiety **C** comprises less atoms than that for ^{∗}, with the proviso that when n is > 1 and a respective point of attachment is indicated on any one of R^{a}, R^{b} and R^{c}, then it can be independently present in one or more of the peptide monomeric units, preferably in one peptide monomeric unit most distant from the other point of attachment indicated in the respective structure.

One or more of **B_{L}** and **B_{S}** can be independently selected from the following structures: 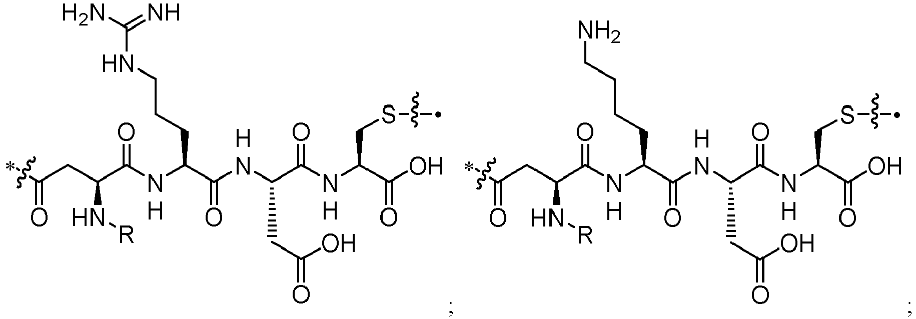 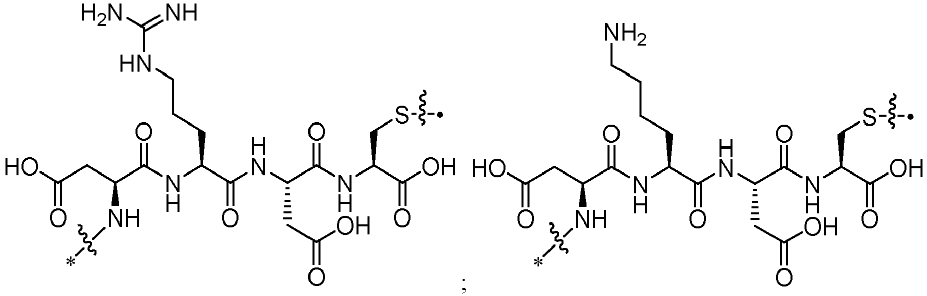
^{∗}―Val-Ala―•; ^{∗}―Val-Lys―•; ^{∗}―Val-Arg―•,
wherein each ^{∗} represents a point of attachment for which the shortest path to moiety A comprises less atoms than that for •; and each • represents a point of attachment for which the shortest path to moiety C comprises less atoms than that for ^{∗}.

In any of the above, y can be 1, 2 or 3; and/or at least one **B_{L}** can further comprise a cleavable linker group independently selected from the following structures:

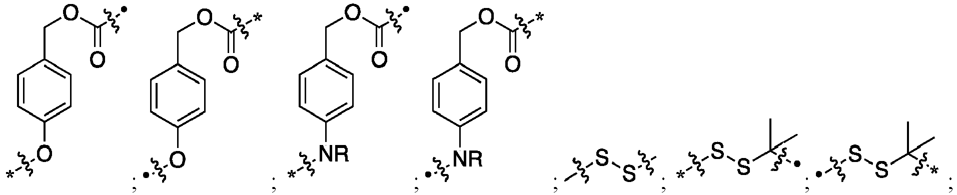

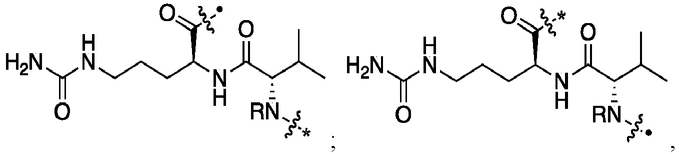

Wherein each ^{∗} represents a point of attachment for which the shortest path to moiety **A** comprises less atoms than that for •; and each • represents a point of attachment for which the shortest path to moiety **C** comprises less atoms than that for ^{∗}; or

Preferably, **B** has the following structure: 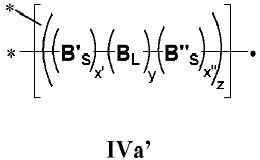 wherein **B'_{S}** and **B"_{S}** are each independently selected from the group consisting of: 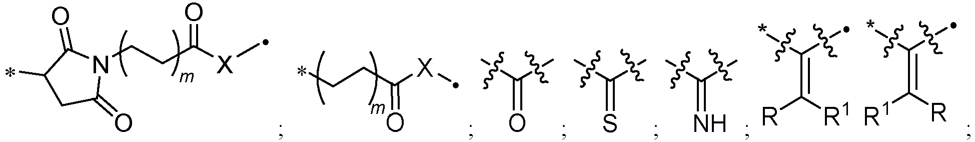 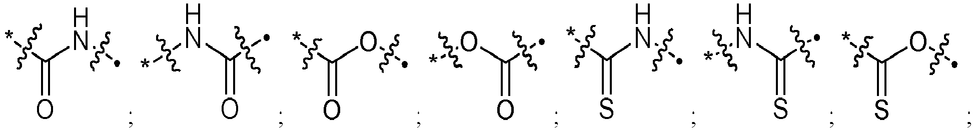 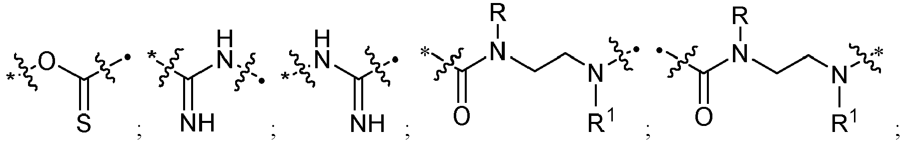
each **B_{L}** is independently selected from the group consisting of: 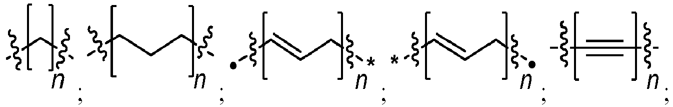 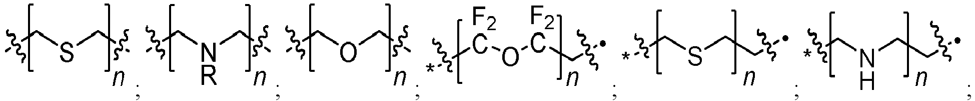 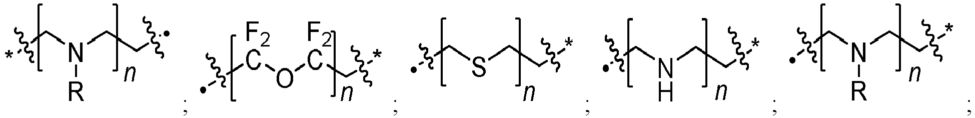 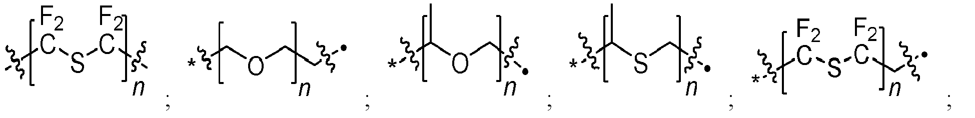 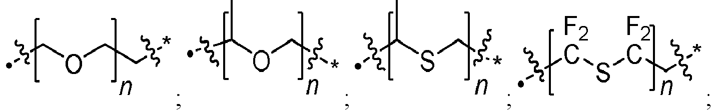 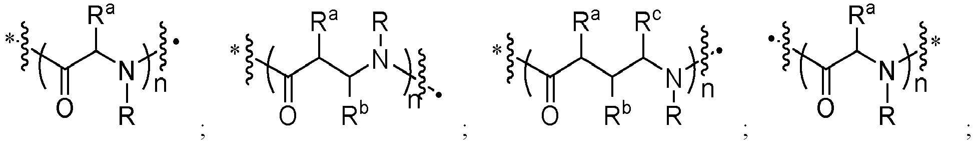 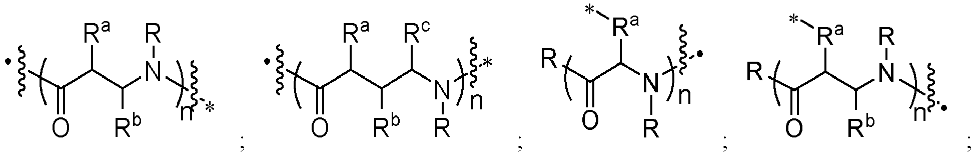 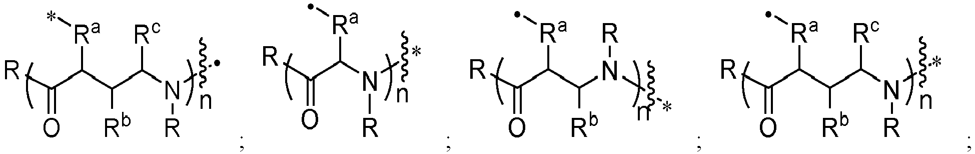 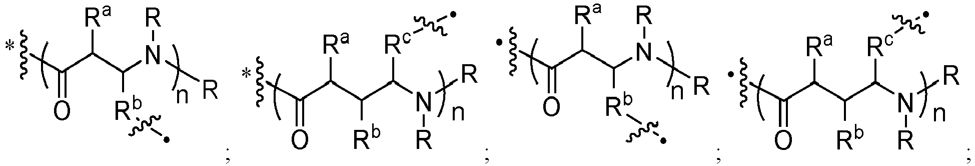 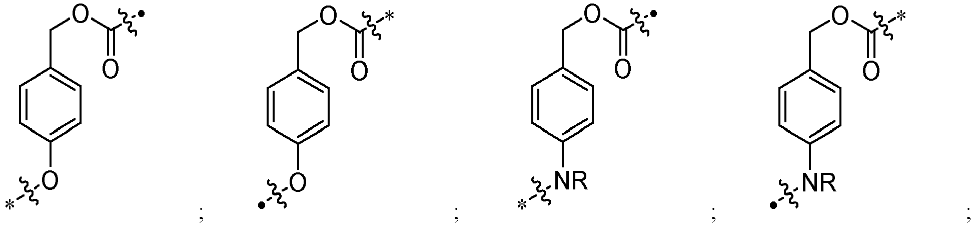 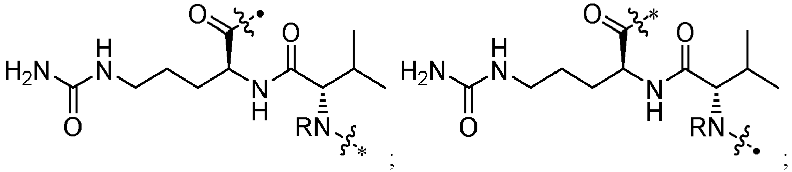
each *n* is 0, 1, 2, 3, 4 or 5;
each m is 0, 1, 2, 3, 4 or 5;
each *x*' is 0, 1 or 2;
each *x"* is 0, 1 or 2;
each *y* is 0, 1 or 2; and
z is 1 or 2,
wherein R, R¹, R², R³, R^{a}, R^{b}, R^{c}, X, ^{∗} and • are previously defined.

### Moiety C

Moiety **C** in the present invention represents a payload, which can be generally any atom (including H), molecule or particle. Preferably, moiety **C** is not a hydrogen atom.

The payload may be a chelator for radiolabelling. Suitably the radionuclide is not released. Chelators are well known to those skilled in the art, and for example, include chelators such as sulfur colloid, diethylenetriaminepentaacetic acid (DTPA), ethylenediaminetetraacetic acid (EDTA), 1,4,7,10-tetraazacyclododecane-N,N′,Nʺ,N‴-tetraacetic acid (DOTA), 1,4,7,10-tetraazacyclododececane,N-(glutaric acid)-N',N",N"'-triacetic acid (DOTAGA), 1,4,7-triazacyclononane-N,N',N"-triacetic acid (NOTA), 1,4,8,11-tetraazacyclotetradecane-N,N′,Nʺ,N‴-tetraacetic acid (TETA), or any of the preferred chelator structures recited in the appended claims.

The payload may be a radioactive group comprising or consisting of radioisotope including isotopes such as ²²³Ra, ⁸⁹Sr, ^{94m}Tc, ^{99m}Tc, ¹⁸⁶Re, ¹⁸⁸Re, ²⁰³Pb, ⁶⁷Ga, ⁶⁸Ga, ⁴⁷Sc, ¹¹¹In, ⁹⁷Ru, ⁶²Cu, ⁶⁴Cu, ⁸⁶Y, ⁸⁸Y, ⁹⁰Y, ¹²¹Sn, ¹⁶¹Tb, ¹⁵³Sm, ¹⁶⁶Ho, ¹⁰⁵Rh, ¹⁷⁷Lu, ¹²³I, ¹²⁴I, ¹²⁵I, ¹³¹I, ¹⁸F, ²¹¹At, ²²⁵Ac, ⁸⁹Sr, ²²⁵Ac, ^{117m}Sn and ¹⁶⁹E. Preferably, positron emitters, such as ¹⁸F and ¹²⁴l, or gamma emitters, such as ^{99m}Tc, ¹¹¹In and ¹²³I, are used for diagnostic applications (e.g. for PET), while beta-emitters, such as ⁸⁹Sr, ¹³¹I, and ¹⁷⁷Lu, are preferably used for therapeutic applications. Alpha-emitters, such as ²¹¹At, ²²⁵Ac and ²²³Ra may also be used for therapy. In one preferred embodiment the radioisotope is ⁸⁹Sr or ²²³Ra. In a further preferred embodiment the radioisotope is ⁶⁸Ga.

The payload may be a chelate of a radioactive isotope, preferably of an isotope listed under above, with a chelating agent, preferably a chelating agent listed above or any of the preferred chelator structures recited in the appended claim 8 (a); or a group selected from the structures listed in claim 8 (c).

The payload may be a fluorophore group, preferably selected from a xanthene dye, acridine dye, oxazine dye, cyanine dye, styryl dye, coumarine dye, porphine dye, fluorescent metal-ligand-complex, fluorescent protein, nanocrystals, perylene dye, boron-dipyrromethene dye and phtalocyanine dye, more preferably selected from the structures listed in claim 8 (d).

The payload may be a cytotoxic and/or cytostatic agent. Such agents can inhibit or prevent the function of cells and/or cause destruction of cells. Examples of cytotoxic agents include radioactive isotopes, chemotherapeutic agents, and toxins such as small molecule toxins or enzymatically active toxins of bacterial, fungal, plant or animal origin, including synthetic analogues and derivatives thereof. The cytotoxic agent may be selected from the group consisting of an auristatin, a DNA minor groove binding agent, a DNA minor groove alkylating agent, an enediyne, a lexitropsin, a duocarmycin, a taxane, a puromycin, a dolastatin, a maytansinoid and a vinca alkaloid or a combination of two or more thereof. Preferred cytotoxic and/or cytostatic payload moieties are listed in claim 8 (e).

In one embodiment the payload is a chemotherapeutic agent selected from the group consisting of a topoisomerase inhibitor, an alkylating agent (*e.g.,* nitrogen mustards; ethylenimes; alkylsulfonates; triazenes; piperazines; and nitrosureas), an antimetabolite (*e.g.,* mercaptopurine, thioguanine, 5-fluorouracil), an antibiotics (*e.g.,* anthracyclines, dactinomycin, bleomycin, adriamycin, mithramycin. dactinomycin) a mitotic disrupter (*e.g.,* plant alkaloids - such as vincristine and/or microtubule antagonists - such as paclitaxel), a DNA methylating agent, a DNA intercalating agent (e.g., carboplatin and/or cisplatin, daunomycin and/or doxorubicin and/or bleomycin and/or thalidomide), a DNA synthesis inhibitor, a DNA-RNA transcription regulator, an enzyme inhibitor, a gene regulator, a hormone response modifier, a hypoxia-selective cytotoxin (*e.g.,* tirapazamine), an epidermal growth factor inhibitor, an anti-vascular agent (*e.g.,* xanthenone 5,6-dimethylxanthenone-4-acetic acid), a radiation-activated prodrug (*e.g.,* nitroarylmethyl quaternary (NMQ) salts) or a bioreductive drug or a combination of two or more thereof. In some embodiments, the payload (i.e., moiety C) is not derived from an anthracycline, preferably not derived from PNU 159682.

The chemotherapeutic agent may selected from the group consisting of Erlotinib (TARCEVA^{®}), Bortezomib (VELCADE^{®}), Fulvestrant (FASLODEX^{®}), Sutent (SU11248), Letrozole (FEMARA^{®}), Imatinib mesylate (GLEEVEC^{®}), PTK787/ZK 222584, Oxaliplatin (Eloxatin^{®}.), 5-FU (5-fluorouracil), Leucovorin, Rapamycin (Sirolimus, RAPAMUNE^{®}.), Lapatinib (GSK572016), Lonafarnib (SCH 66336), Sorafenib (BAY43-9006), and Gefitinib (IRESSA^{®}.), AG1478, AG1571 (SU 5271; Sugen) or a combination of two or more thereof.

The chemotherapeutic agent may be an alkylating agent - such as thiotepa, CYTOXAN^{®} and/or cyclosphosphamide; an alkyl sulfonate - such as busulfan, improsulfan and/or piposulfan; an aziridine - such as benzodopa, carboquone, meturedopa and/or uredopa; ethylenimines and/or methylamelamines - such as altretamine, triethylenemelamine, triethylenepbosphoramide, triethylenethiophosphoramide and/or trimethylomelamine; acetogenin - such as bullatacin and/or bullatacinone; camptothecin; bryostatin; callystatin; cryptophycins; dolastatin; duocarmycin; eleutherobin; pancratistatin; sarcodictyin; spongistatin; nitrogen mustards - such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide and/or uracil mustard; nitrosureas - such as carmustine, chlorozotocin, fotemustine, lomustine, nimustine, and/or ranimnustine; dynemicin; bisphosphonates - such as clodronate; an esperamicin; a neocarzinostatin chromophore; aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN^{®}. doxorubicin - such as morpholino-doxorubicin, cyanomorpholino-doxorubicin, 2-pyrrolino-doxorubicin and/or deoxydoxorubicin, epirubicin, esorubicin, idarubicin, marcellomycin, mitomycins - such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; antimetabolites - such as methotrexate and 5-fluorouracil (5-FU); folic acid analogues - such as denopterin, methotrexate, pteropterin, trimetrexate; purine analogues - such as fludarabine, 6-mercaptopurine, thiamiprine, thioguanine; pyrimidine analogues - such as ancitabine, azacitidine, 6-azauridine, carmofur, cytarabine, dideoxyuridine, doxifluridine, enocitabine, floxuridine; androgens - such as calusterone, dromostanolone propionate, epitiostanol, mepitiostane, testolactone; anti-adrenals - such as aminoglutethimide, mitotane, trilostane; folic acid replenisher - such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; macrocyclic depsipeptides such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; razoxane; rhizoxin; sizofiran; spirogermanium; tenuazonic acid; triaziquone; 2,2',2"-trichlorotriethylamine; trichothecenes - such as verracurin A, roridin A and/or anguidine; urethan; vindesine; dacarbazine; mannomustine; mitobronitol; mitolactol; pipobroman; gacytosine; arabinoside; cyclophosphamide; thiotepa; taxoids - such as TAXOL^{®}. paclitaxel, abraxane, and/or TAXOTERE^{®}, doxetaxel; chloranbucil; GEMZAR^{®}. gemcitabine; 6-thioguanine; mercaptopurine; methotrexate; platinum analogues - such as cisplatin and carboplatin; vinblastine; platinum; etoposide; ifosfamide; mitoxantrone; vincristine; NAVELBINE^{®}, vinorelbine; novantrone; teniposide; edatrexate; daunomycin; aminopterin; xeloda; ibandronate; topoisomerase inhibitor RFS 2000; difluoromethylomithine (DMFO); retinoids - such as retinoic acid; capecitabine; and pharmaceutically acceptable salts, acids, derivatives or combinations of two or more of any of the above.

The payload may be a tubulin disruptor including but are not limited to: taxanes - such as paclitaxel and docetaxel, vinca alkaloids, discodermolide, epothilones A and B, desoxyepothilone, cryptophycins, curacin A, combretastatin A-4-phosphate, BMS 247550, BMS 184476, BMS 188791; LEP, RPR 109881A, EPO 906, TXD 258, ZD 6126, vinflunine, LU 103793, dolastatin 10, E7010, T138067 and T900607, colchicine, phenstatin, chalcones, indanocine, T138067, oncocidin, vincristine, vinblastine, vinorelbine, vinflunine, halichondrin B, isohomohalichondrin B, ER-86526, pironetin, spongistatin 1, spiket P, cryptophycin 1, LU103793 (cematodin or cemadotin), rhizoxin, sarcodictyin, eleutherobin, laulilamide, VP-16 and D-24851 and pharmaceutically acceptable salts, acids, derivatives or combinations of two or more of any of the above.

The payload may be a DNA intercalator including but are not limited to: acridines, actinomycins, anthracyclines, benzothiopyranoindazoles, pixantrone, crisnatol, brostallicin, CI-958, doxorubicin (adriamycin), actinomycin D, daunorubicin (daunomycin), bleomycin, idarubicin, mitoxantrone, cyclophosphamide, melphalan, mitomycin C, bizelesin, etoposide, mitoxantrone, SN-38, carboplatin, cisplatin, actinomycin D, amsacrine, DACA, pyrazoloacridine, irinotecan and topotecan and pharmaceutically acceptable salts, acids, derivatives or combinations of two or more of any of the above.

The payload may be an anti-hormonal agent that acts to regulate or inhibit hormone action on tumours - such as anti-estrogens and selective estrogen receptor modulators, including, but not limited to, tamoxifen, raloxifene, droloxifene, 4-hydroxytamoxifen, trioxifene, keoxifene, LY117018, onapristone, and/or fareston toremifene and pharmaceutically acceptable salts, acids, derivatives or combinations of two or more of any of the above. The payload may be an aromatase inhibitor that inhibits the enzyme aromatase, which regulates estrogen production in the adrenal glands - such as, for example, 4(5)-imidazoles, aminoglutethimide, megestrol acetate, AROMASIN^{®}. exemestane, formestanie, fadrozole, RIVISOR^{®}. vorozole, FEMARA^{®}. letrozole, and ARIMIDEX^{®} and/or anastrozole and pharmaceutically acceptable salts, acids, derivatives or combinations of two or more of any of the above.

The payload may be an anti-androgen such as flutamide, nilutamide, bicalutamide, leuprolide, goserelin and/or troxacitabine and pharmaceutically acceptable salts, acids, derivatives or combinations of two or more of any of the above.

The payload may be a protein or an antibody. Preferably, the payload is a cytokine (e.g., an interleukin such as IL2, IL10, IL12, IL15; a member of the TNF superfamily; or an interferon such as interferon gamma.).

Any payload may be used in unmodified or modified form. Combinations of payloads in which some are unmodified and some are modified may be used. For example, the payload may be chemically modified. One form of chemical modification is the derivatisation of a carbonyl group - such as an aldehyde.

In a preferred embodiment, moiety C is an auristatin (i.e., having a structure derived from an auristatin compound family member) or an auristatin derivative. More preferably, moiety C has a structure according to the following formula: 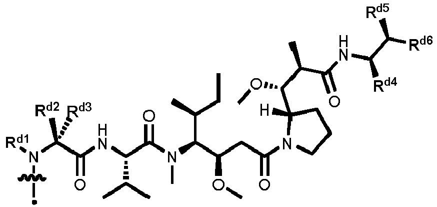 wherein:
R^{1d} is independently H or C₁-C₆ alkyl; preferably H or CH₃;
R^{2d} is independently C₁-C₆ alkyl; preferably CH₃ or iPr;
R^{3d} is independently H or C₁-C₆ alkyl; preferably H or CH₃;
R^{4d} is independently H, C₁-C₆ alkyl, COO(C₁-C₆ alkyl), CON(H or C₁-C₆ alkyl), C₃-C₁₀ aryl or C₃-C₁₀ heteroaryl; preferably H, CH₃, COOH, COOCH₃ or thiazolyl;
R^{5d} is independently H, OH, C₁-C₆ alkyl; preferably H or OH; and
R^{6d} is independently C₃-C₁0 aryl or C₃-C₁0 heteroaryl; preferably optionally substituted phenyl or pyridyl.

More preferably, moiety C is derived from MMAE or MMAF.

In a preferred embodiment, moiety C has a structure according to the following formula: 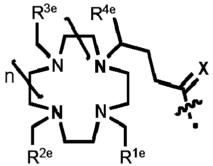 wherein:
n is 0, 1, 2, 3, 4 or 5; preferably 1;
R^{1e} is independently H, COOH, aryl-COOH or heteroaryl-COOH; preferably COOH;
R^{2e} is independently H, COOH, aryl-COOH or heteroaryl-COOH; preferably COOH;
each R^{3e} is independently H, COOH, aryl-COOH or heteroaryl-COOH; preferably COOH;
R^{4e} is independently H, COOH, aryl-COOH or heteroaryl-COOH; preferably COOH; and
X is O, NH or S; preferably O.

In a preferred embodiment, moiety C has a structure according to the following formula: 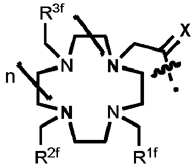 wherein:
n is 0, 1, 2, 3, 4 or 5; preferably 1
R^{1f} is independently H, COOH, aryl-COOH or heteroaryl-COOH; preferably COOH;
R^{2f} is independently H, COOH, aryl-COOH or heteroaryl-COOH; preferably COOH;
R^{3f} is independently H, COOH, aryl-COOH or heteroaryl-COOH; preferably COOH; and
X is O, NH or S; preferably O

Particularly preferred embodiments for the moiety C as well as the compound according to the present invention are shown in the appended claims.

Preferred compounds are those having a structure according to Table 2 or 3, their individual diastereoisomers, hydrates, solvates, crystal forms, individual tautomers or pharmaceutically acceptable salts thereof.

### Further aspects

In one aspect, herein disclosed a compound of the general Formula I as defined above, its individual diastereoisomers, its hydrates, its solvates, its crystal forms, its individual tautomers or a pharmaceutically acceptable salt thereof, wherein: A is a binding moiety having the structure as defined above; **B** is a covalent bond or a moiety comprising a chain of atoms that covalently attaches the moieties **A** und **C;** and **C** is a payload moiety.

In one further aspect, **B** is represented by any of the general Formulae II―V as defined above, wherein each **B_{S}** independently represents a spacer group; each **B_{L}** independently represents a cleavable or non-cleavable linker group; each *x* is an integer independently selected from the range of 0 to 100, preferably 0 to 50, more preferably 0 to 30, yet more preferably selected from 0, 1, 2, 3, 4, 5, 6, 7, 8, 9, 10, 11, 12, 13, 14, 15, 16, 17, 18, 19 and 20; each *y* is an integer independently selected from the range of 0 to 30, preferably selected from 0, 1, 2, 3, 4, 5, 6, 7, 8, 9, 10, 11, 12, 13, 14, 15, 16, 17, 18, 19 and 20; each z is an integer independently selected from the range of 0 to 5, preferably selected from selected from 0, 1, 2, 3 and 4; and ^{∗} represents a point of attachment to moiety **A;** and • represents a point of attachment to moiety **C.**

In one further aspect according to any of the preceding aspects, the binding moiety has the structure **A¹** as defined above.

In one further aspect according to any of the preceding aspects, **B_{S}** and/or **B_{L}** is a group comprising or consisting of a structural unit independently selected from the group consisting of alkylene, cycloalkylene, arylalkylene, heteroarylalkylene, heteroalkylene, heterocycloalkylene, alkenylene, cycloalkenylene, arylalkenylene, heteroarylalkenylene, heteroalkenylene, heterocycloalenkylene, alkynylene, heteroalkynylene, arylene, heteroarylene, aminoacyl, oxyalkylene, aminoalkylene, diacid ester, dialkylsiloxane, amide, thioamide, thioether, thioester, ester, carbamate, hydrazone, thiazolidine, methylene alkoxy carbamate, disulfide, vinylene, imine, imidamide, phosphoramide, saccharide, phosphate ester, phosphoramide, carbamate, dipeptide, tripeptide, tetrapeptide, each of which is substituted or unsubstituted.

In one further aspect, the compound preferably has a structure represented by one of the following formulae: 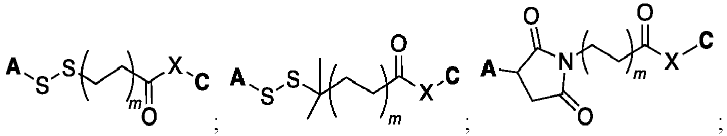 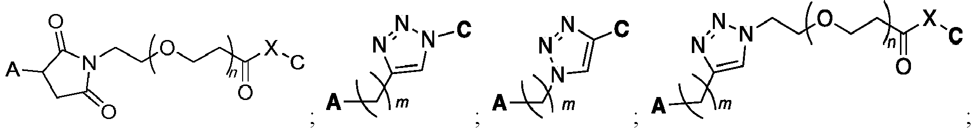 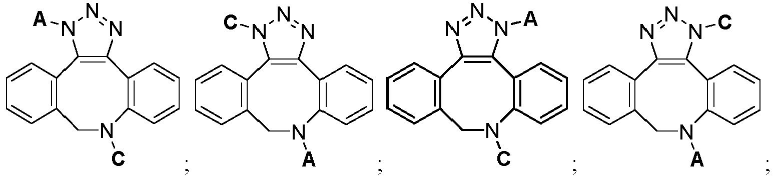 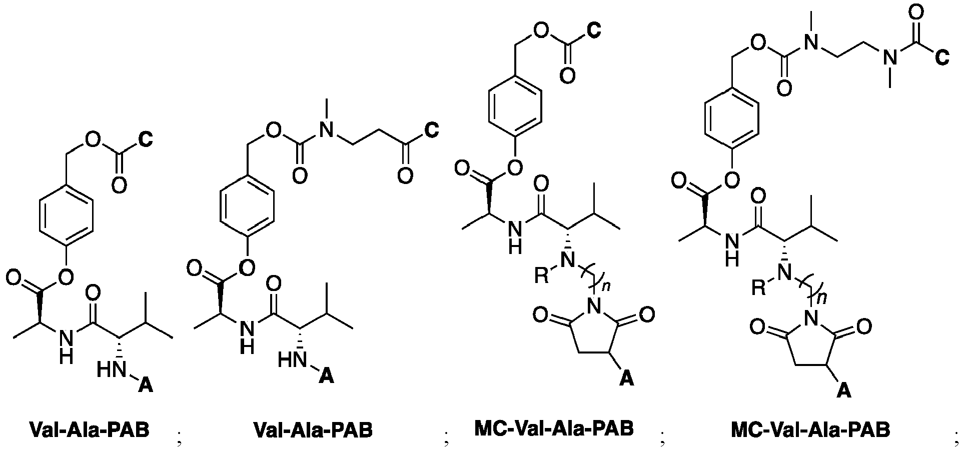 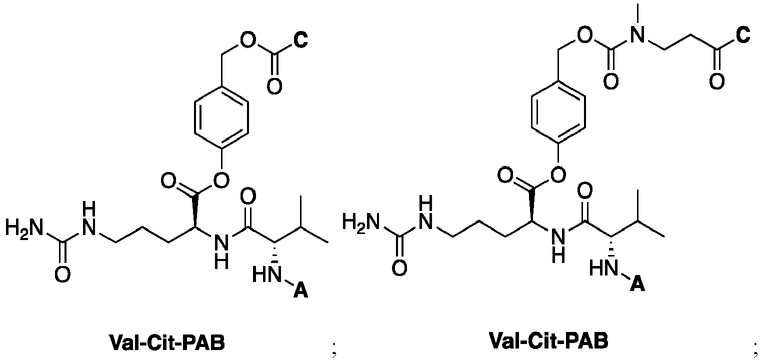 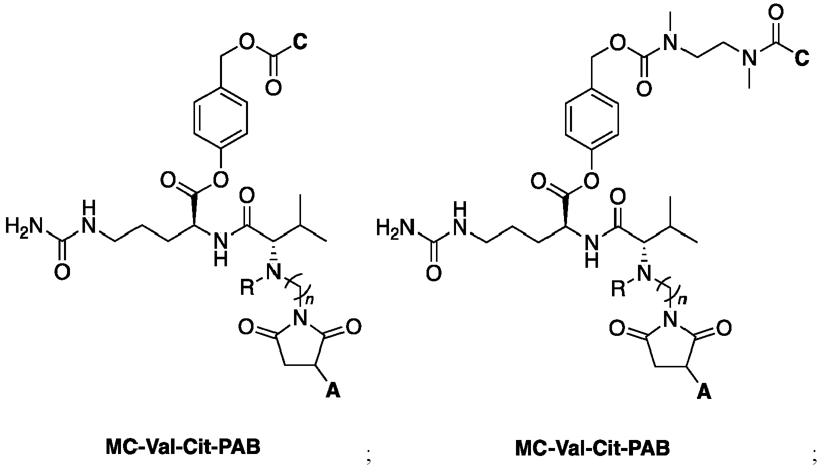 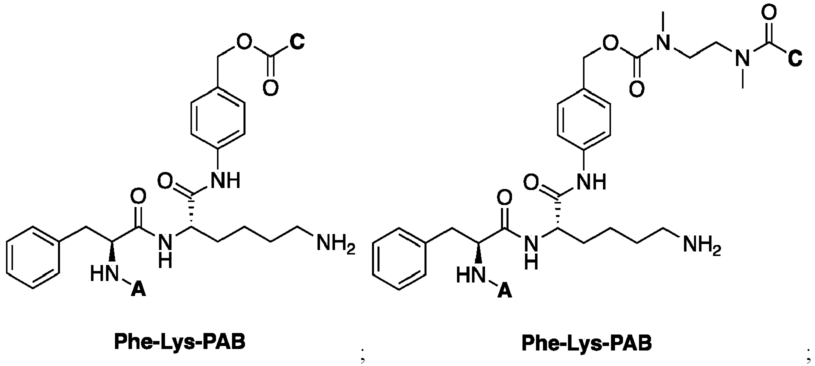 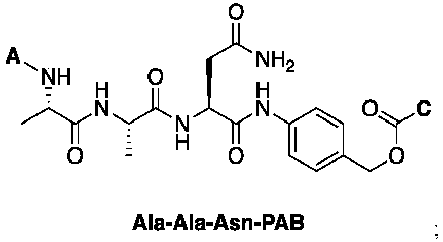 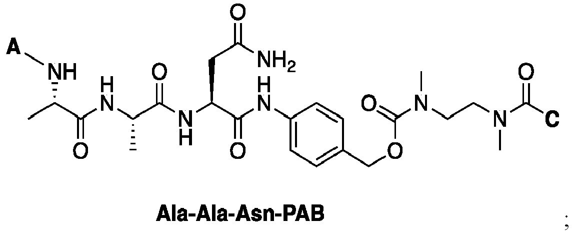 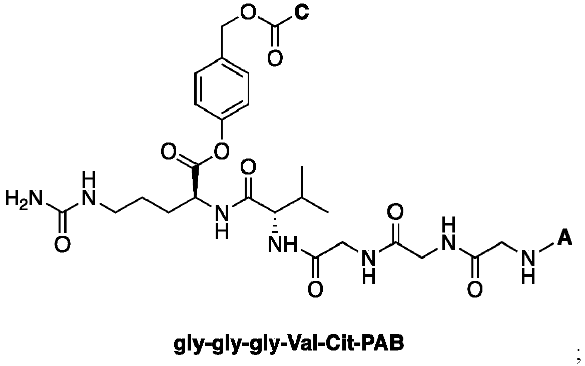 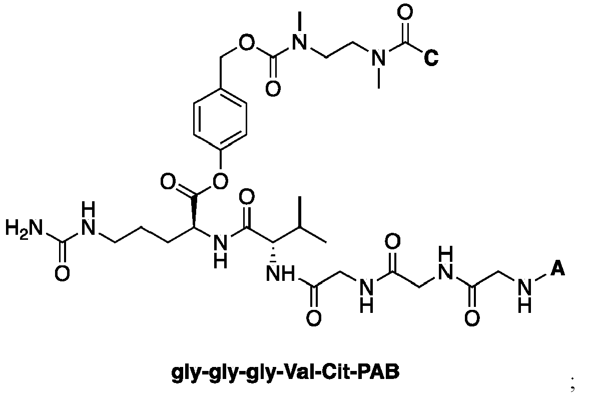 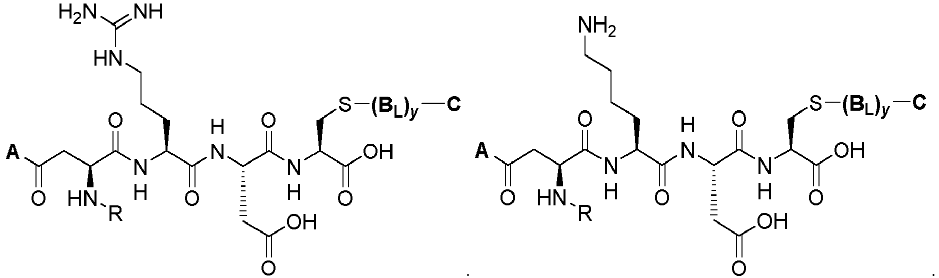 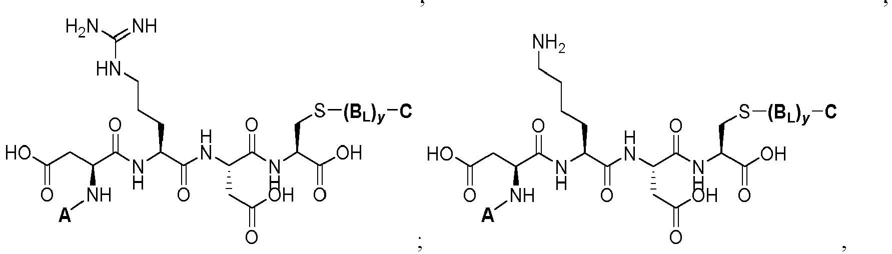 wherein each of the above structures comprises one further moiety A linked to the moiety corresponding to B.

In one further aspect according to any of the preceding aspects, the moiety C is as defined in appended claim 8.

In a preferred aspect, the compound has the structure: 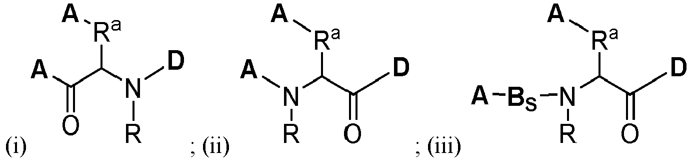 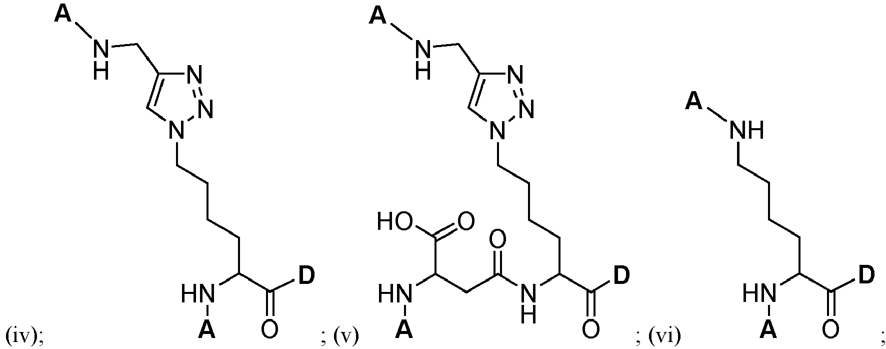 or 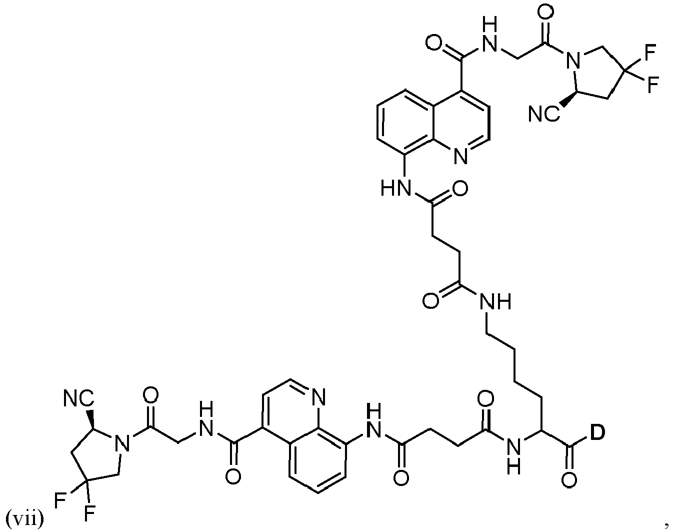 wherein moiety **D** represents **B―C** as previously defined.

In a preferred aspect, the compound comprises the structure: 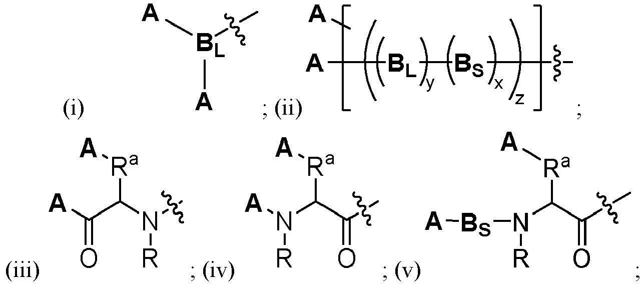 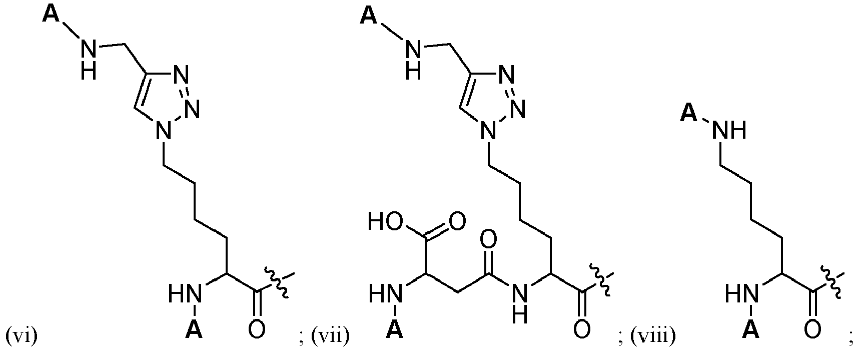 or

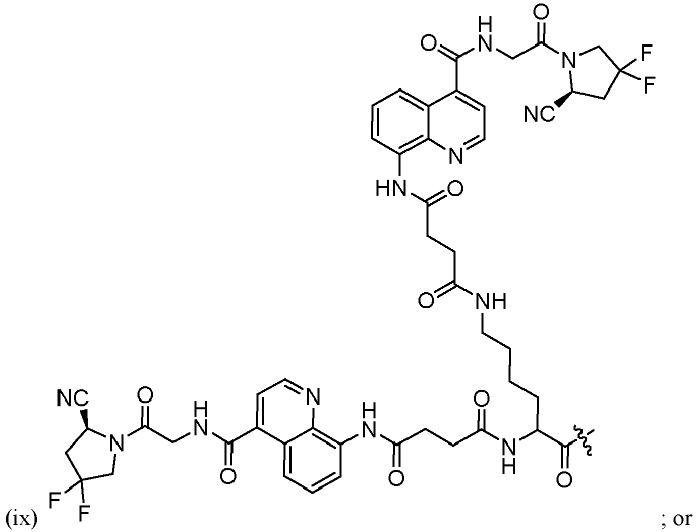

In a preferred aspect, the compound has the structure: 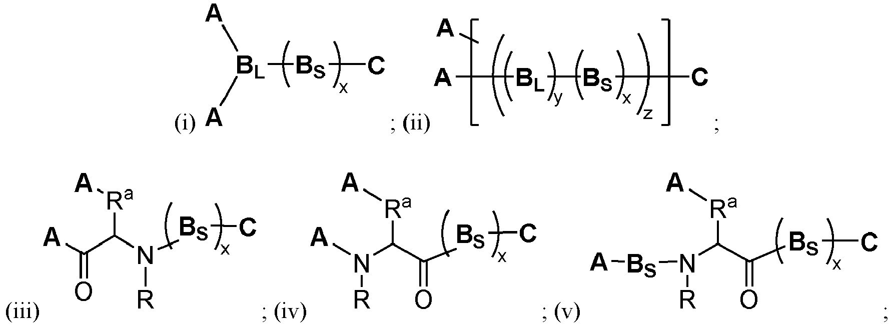 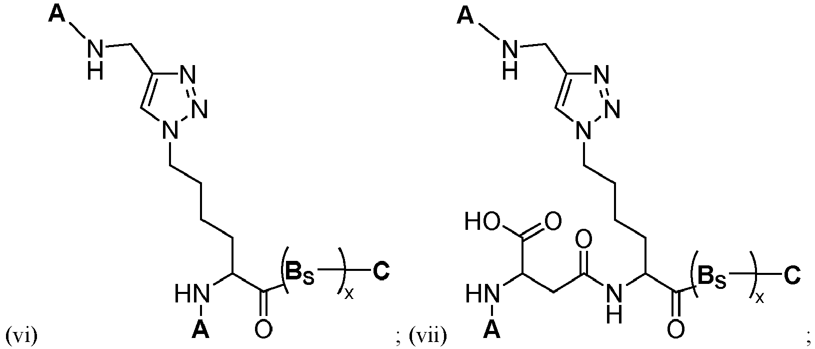 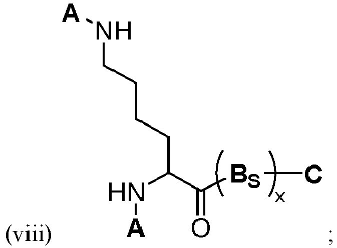 or

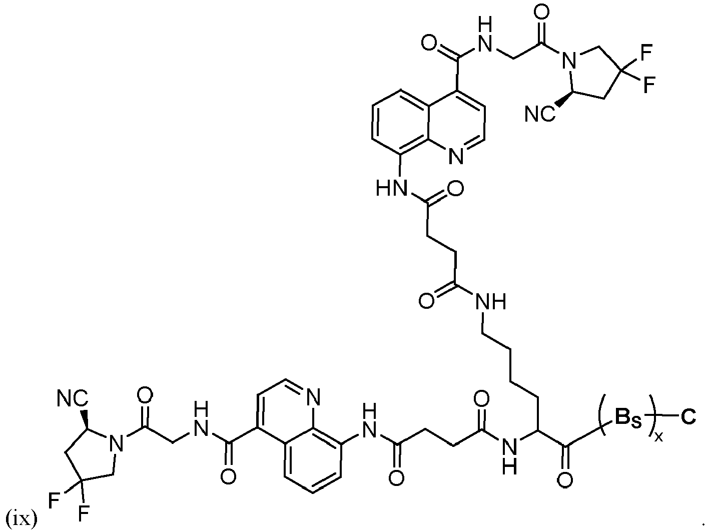

Most preferably, the compound has or comprises the following structure:

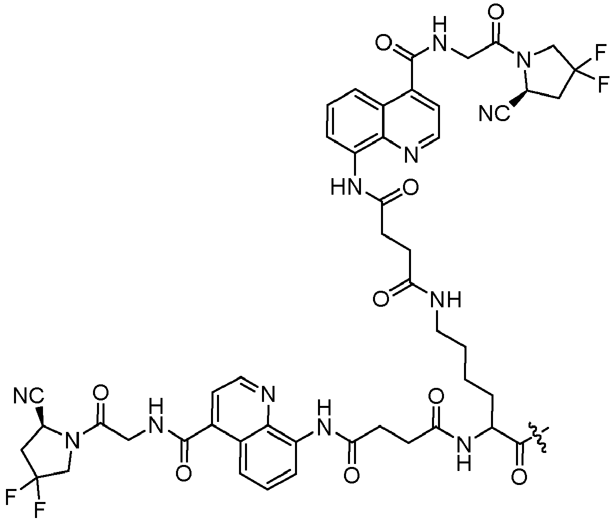

Moiety D or **(B_{S})ₓC** can be represented by one of the following structures: 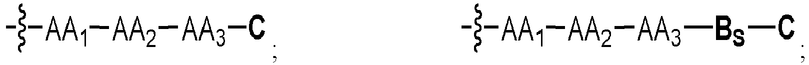 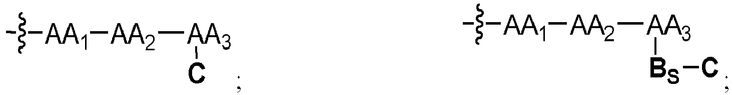 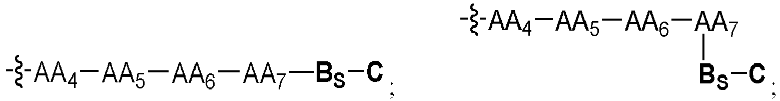 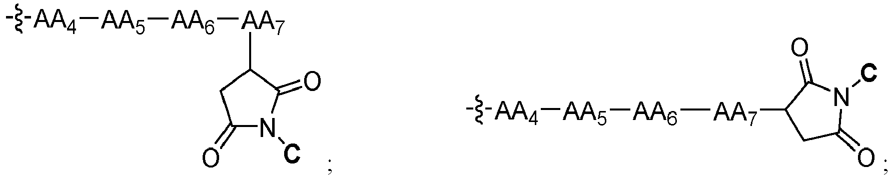 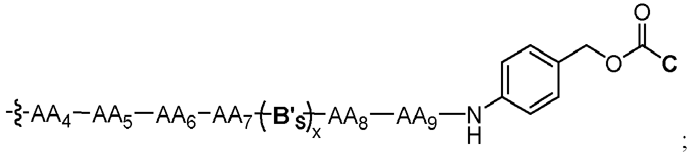 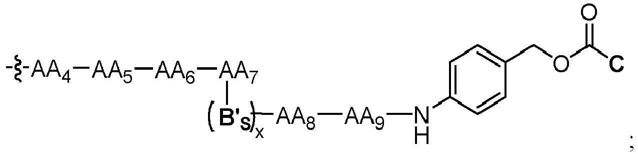 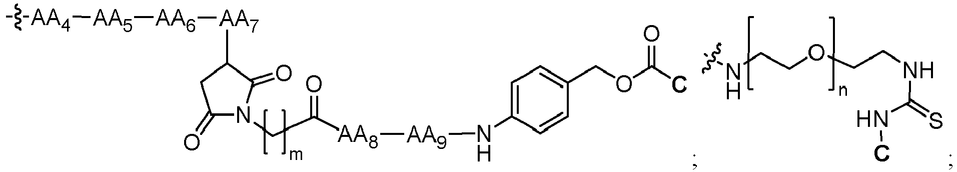 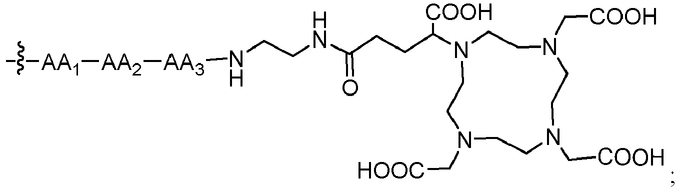 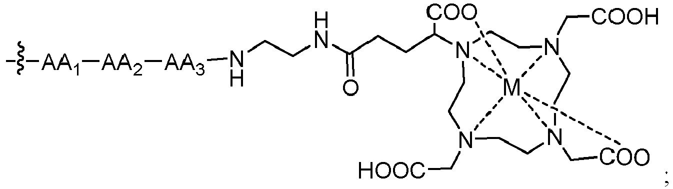 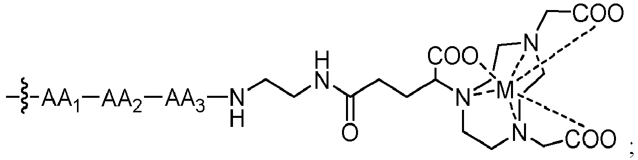 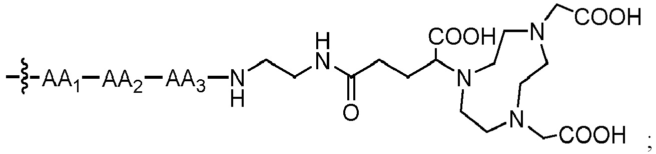 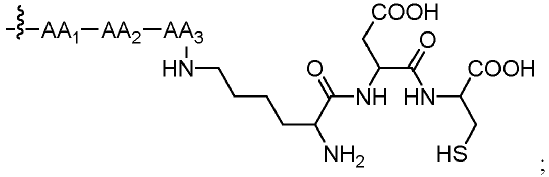 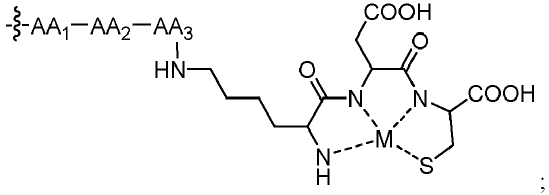 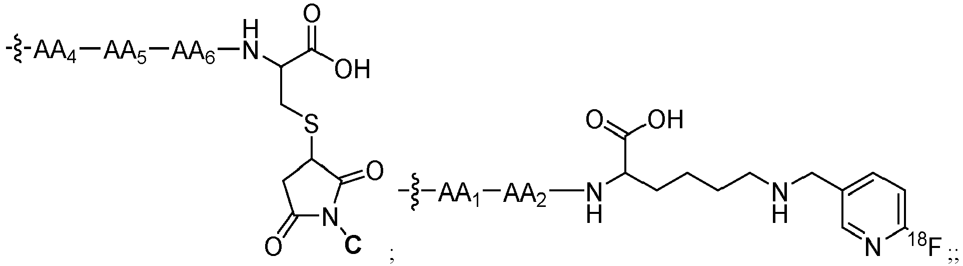 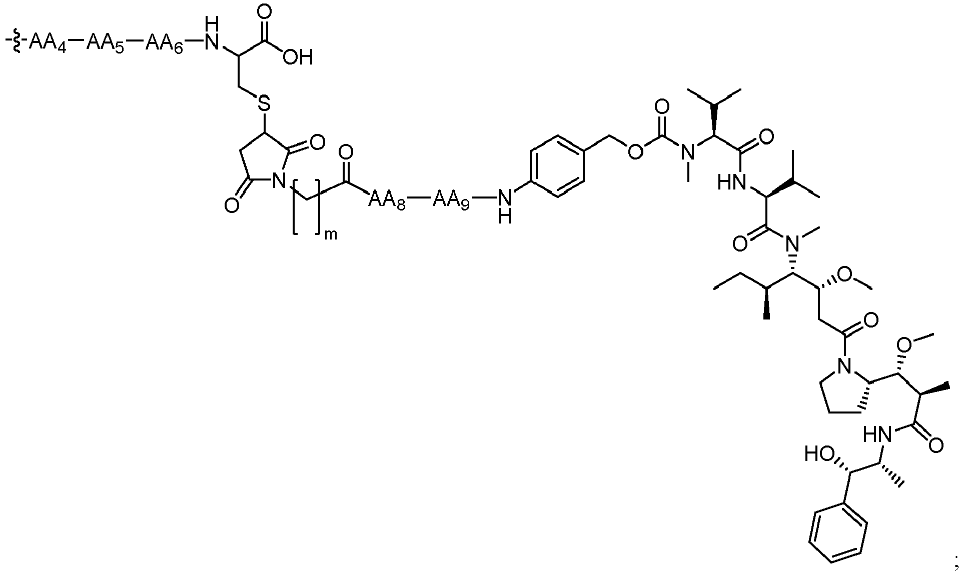 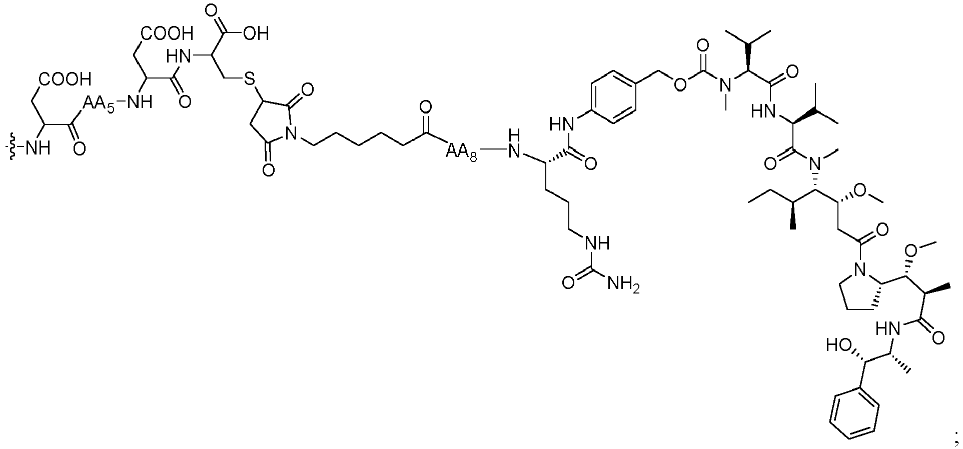 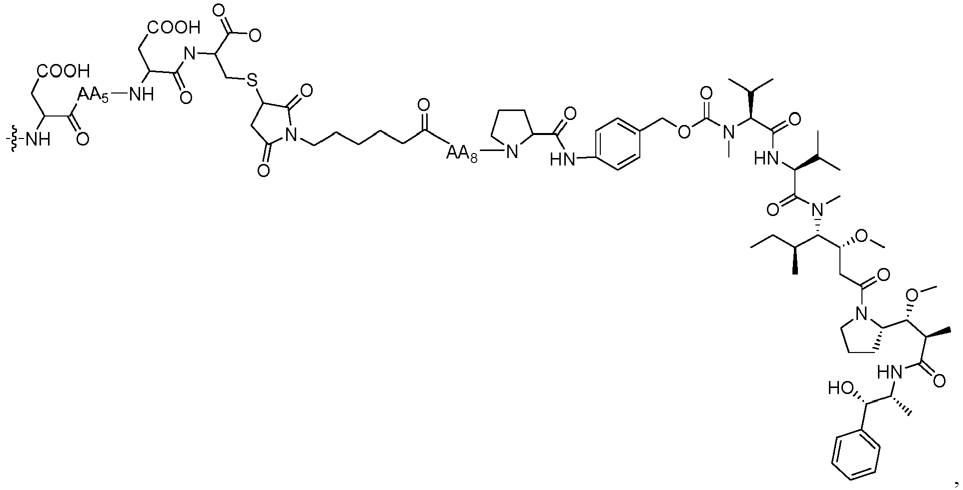
wherein each of AA₁, AA₂, AA₃, AA₄, AA₅, AA₆, AA₇, AA₈, and AA₉ represents a proteinogenic or non-proteinogenic amino acid, or is absent;
preferably wherein: AA₅ is an amino acid with a charged sidechain, and AA₈ is an amino acid with an aliphatic sidechain;
more preferably wherein: AA₁ is selected from Asp and Glu, or is absent; AA₂ is selected from Asp and Glu, or is absent; AA₃ is Lys; AA₄ is selected from Asp and Glu; AA₅ is selected from Lys and Arg; AA6 is selected from Asp and Glu; AA₇ is selected from Cys; and AA₈ is selected from Gly, Ala, and Val; and AA₉ is selected from Pro and citrulline (Cit).

In all structures, unless otherwise specified, all groups and variables are defined as further above in the present disclosure.

Also disclosed is a pharmaceutical composition comprising the compound according to any of the preceding aspects, and a pharmaceutically acceptable excipient. Such pharmaceutical composition is also disclosed for use in: (a) a method for treatment of the human or animal body by surgery or therapy or a diagnostic method practised on the human or animal body; or (b) a method for therapy or prophylaxis of a subject suffering from or having risk for a disease or disorder; or (c) a method for guided surgery practised on a subject suffering from or having risk for a disease or disorder; or (d) a method for diagnosis of a disease or disorder, the method being practised on the human or animal body and involving a nuclear medicine imaging technique, such as Positron Emission Tomography (PET) or Single Photon Emission Computed Tomography (SPECT); or (e) a method for targeted delivery of a therapeutic or diagnostic agent to a subject suffering from or having risk for a disease or disorder, wherein in each of the preceding (b)-(e), said disease or disorder is independently selected from cancer, inflammation, atherosclerosis, fibrosis, tissue remodelling and keloid disorder, preferably wherein the cancer is selected from the group consisting of breast cancer, pancreatic cancer, small intestine cancer, colon cancer, multi-drug resistant colon cancer, rectal cancer, colorectal cancer, metastatic colorectal cancer, lung cancer, non-small cell lung cancer, head and neck cancer, ovarian cancer, hepatocellular cancer, oesophageal cancer, hypopharynx cancer, nasopharynx cancer, larynx cancer, myeloma cells, bladder cancer, cholangiocarcinoma, clear cell renal carcinoma, neuroendocrine tumour, oncogenic osteomalacia, sarcoma, CUP (carcinoma of unknown primary), thymus cancer, desmoid tumours, glioma, astrocytoma, cervix cancer and prostate cancer; preferably wherein the compound has a prolonged residence at the disease site at a therapeutically or diagnostically relevant level, preferably beyond 1 h, more preferably beyond 6 h post injection.

### Treatment

The compounds described herein may be used to treat disease. The treatment may be therapeutic and/or prophylactic treatment, with the aim being to prevent, reduce or stop an undesired physiological change or disorder. The treatment may prolong survival as compared to expected survival if not receiving treatment. The disease that is treated by the compound may be any disease that might benefit from treatment. This includes chronic and acute disorders or diseases including those pathological conditions which predispose to the disorder.

The term "cancer" and "cancerous" is used in its broadest sense as meaning the physiological condition in mammals that is typically characterized by unregulated cell growth. A tumour comprises one or more cancerous cells. When treating cancer, the therapeutically effect that is observed may be a reduction in the number of cancer cells; a reduction in tumour size; inhibition or retardation of cancer cell infiltration into peripheral organs; inhibition of tumour growth; and/or relief of one or more of the symptoms associated with the cancer.

In animal models, efficacy may be assessed by physical measurements of the tumour during the treatment, and/or by determining partial and complete remission of the cancer. For cancer therapy, efficacy can, for example, be measured by assessing the time to disease progression (TTP) and/or determining the response rate (RR).

Particularly preferred embodiments for the methods of treatment related to the present invention are shown in the appended claims.

Herein disclosed are also methods for treatment of the human or animal body, e.g., by surgery or therapy, or diagnostic method practised on the human or animal body, the methods involving a step of administering a therapeutically or diagnostically effective amount of a compound or a pharmaceutical composition as described herein to a subject in need thereof. More specifically, herein disclosed are methods for treatment, e.g., by therapy or prophylaxis, of a subject suffering from or having risk for a disease or disorder; or by guided surgery practised on a subject suffering from or having risk for a disease or disorder; method for diagnosis of a disease or disorder, e.g., diagnostic method practised on the human or animal body and/or involving a nuclear medicine imaging technique, such as Positron Emission Tomography (PET) or Single Photon Emission Computed Tomography (SPECT); method for targeted delivery of a therapeutic or diagnostic agent to a subject suffering from or having risk for a disease or disorder. In the aforementioned methods, said disease or disorder may be independently selected from cancer, inflammation, atherosclerosis, fibrosis, tissue remodelling and keloid disorder, preferably wherein the cancer is selected from the group consisting of breast cancer, pancreatic cancer, small intestine cancer, colon cancer, multi-drug resistant colon cancer, rectal cancer, colorectal cancer, metastatic colorectal cancer, lung cancer, non-small cell lung cancer, head and neck cancer, ovarian cancer, hepatocellular cancer, oesophageal cancer, hypopharynx cancer, nasopharynx cancer, larynx cancer, myeloma cells, bladder cancer, cholangiocarcinoma, clear cell renal carcinoma, neuroendocrine tumour, oncogenic osteomalacia, sarcoma, CUP (carcinoma of unknown primary), thymus cancer, desmoid tumours, glioma, astrocytoma, cervix cancer, skin cancer, kidney cancer and prostate cancer. When used in the methods disclosed herein, the compound has a prolonged residence at the disease site at a therapeutically or diagnostically relevant level, preferably beyond 1 h, more preferably beyond 6 h post injection.

### Pharmaceutical compositions

The compounds described herein may be in the form of pharmaceutical compositions which may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition may be formulated to be administered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be administered by a number of routes.

If the agent is to be administered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions may be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or the pharmaceutical compositions can be injected parenterally, for example, intravenously, intramuscularly or subcutaneously. For parenteral administration, the compositions may be best used in the form of a sterile aqueous solution which may contain other substances, for example, enough salts or monosaccharides to make the solution isotonic with blood. For buccal or sublingual administration, the compositions may be administered in the form of tablets or lozenges which can be formulated in a conventional manner.

The compound of the present invention may be administered in the form of a pharmaceutically acceptable or active salt. Pharmaceutically-acceptable salts are well known to those skilled in the art, and for example, include those mentioned by Berge et al, in J.Pharm.Sci., 66, 1-19 (1977). Salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)) salts.

The routes for administration (delivery) may include, but are not limited to, one or more of oral (e.g. as a tablet, capsule, or as an ingestable solution), topical, mucosal (e.g. as a nasal spray or aerosol for inhalation), nasal, parenteral (e.g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, vaginal, epidural, sublingual.

Typically, a physician will determine the actual dosage which will be most suitable for an individual subject. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

The formulations may be packaged in unit-dose or multi-dose containers, for example sealed ampoules and vials, and may be stored in a freeze-dried (lyophilized) condition requiring only the addition of the sterile liquid carrier, for example water, for administration. Extemporaneous injection solutions and suspensions are prepared from sterile powders, granules and tablets of the kind previously described. Exemplary unit dosage formulations contain a daily dose or unit daily sub-dose, or an appropriate fraction thereof, of the active ingredient.

### Precursor compounds

In one aspect of the invention, herein disclosed is a compound, its individual diastereoisomers, its hydrates, its solvates, its crystal forms, its individual tautomers or a salt thereof, wherein the compound (precursor compound) comprises two moieties **A** and a reactive moiety **L** capable of reacting and forming a covalent bond with a conjugation partner. Upon conjugation (i.e., reacting and forming a covalent bond), the former precursor compound is bound to the former conjugation partner, which in turn to a payload moiety **C.** The conjugation partner can be an atom, a molecule, a particle, a therapeutic agent and/or diagnostic agent. Preferably, the conjugation is a therapeutic agent and/or diagnostic agent, and can correspond to the payload moieties already described in detail above with respect to the conjugates according to the invention.

Each moiety **A** preferably has the structure **A¹** or **A²** as previously defined.

Preferably, the precursor compound is represented by the following Formula **VI:** 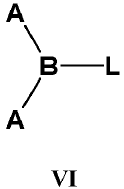 wherein **B** is a covalent bond or a multifunctional moiety covalently attaching the moieties **A** to **L.**

Preferably, **L** is capable of forming, upon reacting, an amide, ester, carbamate, hydrazone, thiazolidine, methylene alkoxy carbamate, disulphide, alkylene, cycloalkylene, arylalkylene, heteroarylalkylene, heteroalkylene, heterocycloalkylene, alkenylene, cycloalkenylene, arylalkenylene, heteroarylalkenylene, heteroalkenylene, heterocycloalenkylene, alkynylene, heteroalkynylene, arylene, heteroarylene, aminoacyl, oxyalkylene, aminoalkylene, diacid ester, dialkylsiloxane, amide, thioamide, thioether, thioester, ester, carbamate, hydrazone, thiazolidine, methylene alkoxy carbamate, disulfide, vinylene, imine, imidamide, phosphoramide, saccharide, phosphate ester, phosphoramide, carbamate, dipeptide, tripeptide or tetrapeptide linking group; and/or

More preferably, the precursor compound has the structure: 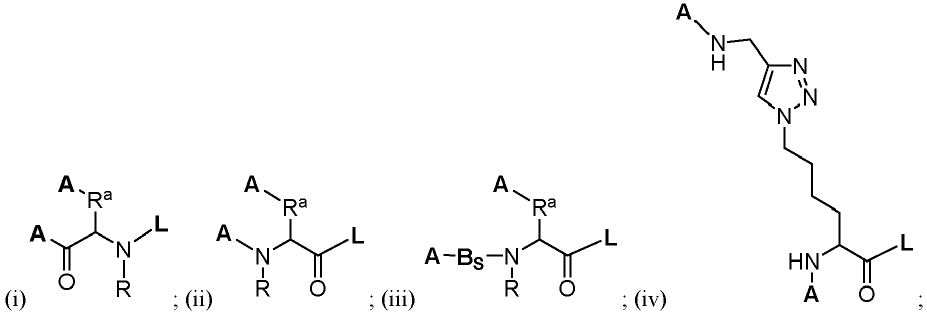 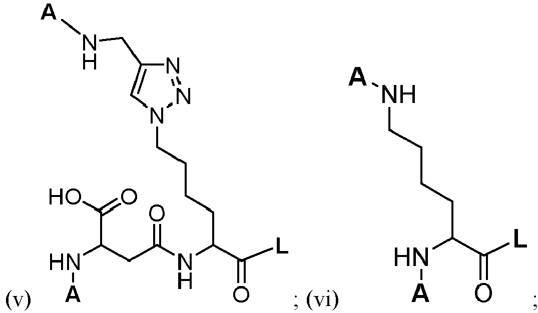 or

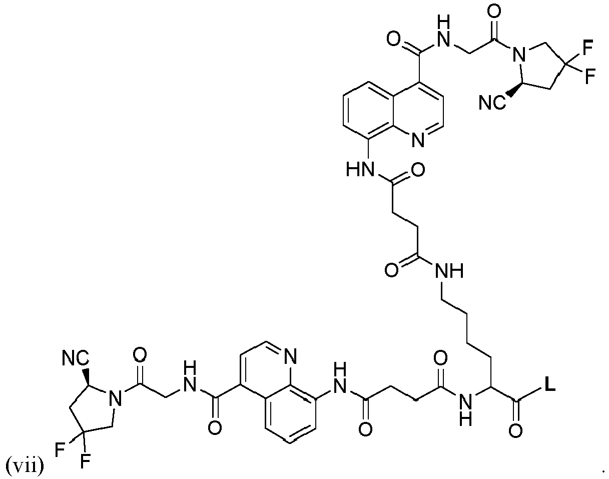

Moiety **B** preferably has a structure as described in detail above with respect to the conjugates according to the invention.

Moiety **L** is preferably capable of forming, upon reacting, an amide, ester, carbamate, hydrazone, thiazolidine, methylene alkoxy carbamate, disulphide, alkylene, cycloalkylene, arylalkylene, heteroarylalkylene, heteroalkylene, heterocycloalkylene, alkenylene, cycloalkenylene, arylalkenylene, heteroarylalkenylene, heteroalkenylene, heterocycloalenkylene, alkynylene, heteroalkynylene, arylene, heteroarylene, aminoacyl, oxyalkylene, aminoalkylene, diacid ester, dialkylsiloxane, amide, thioamide, thioether, thioester, ester, carbamate, hydrazone, thiazolidine, methylene alkoxy carbamate, disulfide, vinylene, imine, imidamide, phosphoramide, saccharide, phosphate ester, phosphoramide, carbamate, dipeptide, tripeptide or tetrapeptide linking group. As will be appreciated by a person skilled in the art, multiple possibilities exist how to provide a reactive group capable of reacting with a conjugation partner to form a linking group according to the aforementioned list, and they are all encompassed by the present disclosure.

The moiety **B** may be cleavable or non-cleavable, multifunctional moiety which can be used to link one or more reactive and/or binder moieties to form the conjugate precursor of the invention. In some embodiments, the structure of the compound comprises, independently, more than one moieties A, preferably 2, 3, 4, 5, 6, 7, 8, 9 or 10 moieties A; and/or more than one moieties L, preferably 2, 3, 4, 5, 6, 7, 8, 9 or 10 moieties L per molecule. Preferably, the structure of the compound comprises 2 moieties A and 1 moiety L; or 1 moiety A and 2 moieties L per molecule.

Moiety L is preferably selected from: H, NH₂, N₃, COOH, SH, Hal, 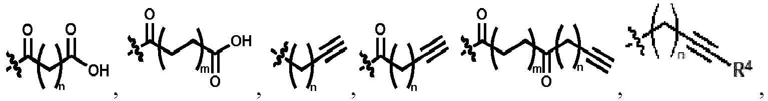 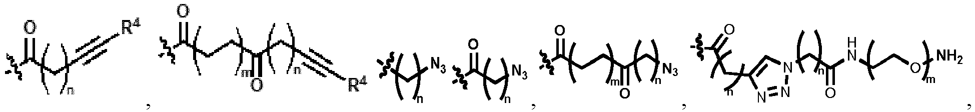 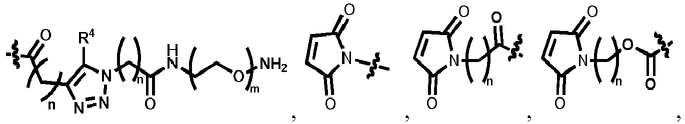 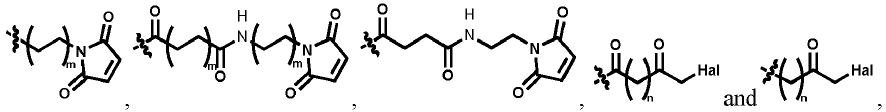 wherein each *n* is, independently, 0, 1, 2, 3, 4, 5, 6, 7, 8, 9 or 10; each *m* is, independently, 0, 1, 2, 3, 4 or 5; each Hal is F, Cl, Br or I; and each R⁴ is, independently selected from carboxy, alkyl, cycloalkyl, aryl and heteroaryl, wherein each of the foregoing is substituted or unsubstituted, halogen, and cyano.

Preferred precursor compounds are those having a structure listed below, their individual diastereoisomers, hydrates, solvates, crystal forms, individual tautomers or salts thereof:

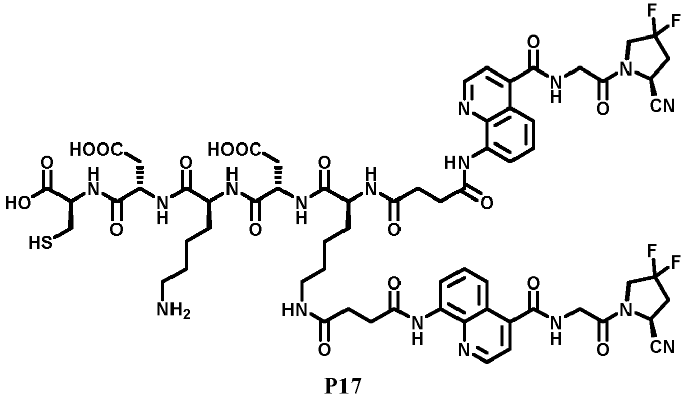

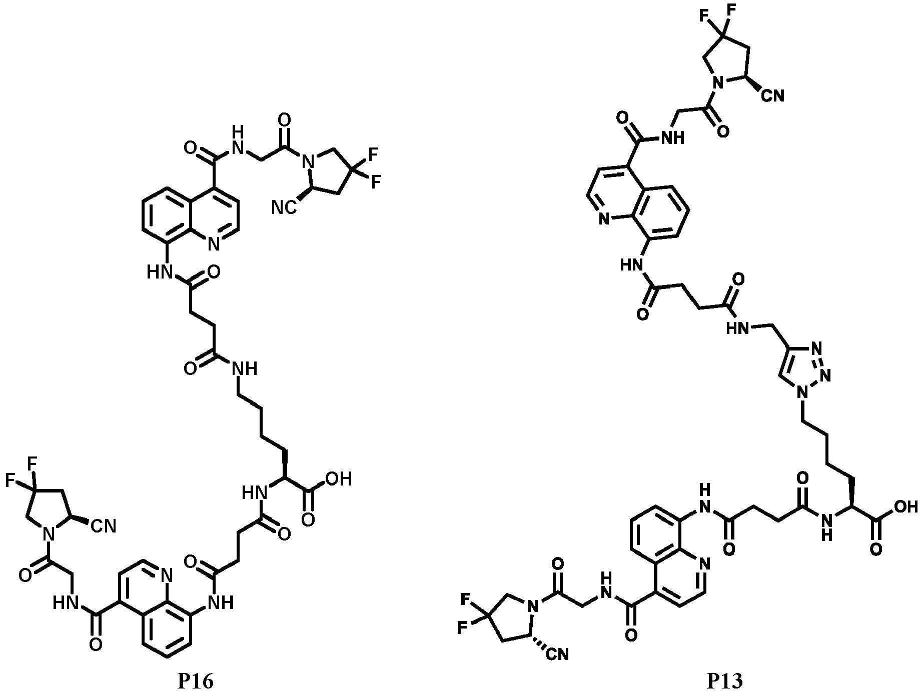

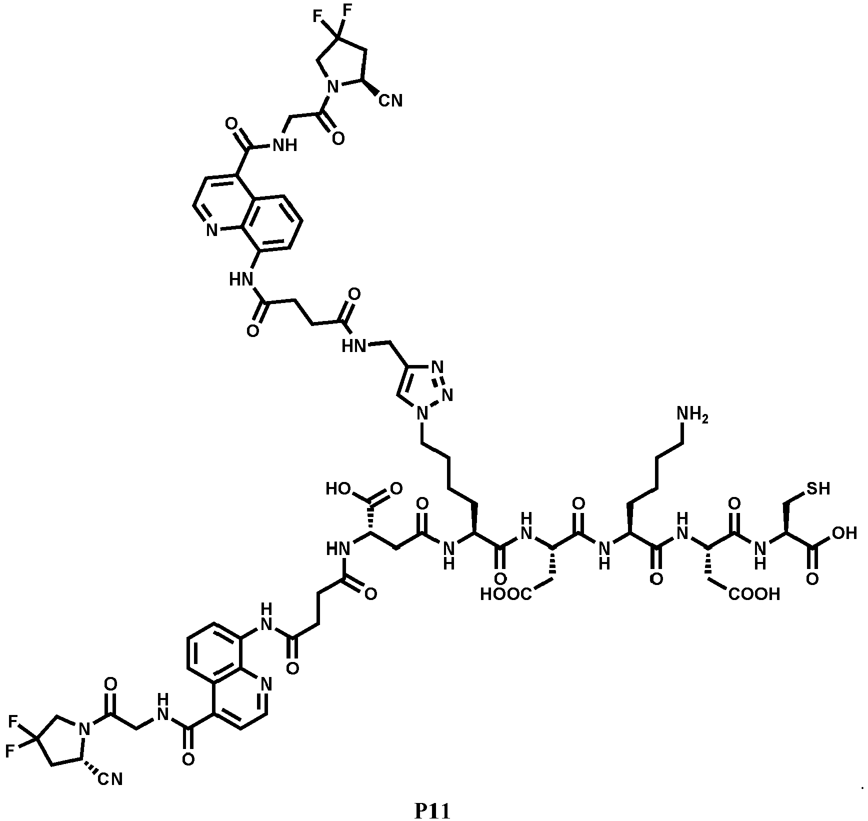

In all structures, unless otherwise specified, all groups and variables are as defined further above throughout the present disclosure.

### Methods for preparing a conjugate

In one aspect of the invention, herein disclosed is a method for preparing a conjugate comprising the step of conjugating with a precursor compound as described above with a conjugation partner. Preferably, the precursor compound is conjugated to the conjugation partner by reacting therewith to form a covalent bond. Preferably, the thus obtained conjugate is a conjugate compound as described elsewhere in the present specification.

The conjugation partner can be an atom, a molecule, a particle, a therapeutic agent and/or diagnostic agent. Preferably, the conjugation is a therapeutic agent and/or diagnostic agent, and can correspond to the payload moieties already described in detail above with respect to the conjugates according to the invention.

Preferably, the method further comprises formulating the conjugate as a pharmaceutical composition or as a diagnostic composition. The pharmaceutical or diagnostic compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of carrier, excipient or diluent can be selected with regard to the intended route of administration and standard pharmaceutical practice. The pharmaceutical or diagnostic compositions may comprise as - or in addition to - the carrier, excipient or diluent any suitable binder(s), lubricant(s), suspending agent(s), coating agent(s), solubilising agent(s). All formulation details and aspects disclosed above in the section "Pharmaceutical compositions" fully apply here too.

### General techniques

The practice of the present invention employs, unless otherwise indicated, conventional methods of chemistry, biochemistry, molecular biology, cell biology, genetics, immunology and pharmacology, known to those of skill of the art. Such techniques are explained fully in the literature. See, e. g. , Gennaro, A. R., ed. (1990) Remington's Pharmaceutical Sciences, 18th ed., Mack Publishing Co.; Hardman, J. G., Limbird, L. E., and Gilman, A. G., eds. (2001) The Pharmacological Basis of Therapeutics, 10th ed., McGraw-Hill Co.; Colowick, S. et al., eds., Methods In Enzymology, Academic Press, Inc.; Weir, D. M. , and Blackwell, C. C., eds. (1986) Handbook of Experimental Immunology, Vols. I-IV, Blackwell Scientific Publications; Maniatis, T. et al., eds. (1989) Molecular Cloning: A Laboratory Manual, 2nd edition, Vols. I-III, Cold Spring Harbor Laboratory Press; Ausubel, F. M. et al., eds. (1999) Short Protocols in Molecular Biology, 4th edition, John Wiley & Sons; Ream et al., eds. (1998) Molecular Biology Techniques: An Intensive Laboratory Course, Academic Press; Newton, C. R., and Graham, A., eds. (1997) PCR (Introduction to Biotechniques Series), 2nd ed., Springer Verlag.

### Chemical synthesis

The compounds described herein may be prepared by chemical synthesis techniques. It will be apparent to those skilled in the art that sensitive functional groups may need to be protected and deprotected during synthesis of a compound. This may be achieved by conventional techniques, for example as described in "Protective Groups in Organic Synthesis" by T W Greene and P G M Wuts, John Wiley and Sons Inc. (1991), and by P.J.Kocienski, in "Protecting Groups", Georg Thieme Verlag (1994). It is possible during some of the reactions that any stereocentres present could, under certain conditions, be epimerised, for example if a base is used in a reaction with a substrate having an optical centre comprising a base-sensitive group. It should be possible to circumvent potential problems such as this by choice of reaction sequence, conditions, reagents, protection/deprotection regimes, etc. as is well-known in the art.

### Definitions

**Antibody.** The term "antibody" is used in its broadest sense and covers monoclonal antibodies, polyclonal antibodies, dimers, multimers, multispecific antibodies (e.g., bispecific antibodies), veneered antibodies, antibody fragments and small immune proteins (SIPs) (see Int. J. Cancer (2002) 102, 75-85). An antibody is a protein generated by the immune system that is capable of recognizing and binding to a specific antigen. A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, *i*.*e*. a molecule that contains an antigen binding site that immunospecifically binds an antigen of a target of interest or part thereof. The antibodies may be of any type - such as IgG, IgE, IgM, IgD, and IgA) - any class - such as IgG1, IgG2, IgG3, IgG4, IgA1 and IgA2 - or subclass thereof. The antibody may be or may be derived from murine, human, rabbit or from other species.

**Antibody fragments.** The term "antibody fragment" refers to a portion of a full length antibody, generally the antigen binding or variable region thereof. Examples of antibody fragments include, but are not limited to, Fab, Fab', F(ab')₂, and Fv fragments; diabodies; linear antibodies; single domain antibodies, including dAbs, camelid V_{HH} antibodies and the IgNAR antibodies of cartilaginous fish. Antibodies and their fragments may be replaced by binding molecules based on alternative non-immunoglobulin scaffolds, peptide aptamers, nucleic acid aptamers, structured polypeptides comprising polypeptide loops subtended on a non-peptide backbone, natural receptors or domains thereof.

**Derivative.** A derivative includes the chemical modification of a compound. Examples of such modifications include the replacement of a hydrogen by a halo group, an alkyl group, an acyl group or an amino group and the like. The modification may increase or decrease one or more hydrogen bonding interactions, charge interactions, hydrophobic interactions, van der Waals interactions and/or dipole interactions.

**Analog.** This term encompasses any enantiomers, racemates and stereoisomers, as well as all pharmaceutically acceptable salts and hydrates of such compounds.

Unless otherwise stated, the following definitions apply to chemical terms used in connection of compounds of the invention and compositions containing such compounds.

**Alkyl** refers to a branched or unbranched saturated hydrocarbyl radical. Suitably, the alkyl group comprises from 1 to 100, preferably 3 to 30, carbon atoms, more preferably from 5 to 25 carbon atoms. Preferably, alkyl refers to methyl, ethyl, propyl, butyl, pentyl, or hexyl.

**Alkenyl** refers to a branched or unbranched hydrocarbyl radical containing one or more carbon-carbon double bonds. Suitably, the alkenyl group comprises from 2 to 30 carbon atoms, preferably from 5 to about 25 carbon atoms.

**Alkynyl** refers to a branched or unbranched hydrocarbyl radical containing one or more carbon-carbon triple bonds. Suitably, the alkynyl group comprises from about 3 to about 30 carbon atoms, for example from about 5 to about 25 carbon atoms.

**Halogen** refers to fluorine, chlorine, bromine or iodine, preferably fluorine or chlorine.

**Cycloalkyl** refers to an alicyclic moiety, suitably having 3, 4, 5, 6, 7 or 8 carbon atoms. The group may be a bridged or polycyclic ring system. More often cycloalkyl groups are monocyclic. This term includes reference to groups such as cyclopropyl, cyclobutyl, cyclopentyl, cyclohexyl, norbornyl, bicyclo[2.2.2]octyl and the like.

**Aryl** refers to an aromatic carbocyclic ring system, suitably comprising 6, 7, 8, 9, 10, 11, 12, 13, 14, 15 or 16 ring carbon atoms. Aryl may be a polycyclic ring system, having two or more rings, at least one of which is aromatic. This term includes reference to groups such as phenyl, naphthyl fluorenyl, azulenyl, indenyl, anthryl and the like.

The prefix **(hetero)** herein signifies that one or more of the carbon atoms of the group may be substituted by nitrogen, oxygen, phosphorus, silicon or sulfur. Heteroalkyl groups include for example, alkyloxy groups and alkythio groups. Heterocycloalkyl or heteroaryl groups herein may have from 3, 4, 5, 6, 7, 8, 9, 10, 11 , 12, 13, 14, 15 or 16 ring atoms, at least one of which is selected from nitrogen, oxygen, phosphorus, silicon and sulfur. In particular, a 3- to 10-membered ring or ring system and more particularly a 5- or 6-membered ring, which may be saturated or unsaturated. For example, selected from oxiranyl, azirinyl, 1,2-oxathiolanyl, imidazolyl, thienyl, furyl, tetrahydrofuryl, pyranyl, thiopyranyl, thianthrenyl, isobenzofuranyl, benzofuranyl, chromenyl, 2H-pyrrolyl, pyrrolyl, pyrrolinyl, pyrrolidinyl, imidazolyl, imidazolidinyl, benzimidazolyl, pyrazolyl, pyrazinyl, pyrazolidinyl, thiazolyl, isothiazolyl, dithiazolyl, oxazolyl, isoxazolyl, pyridyl, pyrazinyl, pyrimidinyl, piperidyl, piperazinyl, pyridazinyl, morpholinyl, thiomorpholinyl, especially thiomorpholino, indolizinyl, 1,3-Dioxo-1,3-dihydro-isoindolyl, 3H-indolyl, indolyl, benzimidazolyl, cumaryl, indazolyl, triazolyl, tetrazolyl, purinyl, 4H-quinolizinyl, isoquinolyl, quinolyl, tetrahydroquinolyl, tetrahydroisoquinolyl, decahydroquinolyl, octahydroisoquinolyl, benzofuranyl, dibenzofuranyl, benzothiophenyl, dibenzothiophenyl, phthalazinyl, naphthyridinyl, quinoxalyl, quinazolinyl, quinazolinyl, cinnolinyl, pteridinyl, carbazolyl, [beta]-carbolinyl, phenanthridinyl, acridinyl, perimidinyl, phenanthrolinyl, furazanyl, phenazinyl, phenothiazinyl, phenoxazinyl, chromenyl, isochromanyl, chromanyl, 3,4-dihydro-2H-isoquinolin-1-one, 3,4-dihydro-2H-isoquinolinyl, and the like.

**"Substituted"** signifies that one or more, especially up to 5, more especially 1, 2 or 3, of the hydrogen atoms in said moiety are replaced independently of each other by the corresponding number of substituents. The term "optionally substituted" as used herein includes substituted or unsubstituted. It will, of course, be understood that substituents are only at positions where they are chemically possible, the person skilled in the art being able to decide (either experimentally or theoretically) without inappropriate effort whether a particular substitution is possible. For example, amino or hydroxy groups with free hydrogen may be unstable if bound to carbon atoms with unsaturated (e.g. olefinic) bonds. Preferably, the term "substituted" signifies one or more, especially up to 5, more especially 1, 2 or 3, of the hydrogen atoms in said moiety are replaced independently of each other by the corresponding number of substituents selected from OH, SH, NH₂, halogen, cyano, carboxy, alkyl, cycloalkyl, aryl and heteroaryl. Additionally, the substituents described herein may themselves be substituted by any substituent, subject to the aforementioned restriction to appropriate substitutions as recognised by the skilled person. Preferably, any of the aforementioned substituents may be further substituted by any of the aforementioned substituents, each of which may be further substituted by any of the aforementioned substituents.

Substituents may suitably include halogen atoms and halomethyl groups such as CF₃ and CCl₃; oxygen containing groups such as oxo, hydroxy, carboxy, carboxyalkyl, alkoxy, alkoyl, alkoyloxy, aryloxy, aryloyl and aryloyloxy; nitrogen containing groups such as amino, alkylamino, dialkylamino, cyano, azide and nitro; sulfur containing groups such as thiol, alkylthiol, sulfonyl and sulfoxide; heterocyclic groups which may themselves be substituted; alkyl groups, which may themselves be substituted; and aryl groups, which may themselves be substituted, such as phenyl and substituted phenyl. Alkyl includes substituted and unsubstituted benzyl.

Where two or more moieties are described as being "each independently" selected from a list of atoms or groups, this means that the moieties may be the same or different. The identity of each moiety is therefore independent of the identities of the one or more other moieties.

### MATERIAL & METHODS

### General remarks and procedures

Yields refer to chromatographically purified compounds, unless specified otherwise.

Mass Spectrometry (LC-ESI-MS) spectra were recorded on an Agilent 6100 Series Single Quadrupole MS System combined with an Agilent 1200 Series LC System, using an InfinityLab Poroshell 120 EC-C18 column, 4.6 mm × 56 mm at a flow rate of 2 mL min⁻¹ with linear gradients of solvents A and B (A = Millipore water with 0.1% formic acid [FA], B = MeCN with 0.1% formic acid [FA]).

High-Resolution Mass Spectrometry (HRMS) spectra and analytical Reversed-Phase Ultra Performance Liquid Chromatography (UPLC) were recorded on a Waters Xevo G2-XS QTOF coupled to a Waters Acquity UPLC H-Class System with PDA UV detector, using a ACQUITY UPLC BEH C18 Column, 130 Å, 1.7 µm, 2.1 mm × 50 mm at a flow rate of 0.6 mL min⁻¹ with linear gradients of solvents A and B (A = Millipore water with 0.1% FA, B = MeCN with 0.1% FA).

Preparative reversed-phase high-pressure liquid chromatography (RP-HPLC) was performed on an Agilent 1200 Series System, using a Phenomenex Gemini^{®} 5 µm NX-C18 semipreparative column, 110 Å, 150 mm × 10 mm at a flow rate of 5 mL min⁻¹ with linear gradients of solvents A and B (A = Millipore water with 0.1% trifluoroacetic acid [TFA], B = MeCN with 0.1% trifluoroacetic acid [TFA]).

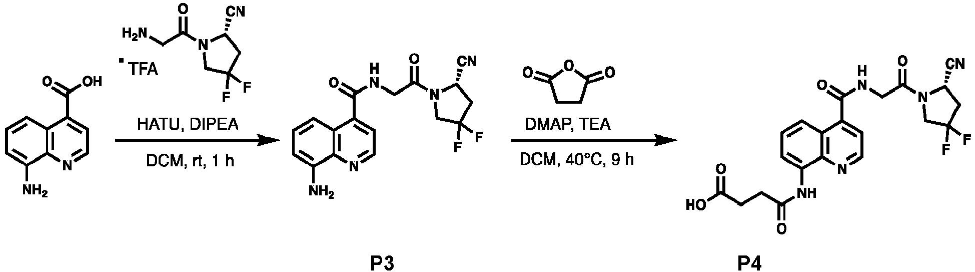

### Synthesis of ESV6-Succinic-COOH (P4)

**Step 1: (S)-8-amino-N-(2-(2-cyano-4,4-difluoropyrrolidin-1-yl)-2-oxoethyl)quinoline-4-carboxamide (P3).** Commercially available 8-amino-quinoline-4-carboxylic acid (19.0 mg, 100 µmol, 1.0 eq), DIPEA (70.0 µL, 400 µmol, 4.0 eq) and HATU (38.0 mg, 100 µmol, 1.0 eq) were dissolved in a 1:1 DCM/DMF mixture (2 mL). After 15 min a solution of (S)-1-(2-aminoacetyl)-4,4- difluoropyrrolidine-2-carbonitrile trifluoroacetate (30.3 mg, 100 µmol, 1.0 eq) in DCM was added. The reaction mixture was stirred for 1 h at room temperature, washed with water, dried over Na₂SO₄, filtered and concentrated to obtain a brown crude as sticky oil. The residue was purified by flash chromatography (DCM/MeOH from 91:1 to 90:10) to yield the pure product as a brownish oil (24.8 mg, 68.9 µmol, 69% yield). MS (ES⁺) *m*/*z* 360 (M+H)⁺.

**Step 2: (S)-4-((4-((2-(2-cyano-4,4-difluoropyrrolidin-1-yl)-2-oxoethyl)carbamoyl)quinolin-8-yl)amino)4-oxobutanoic acid (P4).** Triethylamine (20.8 µL, 150 µmol, 2.0 eq) and 4-dimethylaminopyridine (0.91 mg, 10.0 µmol, 0.1 eq) were added to a cooled solution (0 °C) of **P3** (26.8 mg, 70.0 µmol, 1.0 eq) in DCM, followed by a dropwise addition of succinic anhydride (15.0 mg, 150 µmol, 2.0 eq). The reaction mixture was allowed to warm to room temperature. The reaction mixture was placed in a preheated 40 °C oil bath until full conversion was observed. The solvent was evaporated and the residue was purified by RP-HPLC to yield the pure product as a white powder (9.42 mg, 20.0 µmol, 28% yield). MS (ES⁺) *m*/*z* 460 (M+H)⁺.

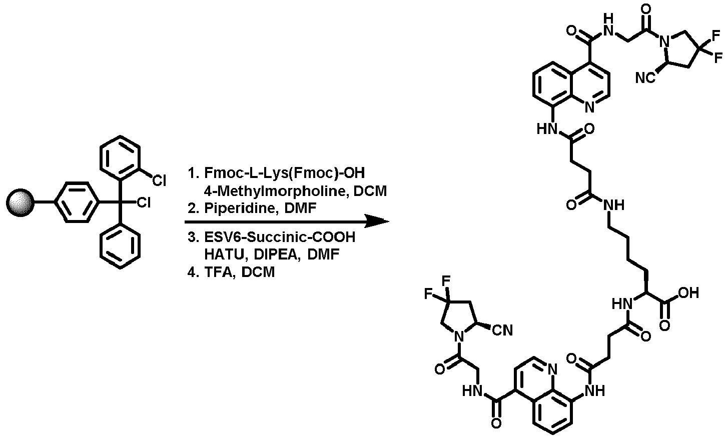

**Synthesis of Bi-ESV6-COOH (P16).** To a solid-phase synthesis syringe, 2-chlorotrityl resin (300 mg) was added and then swollen with dry DCM for 15 min. Fmoc-L-Lys(Fmoc)-OH (89 mg, 0.15 mmol, 1 eq.) and 4-Methylmorpholine (45 µL, 0.40 mmol, 2.7 eq.) were sequentially added to the resin and the mixture was allowed to react for 3 h. Next, a capping step with methanol/ 4-Methylmorpholine / DCM (1:2:7 ratio, 5 mL, 30 min) was carried out, following by a wash with DMF and Fmoc-removal with 20% solution of Piperidine in DMF (10 mL). The resin was then treated with a solution of ESV6-Succinic-COOH **(P4,** 137 mg, 0.300 mmol, 2.0 eq.), HATU (86 mg, 0.22 mmol, 1.5 eq.) and DIPEA (97 µL, 0.75 mmol, 5.0 eq.) in DMF (5 mL) for 1 h. After multiple washing with DMF, the resin was submitted to the cleavage with 30% solution of TFA in DCM (10 mL) for 1 h. The cleaved solution was recovered, concentrated under *vacuo* and purified by RP-Chromatography (gradient: water/acetonitrile + 0.1% FA 98:2 to 0:100 in 45 min). The fractions were collected and lyophilized to afford Bi-ESV6-COOH **(P16)** as a white solid (30 mg, 0.029 mmol, 19% yield). MS(ES+) m/z 1029.3 (M+H)⁺

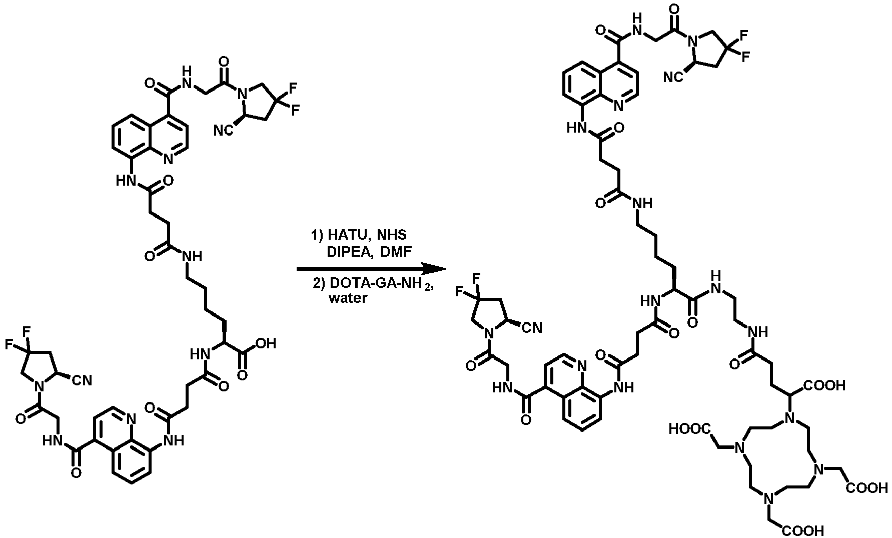

**Synthesis of Bi-ESV6-DOTAGA** (1) To a solution of Bi-ESV6-COOH (P16, 12 mg, 0.012 mmol) in DMF (500 µL), N-hydroxysuccinimmide (2.0 mg, 0.017 mmol, 1.5 eq), HATU (6.7 mg, 0.017 mmol, 1.5 eq) and DIPEA (8 µL 0.05 mmol, 4.0 eq) were added. After 30 min, a solution of DOTA-GA-NH2 (12 mg, 0.023 mmol, 2.0 eq) in water (500 µL) was added dropwise. The reaction mixture was stirred for further 30 min at room temperature, then purified by RP-HPLC (Agilent 1200 series system equipped with Synergi 4µm Polar-RP 80Å 10 × 150 mm C18 column using a gradient of 90:10 to 0:100 water/acetonitrile + 0.1% TFA in 12 min). The fractions were collected and lyophilized to afford a white solid (10 mg, 0.007 mmol, 56% yield). MS(ES+) m/z 1530.5 (M+H)⁺.

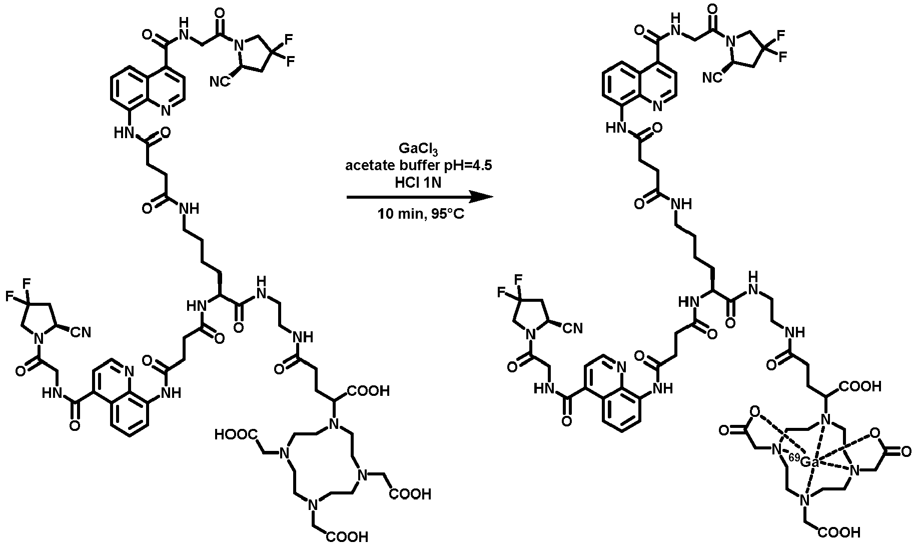

**Synthesis of Bi-ESV6-DOTAGA-⁶⁹Ga (6a).** Bi-ESV6-DOTAGA (1, 4.0 mg, 2.6 µmol, 1 eq.) was dissolved in acetate buffer, pH = 4.5 (2.0 mL). Subsequently a solution of GaCl₃ (23 mg, 26 µmol, 10 eq.) dissolved in 1 N HCl (2.0 mL) was added. The reaction was stirred at 90°C for 10 min, then cooled down to r. t. and purified via RP-HPLC (Agilent 1200 series system equipped with Synergi 4µm Polar-RP 80Å 10 × 150 mm C18 column using a gradient of 90:10 to 50:50 water/acetonitrile + 0.1% TFA in 7 min). The desired fractions were collected and lyophilized to afford a pale-yellow solid. (2.5 mg, 60%).

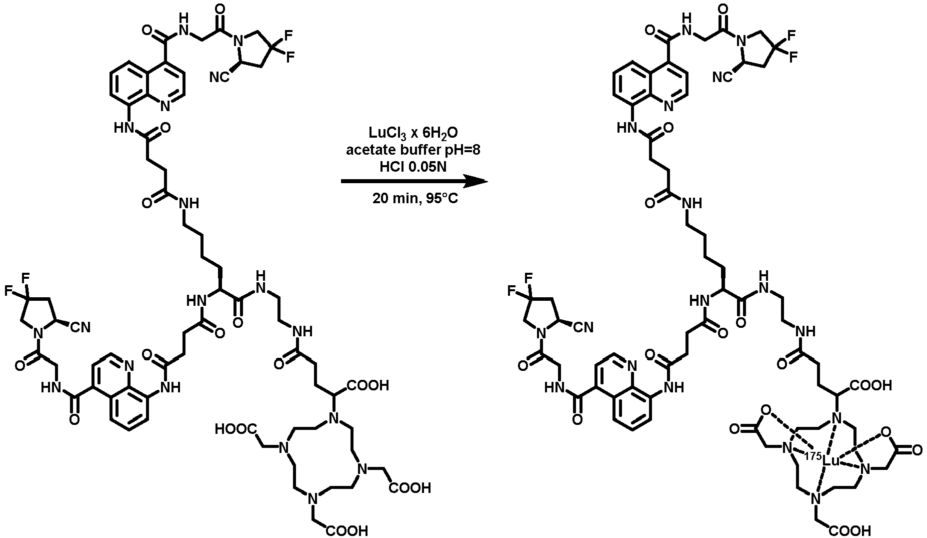

**Synthesis of Bi-ESV6-DOTAGA-¹⁷⁵Lu** (5a). Bi-ESV6-DOTAGA (1, 4.0 mg, 2.6 µmol, 1 eq.) was dissolved in acetate buffer, pH = 8 (300 µL). Subsequently a solution of LuCl₃ hexahydrate (2.0 mg, 5.2 µmol, 2 eq.) dissolved in 0.05 N HCl (1.50 mL) was added. The reaction was stirred at 90°C for 20 min, then cooled down to r. t. and purified via RP-HPLC (Agilent 1200 series system equipped with Synergi 4µm Polar-RP 80Å 10 × 150 mm C18 column using a gradient of 90:10 to 50:50 water/acetonitrile + 0.1% TFA in 7 min). The desired fractions were collected and lyophilized to afford a pale-yellow solid. (2.2 mg, 49%).

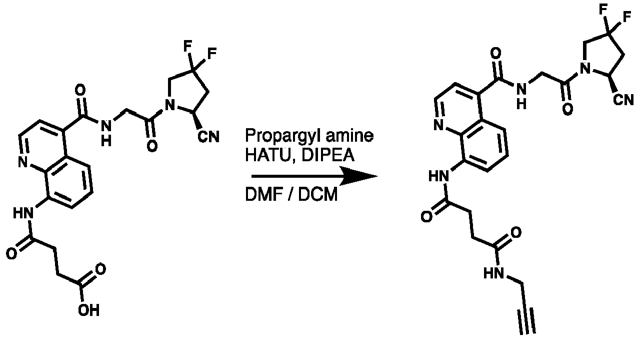

**Synthesis of P10.** (*S*)-4-((4-((2-(2-cyano-4,4-difluoropyrrolidin-1-yl)-2-oxoethyl)carbamoyl)quinolin-8-yl)amino)-4-oxobutanoic acid **(P4,** 50 mg, 0.11 mmol, 1 eq), propargylamine (7 mg, 0.13 mmol, 1.2 eq) and HATU (49 mg, 0.13 mmol, 1.2 eq) were dissolved in 2 mL of DCM and 100 µL of DMF. DIPEA (56 mg, 0.44 mmol, 4 eq) was added dropwise and the reaction was stirred for 30 minutes at room temperature. Water was added, separated from organic layer and then extracted three times with DCM. The crude was dried over sodium sulfate, filtered and evaporated. The crude was purified via chromatography (DCM/MeOH 100:0 to 95:5 in 10 min) to afford a dark oil (32 mg. 0.0638 mmol, 58%). MS(ES+) m/z 495.47 (M+1H)¹⁺

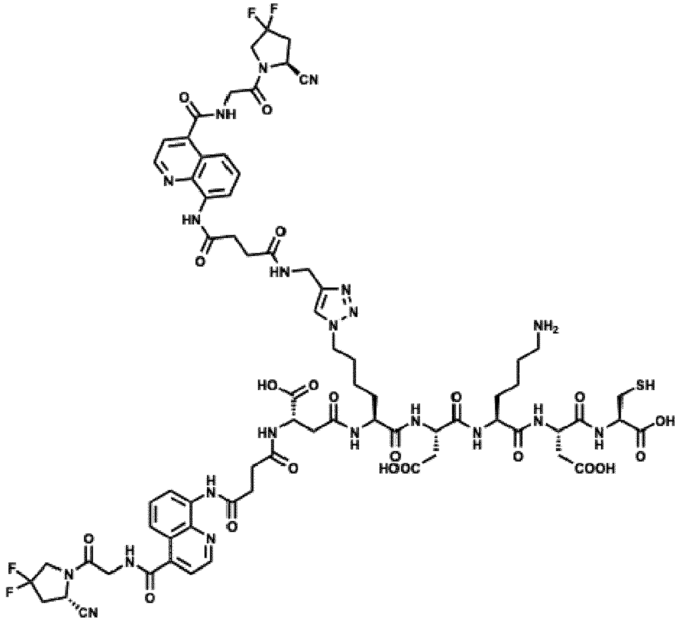

**Synthesis of "Bi-ESV6-triazole" (P11)** Commercially available pre-loaded Fmoc-Cys(Trt) on Tentagel resin (300 mg, 0.18 mmol, RAPP Polymere) was swollen in DMF (3 × 5 min × 5 mL), the Fmoc group removed with 20 % piperidine in DMF (1 × 1 min × 5 mL and 2 × 10 min × 5 mL) and the resin washed with DMF (6 × 1 min × 5 mL). The peptide was extended with Fmoc-Asp(tBu)-OH, Fmoc-Lys(NHBoc)-OH, Fmoc-Asp(tBu)-OH, Fmoc-N3-Lys, Fmoc-Asp(tBu)-OH and (*S*)-4-((4-((2-(2-cyano-4,4-difluoropyrrolidin-1-yl)-2-oxoethyl)carbamoyl)quinolin-8-yl)amino)-4-oxobutanoic acid **(P4)** in the indicated order. For this purpose, the Fmoc protected amino acid (2.0 eq), HBTU (2.0 eq), HOBt (2.0 eq) and DIPEA (4.0 eq) were dissolved in DMF (5 mL). The mixture was allowed to stand for 10 min at 0°C and then reacted with the resin for 1 h under gentle agitation. After washing with DMF (6 × 1 min × 5 mL) the Fmoc group was removed with 20 % piperidine in DMF (1 × 1 min × 5 min and 2 × 10 min × 5 mL). Deprotection steps were followed by wash steps with DMF (6 × 1 min × 5 mL) prior to coupling with the next aminoacid. **P10** (174 mg, 0.35 mmol, 2 eq), CuI (4 mg, 0.02 mmol, 0.1 eq) and TBTA (28 mg, 0.05 mmol, 0.3 eq) were dissolved in 5 mL of a mixture 1:1 DMF/THF. The peptide was cleaved from the resin with a mixture of 20 % TFA 20 in DCM at room temperature for 1 h. The solvent was removed under reduced pressure and the crude precipitated in cold diethyl ether, centrifuged, dissolved in water/ACN and purify via HPLC (Water 0.1% TFA/Acetonitrile 0.1%TFA 9.5:0.5 to 5:5 in 15 min) and lyophilized, to obtain a white solid (18 mg, 6%). MS(ES+) m/z 1687.7 (M+H)⁺

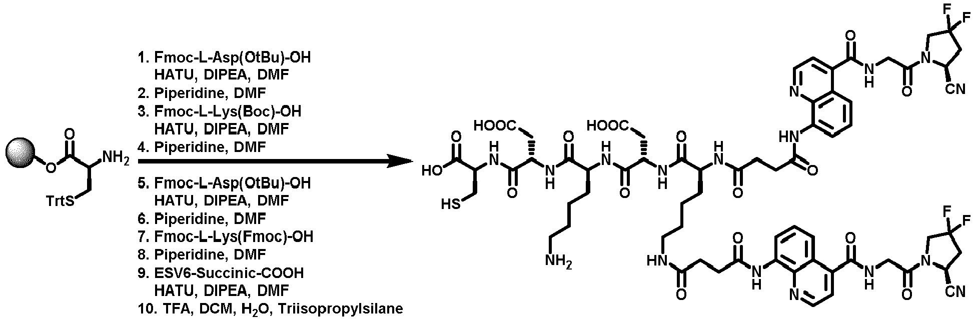

**Synthesis of Bi-ESV6-Asp-Lys-Asp-Cys-COOH (P17).** To a solid-phase synthesis syringe, H-Cys(Trt)-2-CT-polystyrene resin (900 mg) was added and then swollen with DMF for 15 min. Fmoc-L-Asp(OtBu)-OH (444 mg, 1.08 mmol, 2 eq.), HATU (411 mg, 1.08 mmol, 2 eq.) and DIPEA (377 µL, 2.16 mmol, 4 eq.) were sequentially added to the resin. The mixture was allowed to react for 2 h, then treated with a 20% solution of Piperidine in DMF (10 mL) for the Fmoc-removal and washed several times with DMF. The resin was then treated with a solution of Fmoc-L-Lys(Boc)-OH (506 mg, 1.08 mmol, 2 eq.), HATU (411 mg, 1.08 mmol, 2 eq.) and DIPEA (377 µL, 2.16 mmol, 4 eq.) in DMF (10 mL) for 2 h, following Fmoc-removal with a 20% solution of Piperidine in DMF (10 mL). After washing with DMF, a solution of Fmoc-L-Asp(OtBu)-OH (444 mg, 1.08 mmol, 2 eq.), HATU (411 mg, 1.08 mmol, 2 eq.) and DIPEA (377 µL, 2.16 mmol, 4 eq.) in DMF (10 mL) was added to the resin. After 1 h, the resin was washed and treated with a 20% solution of Piperidine in DMF (10 mL). Subsequently, Fmoc-L-Lys(Fmoc)-OH (647 mg, 1.08 mmol, 2 eq.), HATU (411 mg, 1.08 mmol, 2 eq.) and DIPEA (377 µL, 2.16 mmol, 4 eq.) and DMF (10 mL) were added to the resin and the mixture was allowed to react for 2 h, following Fmoc-removal with 20% solution of Piperidine in DMF (10 mL). Lastly, the resin was treated with a solution of ESV6-Succinic-COOH **(P4,** 992 mg, 2.16 mmol, 4 eq.), HATU (822 mg, 2.16 mmol, 4 eq.) and DIPEA (754 µL, 4.32 mmol, 8 eq.) in DMF (15 mL) for 2 h. The peptide was then cleaved from the resin using 15 mL of a solution of TFA/Triisopropylsilane/Thioanisol/water in DCM (30 : 5 : 2.5 : 2.5 : 60) for 1 h. The residue was concentrated under vacuo, resuspended in cold diethyl ether and centrifugated. The supernatant was discarded, and the pellet was dissolved in DMF and purified via RP-HPLC (Agilent 1200 series system equipped with Synergi 4µm Polar-RP 80Å 10 × 150 mm C18 column using a gradient of 90:10 to 50:50 water/acetonitrile + 0.1% TFA in 7 min). The desired fractions were collected and lyophilized to afford a white solid. (36 mg, 4.5%).

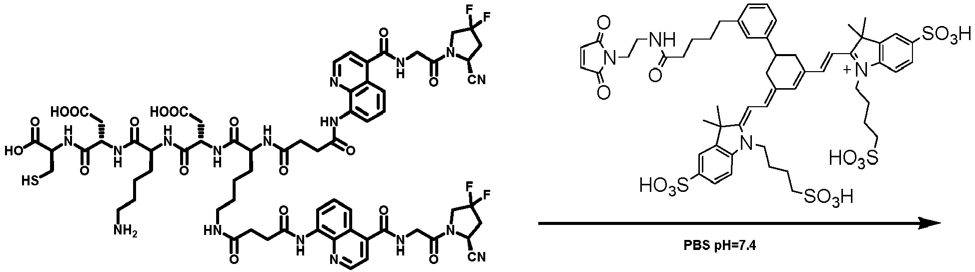

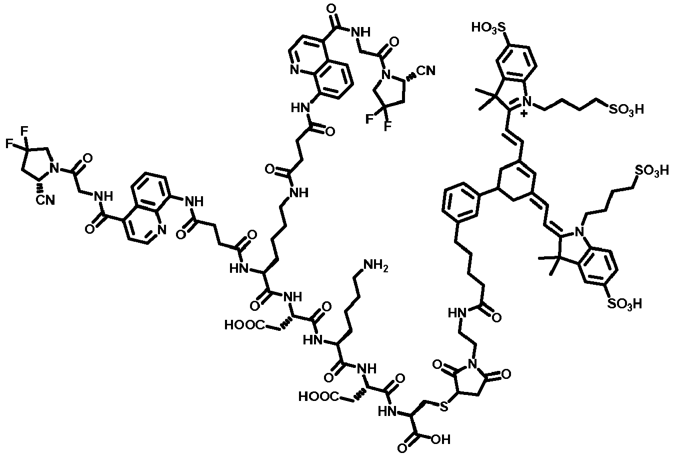

**Synthesis of Bi-ESV6-Asp-Lys-Asp-Cys-IRDye750 (18).** To a solution of Bi-ESV6-Asp-Lys-Asp-Cys-COOH (204 µg, 0.14 µmol, 1 eq.) in PBS pH=7.4 (200 µL) was added a solution of IRDye750 maleimide (150 µg, 0.12 µmol, 0.9 eq.) in DMSO (150 µL). The mixture was stirred at room temperature for 3 h and purified via RP-HPLC (Agilent 1200 series system equipped with Synergi 4µm Polar-RP 80Å 10 × 150 mm C18 column using a gradient of 90:10 to 50:50 water/acetonitrile + 0.1% TFA in 7 min). The desired fractions were collected and lyophilized to afford a blue solid collect the desired fractions and lyophilize overnight to afford a blue solid.

**Synthesis of Conjugate 47.** Bi-ESV6-Peptide **(P11**, 1 mg, 0.59 µmol, 1.0 eq) is dissolved in PBS pH 7.4 (840 µL). Maleimido-Fluorescein (0.76 mg, 1.77 µmol, 3.0 eq) is added as dry DMF solution (160 µL). The reaction is stirred for 3 h. The crude material is purified by reversed-phase HPLC (Water 0.1% TFA/Acetonitrile 0.1%TFA 9.5:0.5 to 2:8 in 20 min) and lyophilized, to obtain a yellow solid. MS(ES+) m/z 2114.7 (M+H)⁺

**Synthesis of Conjugate 48.** Bi-ESV6-Peptide **(P11**, 1 mg, 0.59 µmol, 1.0 eq) is dissolved in PBS pH 7.4 (300 µL). Alexa Fluor^{™} 488 C5 Maleimide (200 µg, 0.29 µmol, 0.5 eq) is added as dry DMSO solution (200 µL). The reaction is stirred for 3 h. The crude material is purified by reversed-phase HPLC (Water 0.1% TFA/Acetonitrile 0.1%TFA 9.5:0.5 to 2:8 in 20 min) and lyophilized, to obtain an orange solid. MS(ES+) m/z 2385. 8 (M+1H)¹⁺

**Synthesis of Conjugate 49.** Bi-ESV6-Peptide **(P11**, 1 mg, 0.59 µmol, 1.0 eq) is dissolved in PBS pH 7.4 (840 µL). MC-ValCit-PAB-MMAE (1 mg, 0.76 µmol, 1.3 eq) is added as dry DMF solution (160 µL). The reaction is stirred for 3 h. The crude material is purified by reversed-phase HPLC (Water 0.1% TFA/Acetonitrile 0.1%TFA 9.5:0.5 to 2:8 in 20 min) and lyophilized, to obtain a white solid. MS(ES+) m/z 3003.5 (M+H)⁺

**Synthesis of Conjugate 50.** Bi-ESV6-Peptide **(P11**, 1 mg, 0.59 µmol, 3.3 eq) is dissolved in PBS pH 7.4 (300 µL). IRDye750 (200µg, 0.174 µmol, 1.0 eq) is added as dry DMSO solution (200 µL). The reaction is stirred for 3 h. The crude material is purified by reversed-phase HPLC (Water 0.1% TFA/Acetonitrile 0.1%TFA 9.5:0.5 to 2:8 in 20 min) and lyophilized, to obtain an orange solid. MS(ES+) m/z 2838.0 (M+1H)¹⁺

**Synthesis of Conjugate 51.** Bi-ESV6-Peptide **(P11**, 1 mg, 0.59 µmol, 1 eq) is dissolved in PBS pH 7.4 (300 µL). Maleimide-DOTA (465 µg, 0.59 µmol, 1.0 eq) is added as dry DMSO solution (200 µL). The reaction is stirred for 3 h. The crude material is purified by reversed-phase HPLC (Water 0.1% TFA/Acetonitrile 0.1%TFA 9.5:0.5 to 2:8 in 20 min) and lyophilized, to obtain an orange solid. MS(ES+) m/z 2213.9 (M+1H)¹⁺

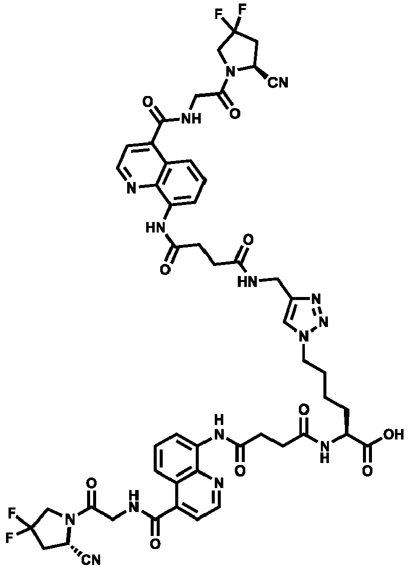

**Synthesis of P13**. Commercially available 2-Chlro-trityl chloride resin (300 mg) is swollen in DMF (3 × 5 min × 5 mL). The resin is extended with NHFmoc-Azido-Lysine (1 mmol), HBTU (1.0 eq), HOBt (1.0 eq) and DIPEA (2.0 eq) in DMF (5 mL). The mixture is allowed to stand for 10 min at 0°C and then react with the resin for 1 h under gentle agitation. The resin is then washed with methanol. The resin is extended with (*S*)-4-((4-((2-(2-cyano-4,4-difluoropyrrolidin-1-yl)-2-oxoethyl)carbarnoyl)quinolin-8-yl)amino)-4-oxobutanoic acid (P4, 1 mmol), HOBt (1.0 eq) and DIPEA (2.0 eq) in DMF (5 mL). **P10** (78 mg, 0.17 mmol, 0.86 eq), CuI (4 mg, 0.02 mmol, 0.1 eq) and TBTA (34 mg, 0.06 mmol, 0.3 eq) is dissolved in 5 mL of a mixture 1:1 DMF/THF. The peptide is cleaved from the resin with a mixture of 50 % HFIP in DCM at room temperature for 1 h. The solvent is removed under reduced pressure and the crude precipitated in cold diethyl ether, centrifuged, dissolved in water/ACN and purify via HPLC (Water 0.1% TFA/Acetonitrile 0.1%TFA 9.5:0.5 to 5:5 in 15 min) and lyophilized, to obtain a white solid. MS(ES+) m/z 1111.1 (M+H)⁺

**Synthesis of DOTA-GA-Bi-ESV6 (57'). P13** (45 mg, 40.5 µmol, 1.0 eq) is dissolved in dry DMSO (400 µL). Dicyclohexylcarbodiimide (10.9 mg, 52.7µmol, 1.3 eq) and N-hydroxysuccinimide (14 mg, 122 µmol, 3 eq) are added and the reaction was stirred overnight at room temperature, protected from light. 100 µL of PBS solution containing 2,2',2"-(10-(4-((2-aminoethyl)amino)-1-carboxy-4-oxobutyl)-1,4,7,10-tetraazacyclododecane-1,4,7-triyl)triacetic acid (25 mg, 48.6 µmol, 1.2 eq) is added and the reaction was stirred for 2h. The crude product is purified by reversed-phase HPLC (Water 0.1% TFA/Acetonitrile 0.1%TFA 9.5:0.5 to 2:8 in 20 min) and lyophilized, to obtain a white solid. MS(ES+) m/z 1624.8 (M+H)⁺

### Example 1: Synthesis of bivalent molecules

Two different bivalent molecules based on "ESV6" termed "Bi-ESV6" and "Bi-ESV6-triazole" have been synthetized. Bi-ESV6 **(P16)** has superior production yields as compared to "Bi-ESV6-triazole" **(P11)** as shown in Table 2 below.

**Table 2. Yields for the synthesis of bivalent molecules**

| Name | Yield (%) |
|---|---|
| Bi-ESV6 | 19 |
| Bi-ESV6-triazole | 6 |

### Example 2: Tumour targeting experiments

### Radiolabelling of ESV6-DOTAGA and Bi-ESV6-DOTAGA with ¹⁷⁷Lutetium

25 µL of ESV6-DOTAGA (1mM solution in mQ Water, 1% DMSO) were diluted with 50 µL of 1M sodium acetate buffer, pH=4. Then, 25 µL of ¹⁷⁷LuCl₃ (5 MBq) were added and the solution was heated at 95°C for 15 minutes at 300 rpm. After the radiolabeling, the solution was cooled down and then diluted with 375 µL of PBS and checked via Radio-HPLC (injection of 50 µL, 0.5 MBq), showing a single peak with >95 % of conversion.

25 µL of Bi-ESV6-DOTAGA (1, ImM solution in mQ Water, 1% DMSO) were diluted with 50 µL of 0.5M sodium acetate buffer, pH=8. Then, 25 µL of ¹⁷⁷LuCl₃ (5 MBq) were added and the solution was heated at 95°C for 15 minutes at 300 rpm. the solution was cooled down and then diluted with 375 µL of PBS and checked via Radio-HPLC (injection of 50 µL, 0.5 MBq), showing a single peak with >95 % of conversion.

The HPLC profiles of ¹⁷⁷Lutetium-labeled preparations of ESV6-DOTAGA and Bi-ESV6-DOTAGA shown in **Figure 1** indicate a high degree of purity of the radioconjugate.

### Gel filtration experiment

PD-10 columns were pre-equilibrated with running buffer (50 mM Tris, 100 mM NaCl, pH = 7.4). 150 µL of a solution containing hFAP (2 µM) or hCAIX (2 µM) was pre-incubated with 2µL of ¹⁷⁷Lu-ESV6-DOTAGA stock solution (50 µM, 5 MBq). The final solution was loaded on the column and flushed with running buffer. Fractions of the flow-through (200 µL) were collected in test tubes and the radioactivity measured with a Packard Cobra γ-counter.

As negative control, 2µL of ¹⁷⁷Lu-ESV6-DOTAGA stock solution (50 µM, 5 MBq) were diluted in 150 µL of running buffer (50 mM Tris, 100 mM NaCl, pH = 7.4), without proteins. The final solution was loaded on the column and flushed with running buffer. Fractions of the flow-through (200 µL) were collected in test tubes and the radioactivity measured with a Packard Cobra γ-counter.

Results of the co-elution experiments performed with ¹⁷⁷Lu-ESV6-DOTAGA and ¹⁷⁷Lu-Bi-ESV6-DOTAGA on hFAP, hCAIX and without protein are shown in Figure 2. Both compounds form a stable complex with hFAP and were eluted in the first 2 mL, as expected. When the compounds were incubated with the irrelevant protein CAIX or without any protein, the peak of radioactivity was detected after more than 3000 µL of elutate. ¹⁷⁷Lu -ESV6-DOTAGA and ¹⁷⁷Lu-Bi-ESV6-DOTAGA form a stable complex with recombinant human FAP.

### Implantation of Subcutaneous HT-1080 Tumours

Upon thawing, HT-1080.hFAP positive cells and HT-1080.wt cells were kept in culture in DMEM medium supplemented with fetal bovine serum (10%, FBS) and Antibiotic-Antimycotic (1%, AA) at 37°C and 5% CO₂. For passaging, cells were detached using Trypsin-EDTA 0.05% when reaching 90% confluence and re-seeded at a dilution of 1:4.

Cells were grown to 80% confluence and detached with Trypsin-EDTA 0.05%. Cells were re-suspended in cold HBSS medium to a final concentration of 5 × 10⁷ cells/mL.

Aliquots of 5 × 10⁶ cells (100 µL of suspension) were injected subcutaneously in the flank of female athymic BALB/C nu/nu mice (6-8 weeks of age).

All animal experiments were conducted in accordance with Swiss animal welfare laws and regulations under the license number ZH04/2018 granted by the Veterinäramt des Kantons Zürich.

### Biodistribution experiment

HT-1080.hFAP tumour cells were xenografted in the right flank and HT-1080.wt tumour cells were xenografted in the left flank as described above and allowed to grow to an average volume of 1.2 ± 0.2 mL.

Mice were randomized (n = 4 or 5 per group) and injected intravenously with preparations of ¹⁷⁷Lutetium-labeled ESV6-DOTAGA and Bi-ESV6-DOTAGA (250 nmol/Kg; 50 MBq/Kg).

Mice were sacrificed 1h, 4h, 17h and 24h after the injection by CO2 asphyxiation and organs extracted, weighted and radioactivity measured with a Packard Cobra γ-counter. Values are expressed as %ID/g ± SD (Figure C). Food and water were given ad libitum during that period.

As shown in **Figure 3****,** injected dose per gram of tissue (ID%/g) at 1h, 4h, 17h and 24h indicate a very high uptake in FAP-expressing tumour in mice treated with ¹⁷⁷Lu-Bi-ESV6-DOTAGA and a high uptake in mice treated with ¹⁷⁷Lu-ESV6-DOTAGA. Negligible uptake in non FAP-expressing tumour (HT-1080.wt) is registered for both radio-conjugates indicating their high degree of specificity for FAP. Negligible uptake in normal organs is registered for both radio-conjugates indicating their high degree of tolerability. The kidney uptake for ¹⁷⁷Lu-Bi-ESV6-DOTAGA is transient and becomes negligible 24 hours after injection. The tumour-to-organ ratios are shown in the below **Tables 3** and **4.**

**Table 3. Tumour-to-organ ratios in mice treated with ¹⁷⁷Lu-ESV6-DOTAGA**

| | **1h (n=4)** | **4h (n=4)** | **17h (n=4)** | **24h (n=5)** |
|---|---|---|---|---|
| **HT-1080.hFAP** | 34.89 ± 2.79 | 26.81 ± 6.42 | 32.63 ± 17.40 | 13.29 ± 5.95 |
| **Liver** | 5.89 ± 0.82 | 21.58 ± 2.99 | 40.26 ± 13.26 | 30.37 ± 6.70 |
| **Lung** | 37.92 ± 2.59 | 90.33 ± 29.30 | 149.76 ± 88.53 | 139.74 ± 83.00 |
| **Spleen** | 108.64 ± 8.98 | 168.84 ± 67.83 | 200.43 ± 45.69 | 103.25 ± 66.71 |
| **Heart** | 134.56 ± 19.75 | 208.15 ± 113.45 | 354.12 ± 231.16 | 168.51 ± 171.71 |
| **Kidney** | 9.16 ± 0.88 | 8.25 ± 1.45 | 5.72 ± 2.04 | 11.90 ± 16.00 |
| **Intestine** | 41.01 ± 41.84 | 39.79 ± 11.37 | 162.57 ± 94.89 | 76.98 ± 58.64 |
| **Tail** | 31.19 ± 8.39 | 10.36 ± 7.60 | 4.74 ± 5.11 | 8.77 ± 2.30 |
| **Blood** | 43.13 ± 17.25 | 112.90 ± 49.79 | 235.58 ± 189.65 | 855.02 ± 368.48 |

**Table 4. Tumour-to-organ ratios in mice treated with ¹⁷⁷Lu-Bi-ESV6-DOTAGA (5)**

| | **1h (n=3)** | **4h (n=4)** | **17h (n=4)** | **24h (n=5)** |
|---|---|---|---|---|
| **HT-1080.hFAP** | 22.99 ± 8.09 | 25.01 ± 1.27 | 27.24 ± 11.33 | 29.70 ± 13.76 |
| **Liver** | 18.76 ± 5.31 | 29.89 ± 4.12 | 27.85 ± 2.25 | 30.55 ± 8.00 |
| **Lung** | 19.54 ± 4.09 | 65.51 ± 10.53 | 123.97 ± 74.71 | 98.72 ± 18.89 |
| **Spleen** | 42.09 ± 17.74 | 73.84 ± 10.30 | 68.20 ±11.67 | 93.64 ± 64.51 |
| **Heart** | 52.23 ± 10.83 | 125.03 ± 17.09 | 147.81 ± 12.93 | 200.88 ± 59.49 |
| **Kidney** | 7.19 ± 2.29 | 8.61 ± 0.84 | 8.40 ± 1.31 | 9.90 ± 2.38 |
| **Intestine** | 59.58 ± 6.48 | 82.38 ± 31.23 | 110.81 ± 32.15 | 103.58 ± 60.62 |
| **Tail** | 10.43 ± 1.72 | 9.68 ± 4.14 | 14.04 ± 8.08 | 15.54 ± 7.98 |
| **Blood** | 32.75 ± 8.97 | 62.84 ± 11.21 | 95.40 ± 19.11 | 178.47 ± 57.65 |

## Claims

1. A compound, its individual diastereoisomers, its hydrates, its solvates, its crystal forms, its individual tautomers or a pharmaceutically acceptable salt thereof, wherein the compound structure comprises two moieties A having the following structure:

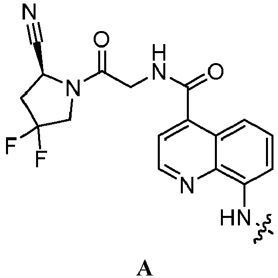

2. The compound of claim 1, wherein the compound is represented by the following Formula **I:** 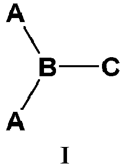
wherein **B** is a multifunctional moiety covalently attaching the moieties **A** to **C;**
and **C** is an atom, a molecule or a particle, and/or is a therapeutic or diagnostic agent.

3. The compound according to any one of the preceding claims, wherein each moiety **A** has the following structure **A¹** or **A²**, wherein m is 0, 1, 2, 3, 4 or 5:

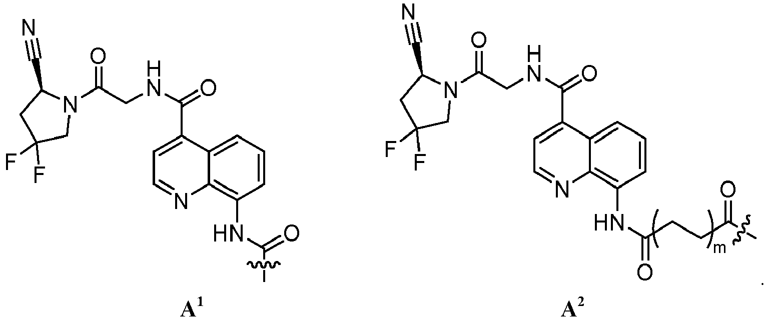

4. The compound according to claim 2 or 3, wherein **B** is represented by any of the following general Formulae **II―V**, wherein: 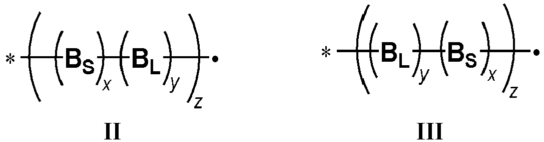 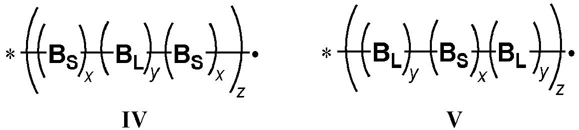
each *x* is an integer independently selected from the range of 0 to 100, preferably 0 to 50, more preferably 0 to 30, yet more preferably selected from 0, 1, 2, 3, 4, 5, 6, 7, 8, 9, 10, 11, 12, 13, 14, 15, 16, 17, 18, 19 and 20;
each *y* is an integer independently selected from the range of 0 to 30, preferably selected from 0, 1, 2, 3, 4, 5, 6, 7, 8, 9, 10, 11, 12, 13, 14, 15, 16, 17, 18, 19 and 20;
each z is an integer independently selected from the range of 0 to 5, preferably selected from selected from 0, 1, 2, 3 and 4;
**B** is a multifunctional moiety linking moiety **C** and the two moieties **A;**
^{∗} represents a point of attachment to a moiety **A;** and
• represents a point of attachment to moiety **C.**

5. The compound according to any one of the preceding claims, wherein the compound comprises moiety **B** represented by any of the following general Formulae **IIa―Va**: 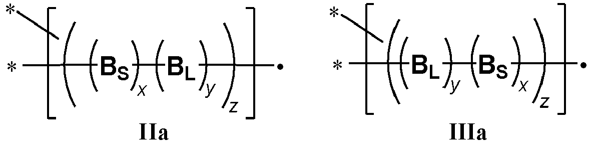 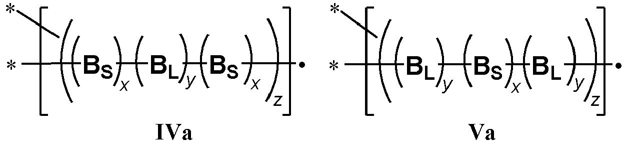
wherein *x, y* and z are defined as in any one of the preceding claims;
each ^{∗} represents a point of attachment to a moiety **A;** and
• represents a point of attachment to moiety **C.**

6. The compound according to any one of the preceding claims, wherein:
(a) **B_{S}** and/or **B_{L}** is a group comprising or consisting of a structural unit independently selected from the group consisting of alkylene, cycloalkylene, arylalkylene, heteroarylalkylene, heteroalkylene, heterocycloalkylene, alkenylene, cycloalkenylene, arylalkenylene, heteroarylalkenylene, heteroalkenylene, heterocycloalenkylene, alkynylene, heteroalkynylene, arylene, heteroarylene, aminoacyl, oxyalkylene, aminoalkylene, diacid ester, dialkylsiloxane, amide, thioamide, thioether, thioester, ester, carbamate, hydrazone, thiazolidine, methylene alkoxy carbamate, disulfide, vinylene, imine, imidamide, phosphoramide, saccharide, phosphate ester, phosphoramide, carbamate, dipeptide, tripeptide, tetrapeptide, each of which is substituted or unsubstituted;
(b) **B_{S}** and/or **B_{L}** is a group comprising or consisting of a structural unit independently selected from the group consisting of: 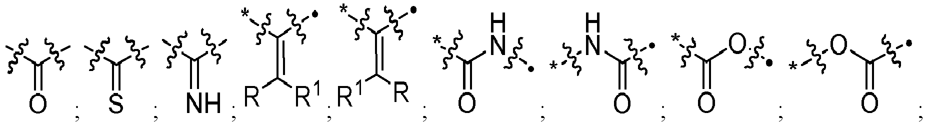 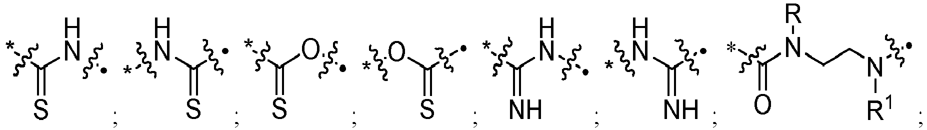 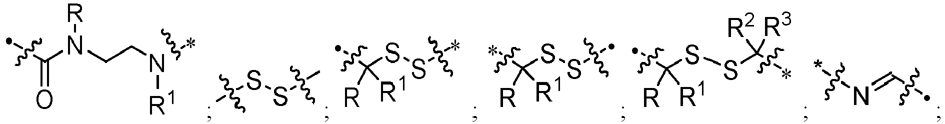 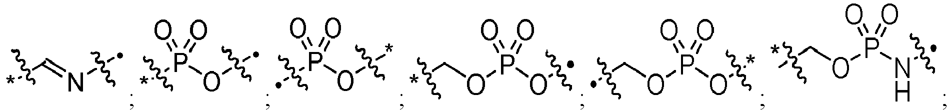 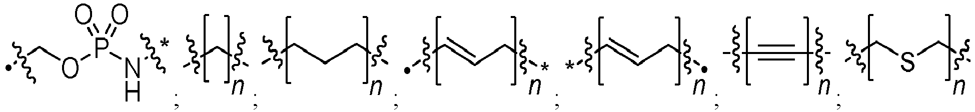 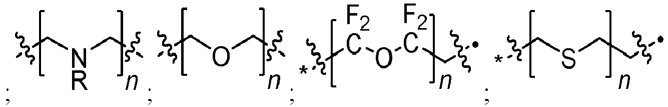 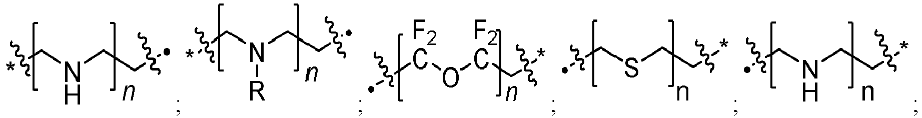 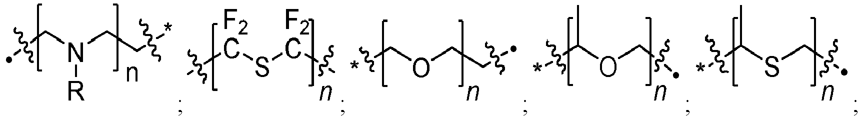 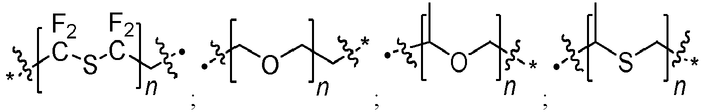 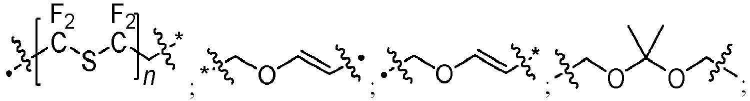 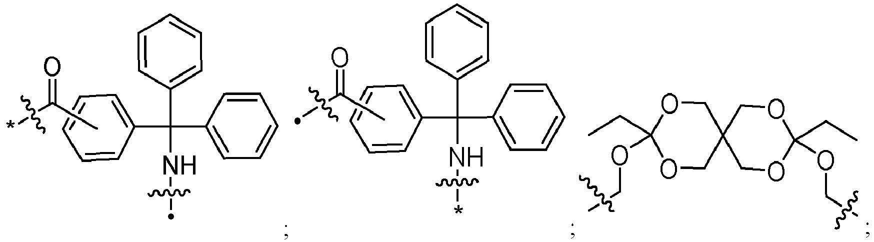 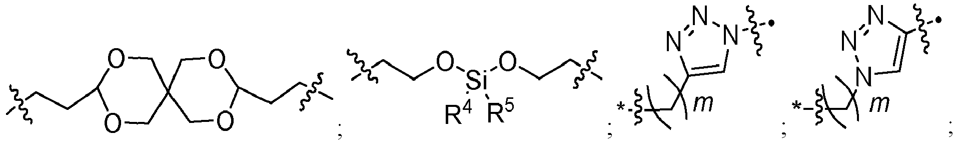 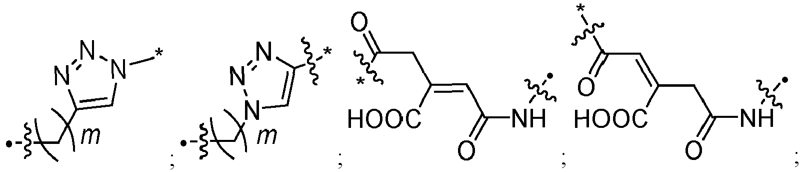 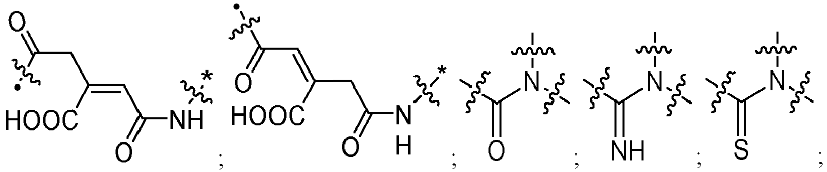 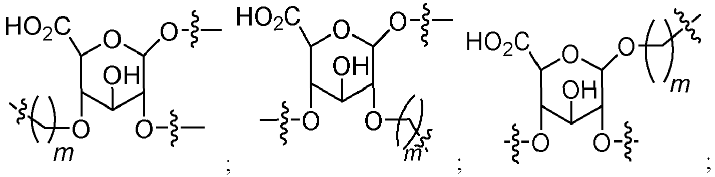 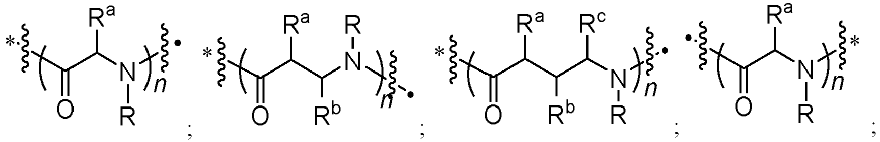 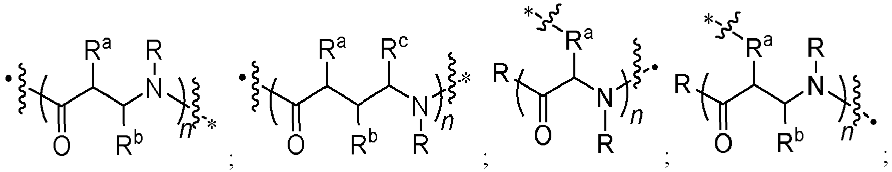 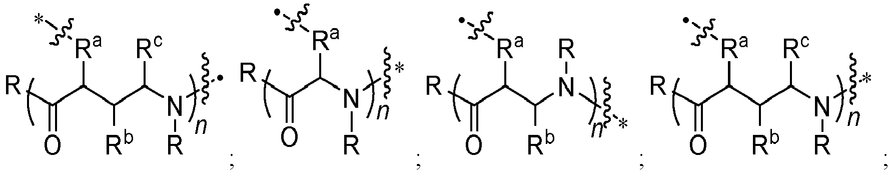 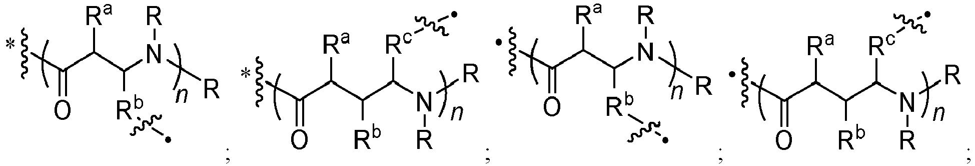 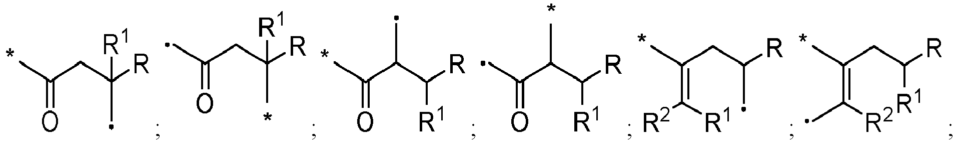 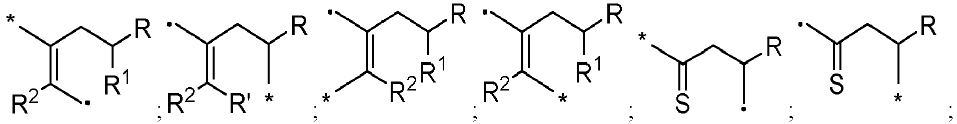 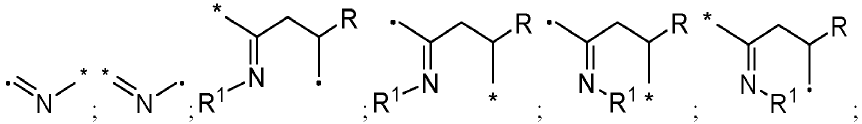 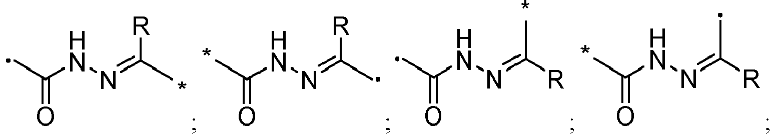 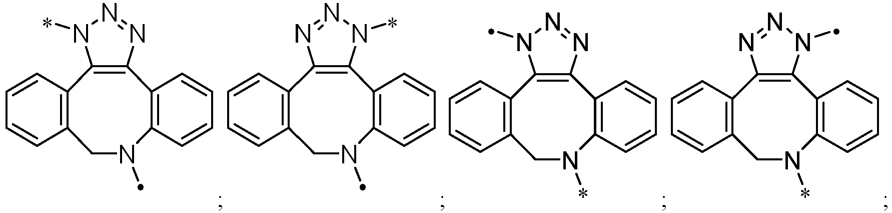 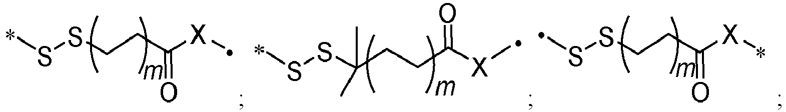 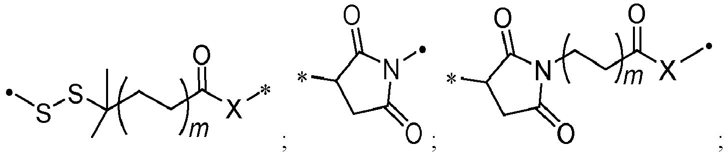 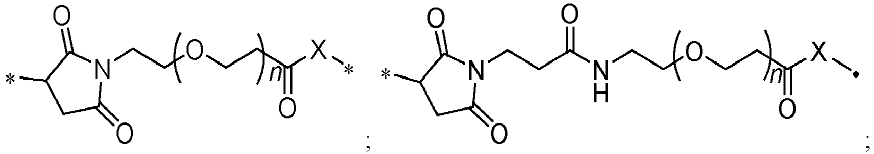 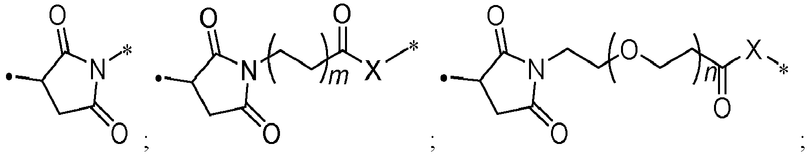 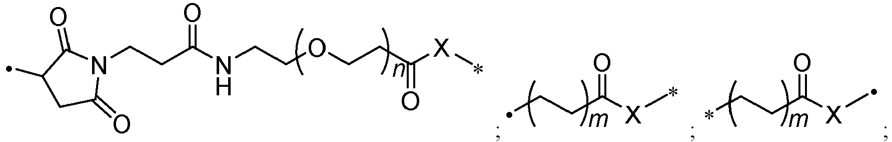 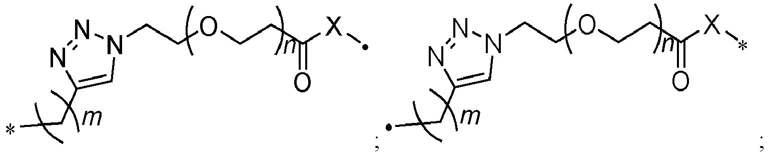
wherein each of R, R¹, R² and R³ is independently selected from H, OH, SH, NH₂, halogen, cyano, carboxy, alkyl, cycloalkyl, aryl and heteroaryl, each of which is substituted or unsubstituted;
each of R⁴ and R⁵ is independently selected from alkyl, cycloalkyl, aryl and heteroaryl, each of which is substituted or unsubstituted;
each of R^{a}, R^{b} and R^{c} is independently selected from side-chain residues of a proteinogenic or a non-proteinogenic amino acid, each of which can be further substituted;
each X is independently selected from NH, NR, S, O and CH₂, preferably NH;
each of *n* and *m* is independently an integer from 0 to 100, preferably 0 to 50, more preferably 0 to 30, yet more preferably selected from 0, 1, 2, 3, 4, 5, 6, 7, 8, 9, 10, 11, 12, 13, 14, 15, 16, 17, 18, 19 and 20; and
wherein each ^{∗} represents a point of attachment for which the shortest path to a moiety A comprises less atoms than that for •; and each • represents a point of attachment for which the shortest path to moiety C comprises less atoms than that for ^{∗};
(c) one or more **B_{L}** independently comprises or consists of one or more of the following structural units: 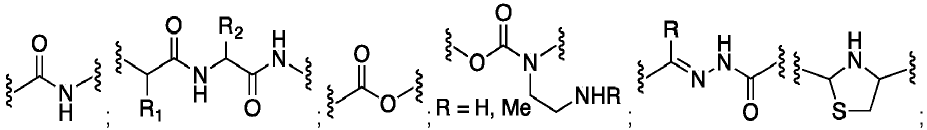 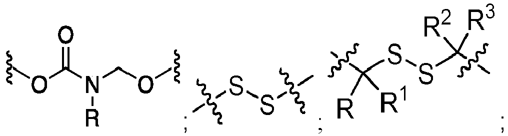 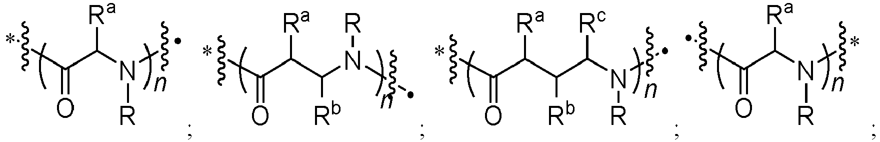 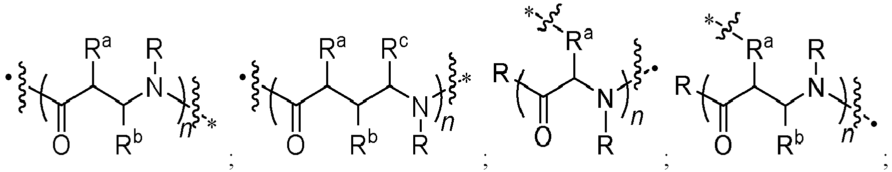 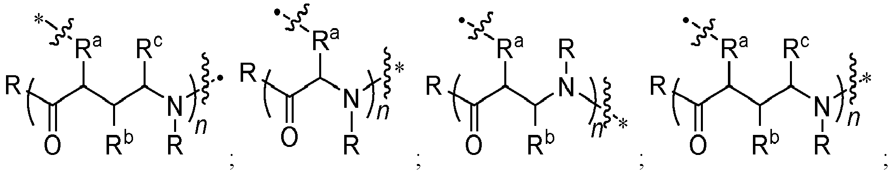 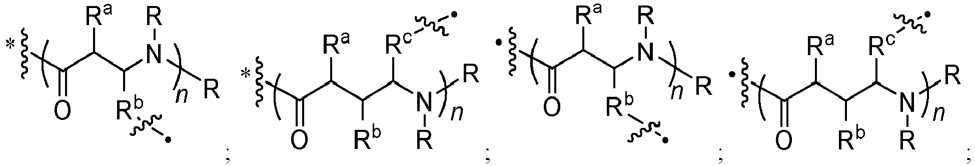
wherein in each of the above structures, n is 1, 2, 3 or 4; and
each ^{∗} represents a point of attachment for which the shortest path to a moiety A comprises less atoms than that for •; and each • represents a point of attachment for which the shortest path to moiety C comprises less atoms than that for ^{∗}, with the proviso that when n is > 1 and a respective point of attachment is indicated on any one of R^{a}, R^{b} and R^{c}, then it can be independently present in one or more of the peptide monomeric units, preferably in one peptide monomeric unit most distant from the other point of attachment indicated in the respective structure;
(d) one or more of **B_{L}** and **B_{S}** is independently selected from the following structures: 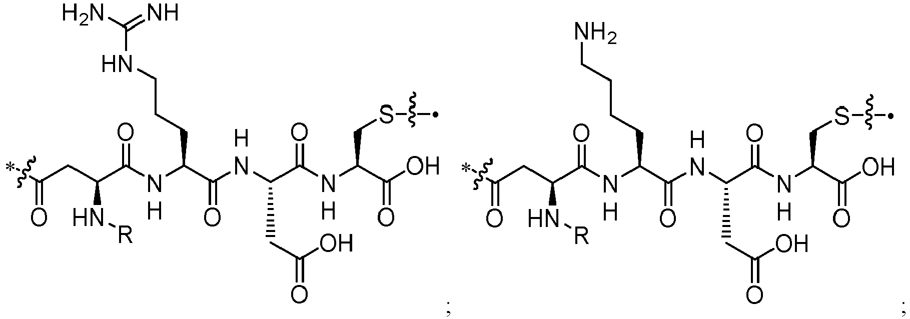 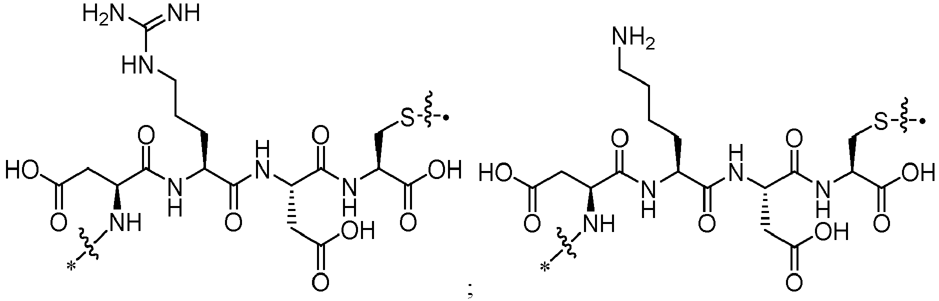
^{∗}―Val-Ala―•; ^{∗}―Val-Lys―•; ^{∗}―Val-Arg―•,
wherein each ^{∗} represents a point of attachment for which the shortest path to moiety **A** comprises less atoms than that for •; and each • represents a point of attachment for which the shortest path to moiety **C** comprises less atoms than that for ^{∗};
and/or
(e) y is 1, 2 or 3; and/or at least one **B_{L}** further comprises a cleavable linker group independently selected from the following structures: 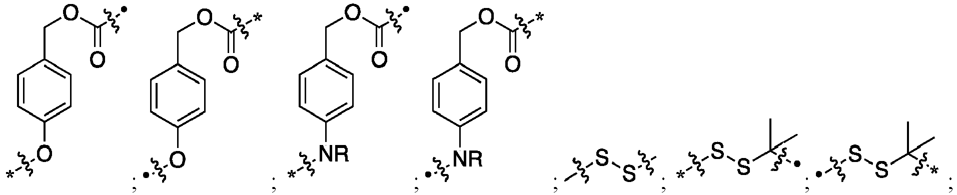 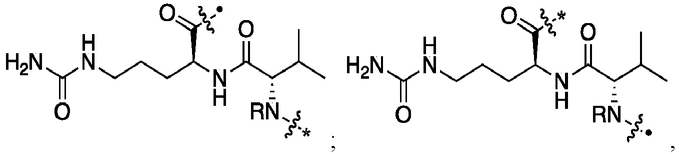 each ^{∗} represents a point of attachment for which the shortest path to moiety **A** comprises less atoms than that for •; and each • represents a point of attachment for which the shortest path to moiety **C** comprises less atoms than that for ^{∗}; or
(f) wherein B has the following structure: 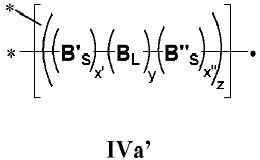 wherein **B'_{S}** and **B"_{S}** are each independently selected from the group consisting of: 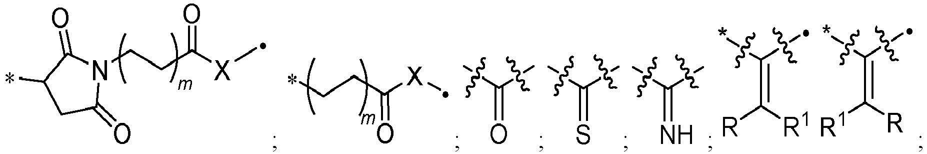 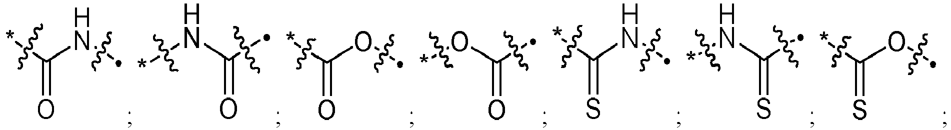 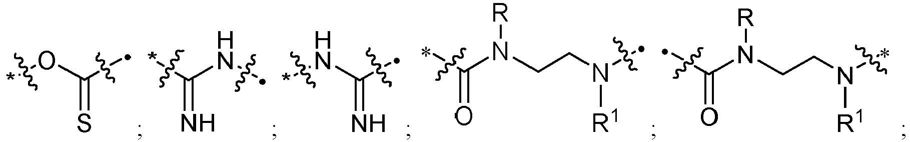 each **B_{L}** is independently selected from the group consisting of: 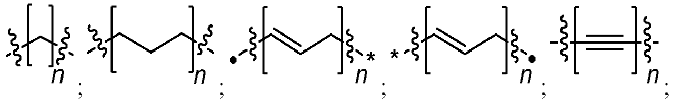 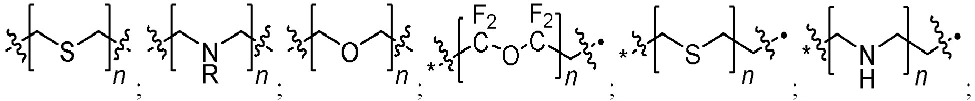 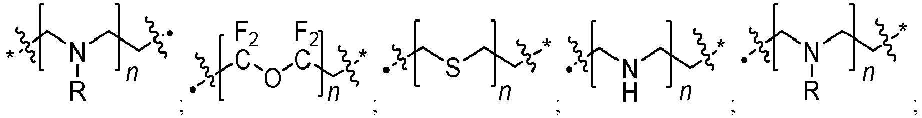 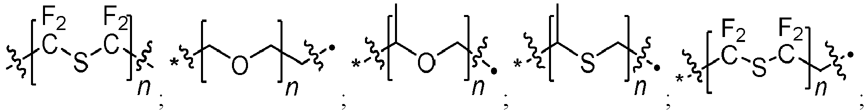 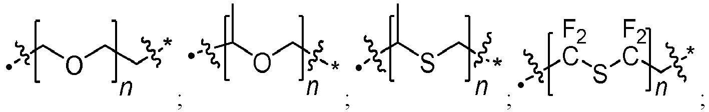 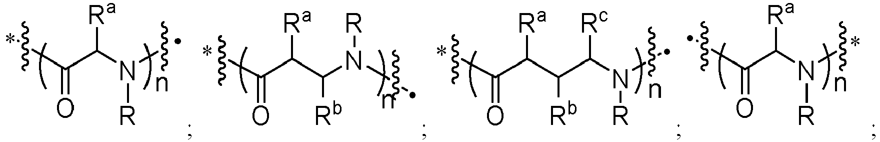 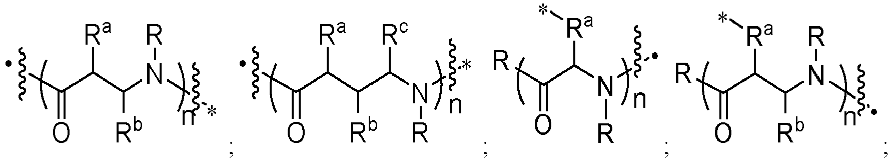 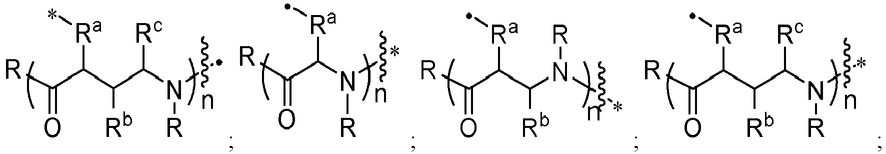 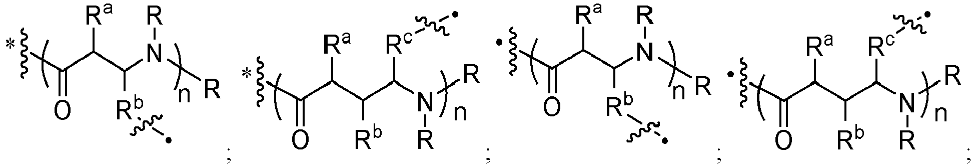 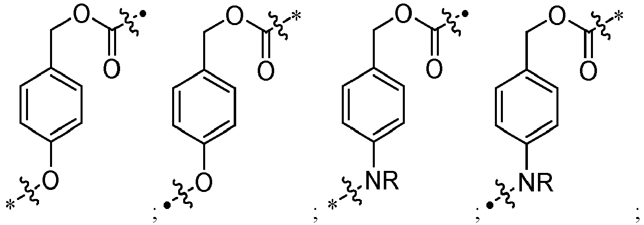 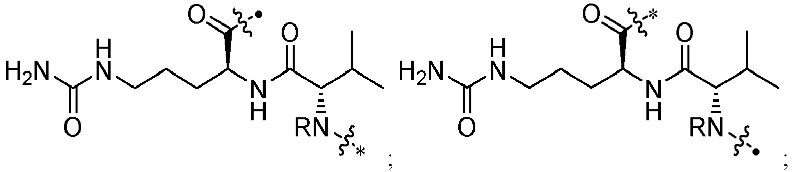
each *n* is 0, 1, 2, 3, 4 or 5;
each m is 0, 1, 2, 3, 4 or 5;
each *x'* is 0, 1 or 2;
each *x"* is 0, 1 or 2;
each *y* is 0, 1 or 2; and
z is 1 or 2,
wherein R, R¹, R², R³, R^{a}, R^{b}, R^{c}, X, ^{∗} and • are defined as in any one of the preceding claims.

7. The compound according to any one of the preceding claims having a structure represented by one of the following formulae: 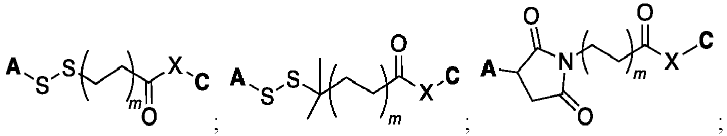 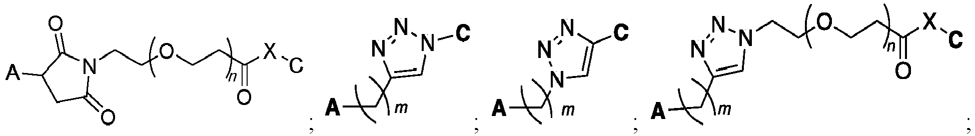 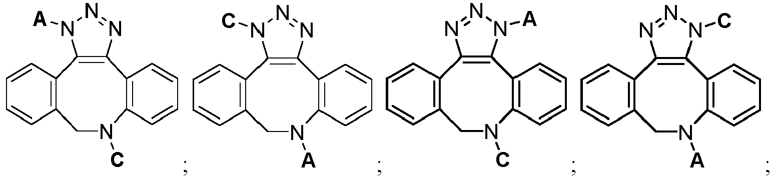 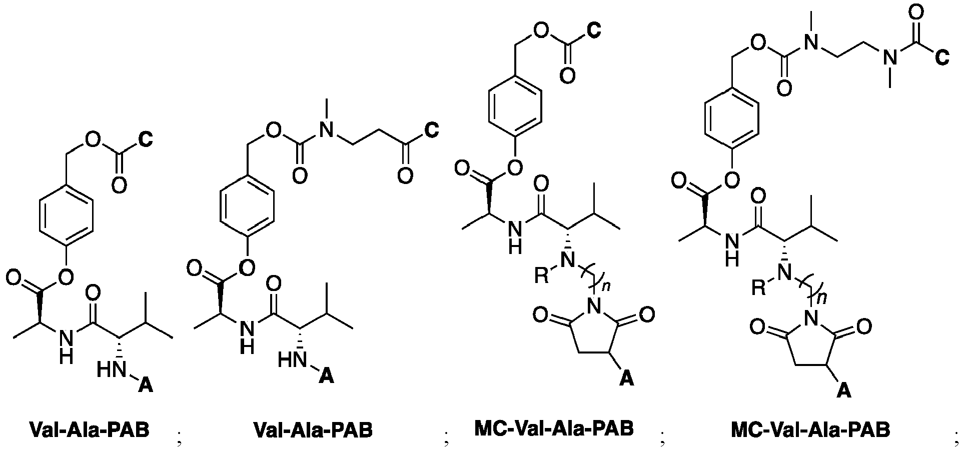 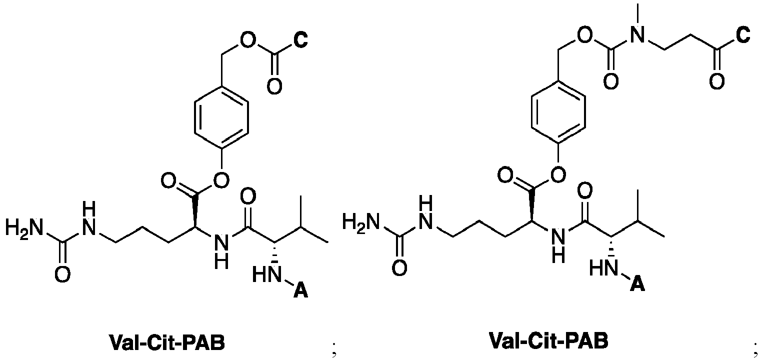 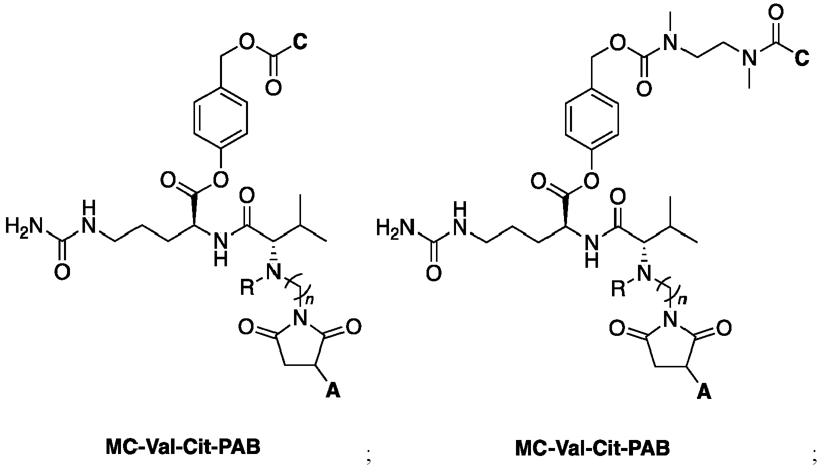 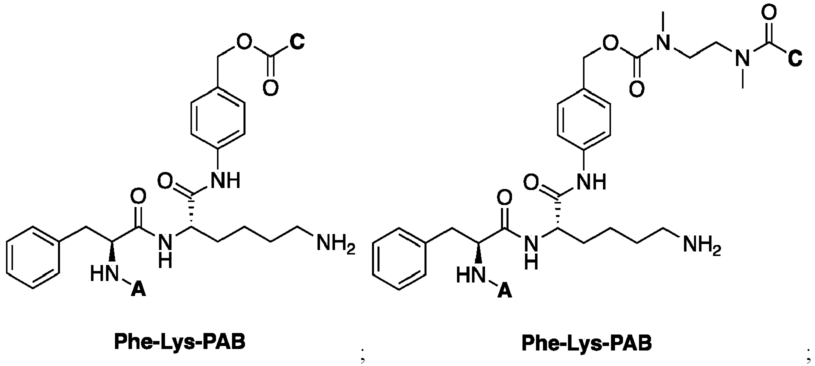 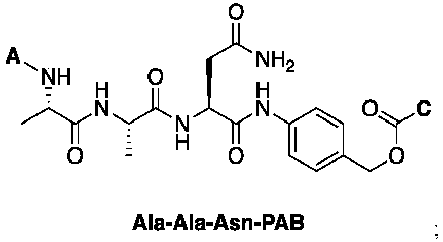 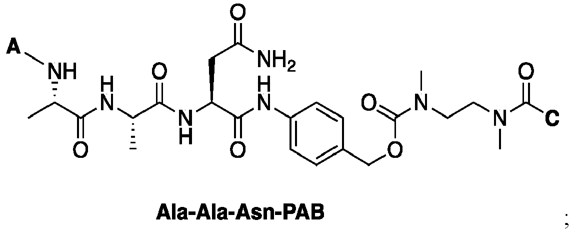 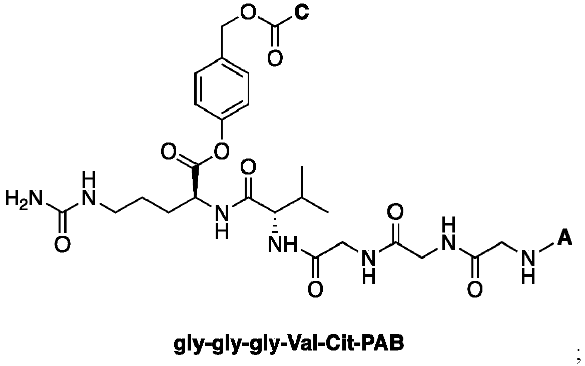 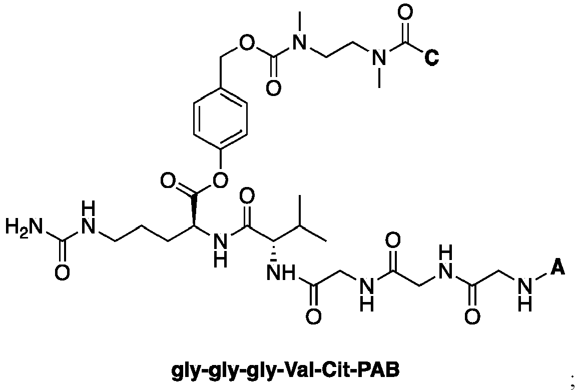 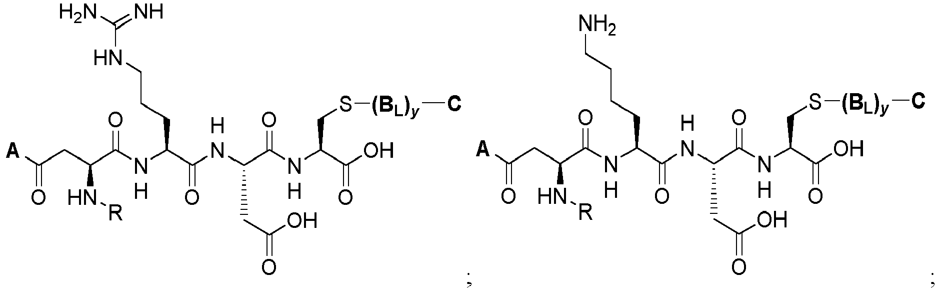 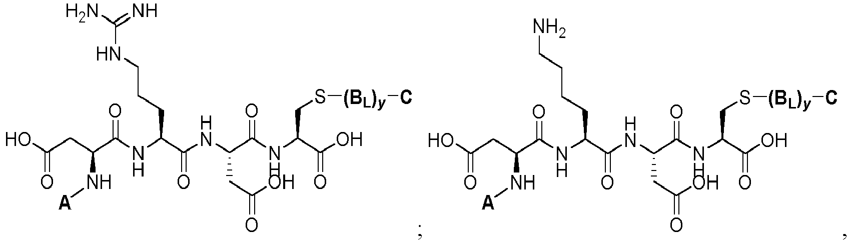 wherein each of the above structures comprises one further moiety **A** linked to the moiety corresponding to **B**.

8. The compound according to any one of the preceding claims, wherein the moiety **C** is selected from: (a) a chelating agent group suitable for radiolabelling; (b) a radioactive group comprising a radioisotope; (c) a chelate of a radioactive isotope with a chelating agent; (d) a fluorophore group; (e) a cytotoxic and/or cytostatic agent; (f) immunomodulator agent; or (g) a protein, wherein preferably:
(a) the chelating agent group suitable for radiolabelling is selected from sulfur colloid, diethylenetriaminepentaacetic acid (DTPA), ethylenediaminetetraacetic acid (EDTA), 1,4,7,10-tetraazacyclododecane-N,N′,Nʺ,N‴-tetraacetic acid (DOTA), 1,4,7-triazacyclononane-N,N′,Nʺ-triacetic acid (NOTA), 1,4,8,11-tetraazacyclotetradecane-N,N′,Nʺ,N‴-tetraacetic acid (TETA), iminodiacetic acid, bis(carboxymethylimidazole)glycine, 6-Hydrazinopyridine-3-carboxylic acid (HYNIC), 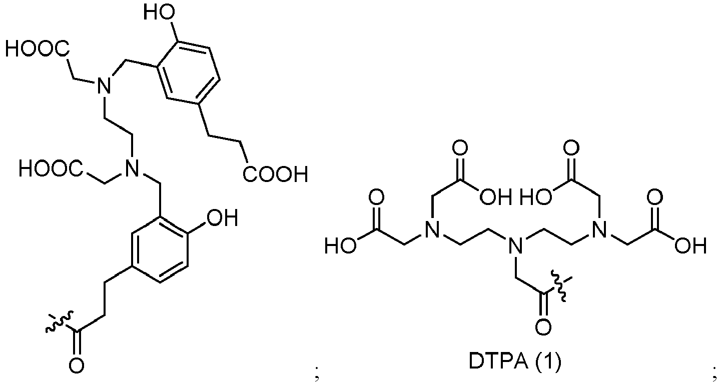 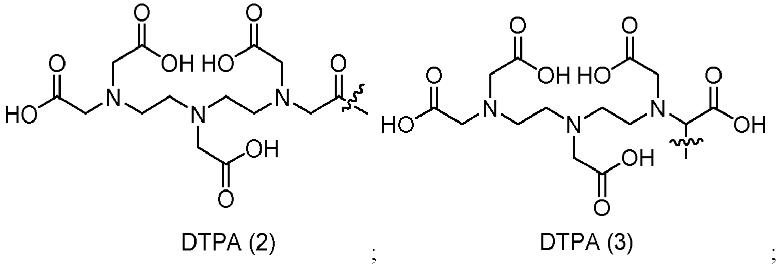 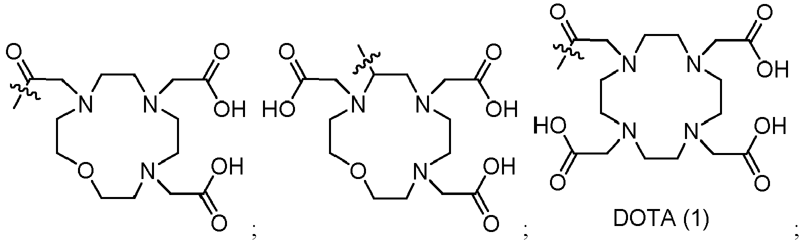 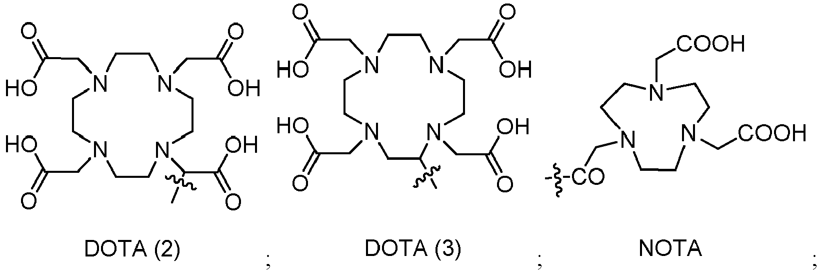 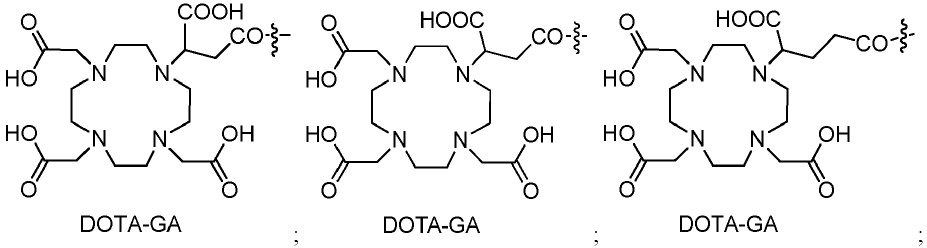 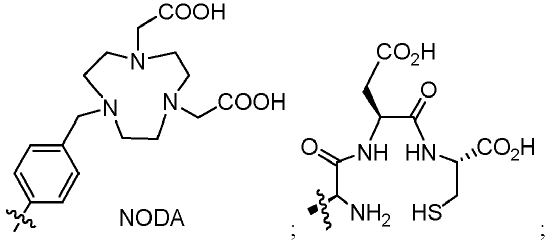 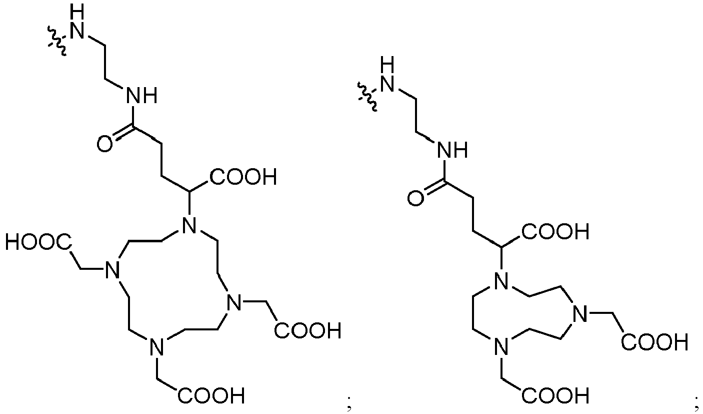 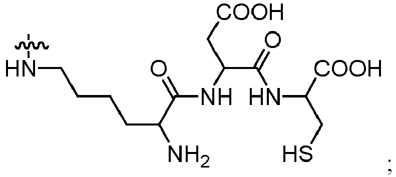 or
has a structure according to the following formula: 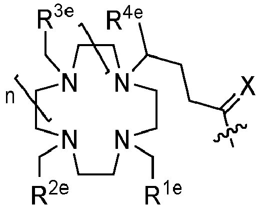 wherein:
n is 0, 1, 2, 3, 4 or 5; preferably 1;
R^{1e} is independently H, COOH, aryl-COOH or heteroaryl-COOH; preferably COOH;
R^{2e} is independently H, COOH, aryl-COOH or heteroaryl-COOH; preferably COOH;
each R^{3e} is independently H, COOH, aryl-COOH or heteroaryl-COOH; preferably COOH;
R^{4e} is independently H, COOH, aryl-COOH or heteroaryl-COOH; preferably COOH; and
X is O, NH or S; preferably O; or
has a structure according to the following formula: 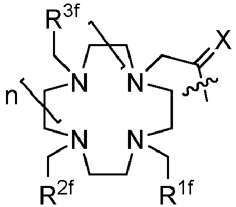
wherein:
n is 0, 1, 2, 3, 4 or 5; preferably 1
R^{1f} is independently H, COOH, aryl-COOH or heteroaryl-COOH; preferably COOH;
R^{2f} is independently H, COOH, aryl-COOH or heteroaryl-COOH; preferably COOH;
R^{3f} is independently H, COOH, aryl-COOH or heteroaryl-COOH; preferably COOH; and
X is O, NH or S; preferably O;
(b) the radioactive group comprising a radioisotope is selected from ²²³Ra, ⁸⁹Sr, ^{94m}Tc, ^{99m}Tc, ¹⁸⁶Re, ¹⁸⁸Re, ²⁰³Pb, ⁶⁷Ga, ⁶⁸Ga, ⁴⁷Sc, ¹¹¹In, ⁹⁷Ru, ⁶²Cu, ⁶⁴Cu, ⁸⁶Y, ⁸⁸Y, ⁹⁰Y, ¹²¹Sn, ¹⁶¹Tb, ¹⁵³Sm, ¹⁶⁶Ho, ¹⁰⁵Rh, ¹⁷⁷Lu, ¹²³I, ¹²⁴I, ¹²⁵I, ¹³¹I, ¹⁸F, ²¹¹At, ²²⁵Ac, ⁸⁹Sr, ²²⁵Ac, ^{117m}Sn and ¹⁶⁹Er;
(c) the chelate of a radioactive isotope is a chelate of an isotope listed under (b) above and/or with a chelating agent listed under (a) above; or moiety C is a group selected from any of the following structures: 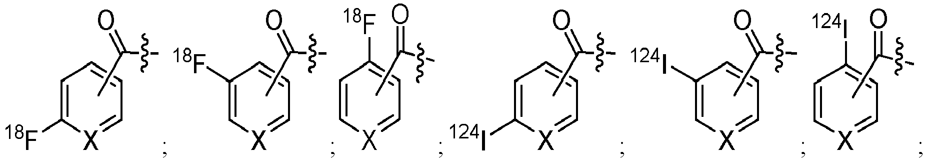 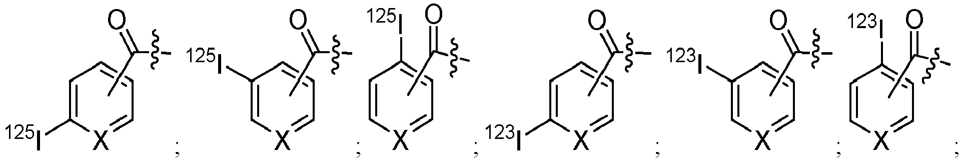 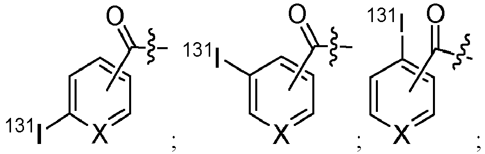 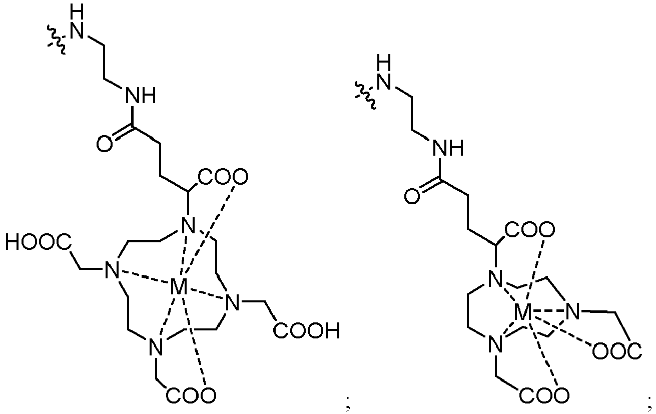 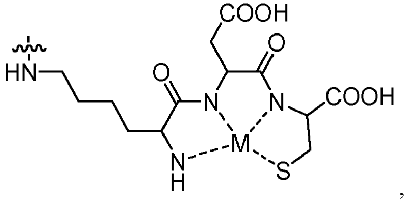 wherein M is a radioactive isotope, preferably selected among the list underr (b) above;
(d) the fluorophore group is selected from a xanthene dye, acridine dye, oxazine dye, cyanine dye, styryl dye, coumarine dye, porphine dye, fluorescent metal-ligand-complex, fluorescent protein, nanocrystals, perylene dye, boron-dipyrromethene dye and phtalocyanine dye, preferably selected from the following structures: 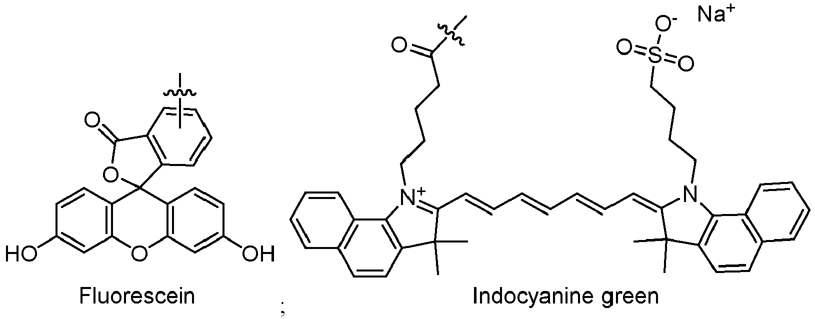 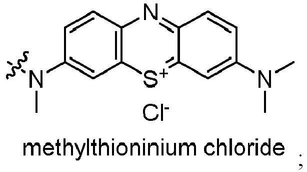 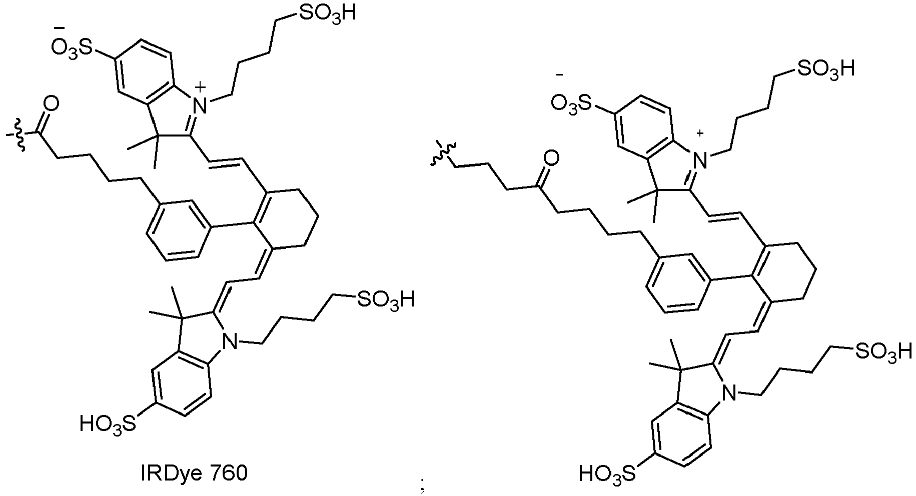 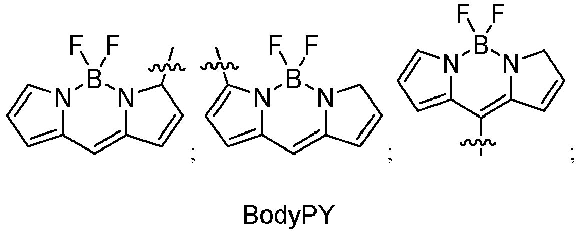 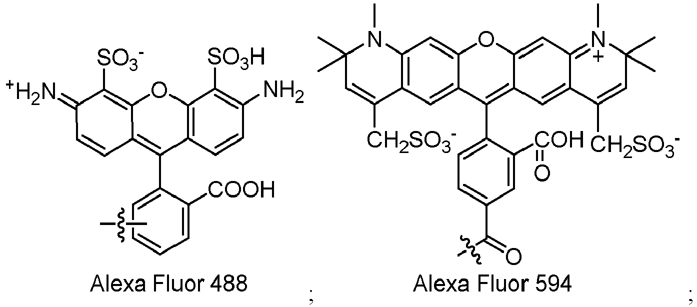
(e) the cytotoxic and/or cytostatic agent is selected from chemotherapeutic agent selected from the group consisting of topoisomerase inhibitors, alkylating agents, antimetabolites, antibiotics, mitotic disrupters, DNA intercalating agents, DNA synthesis inhibitors, DNA-RNA transcription regulator, enzyme inhibitors, gene regulators, hormone response modifiers, hypoxia-selective cytotoxins, epidermal growth factor inhibitors, anti-vascular agents and a combination of two or more thereof, preferably selected from the following structures: 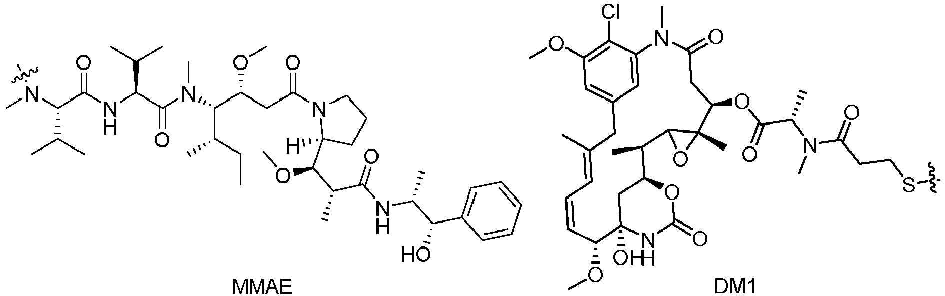 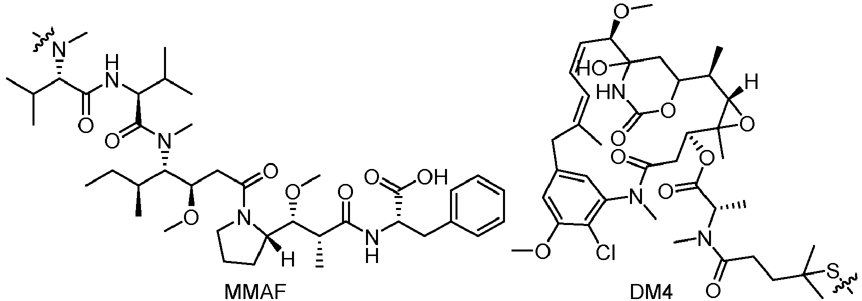 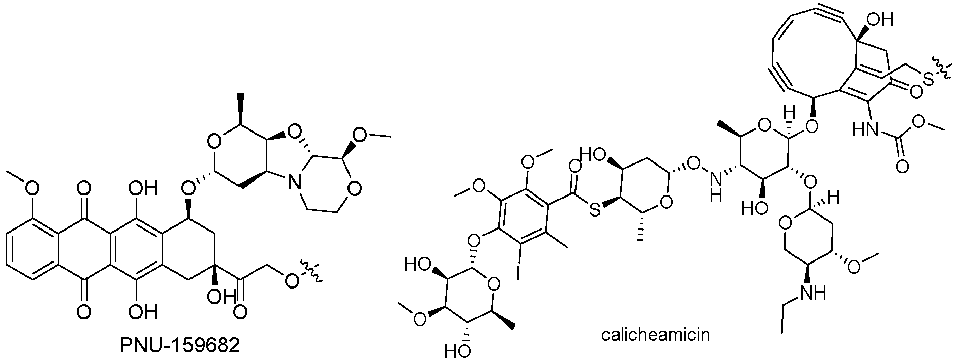 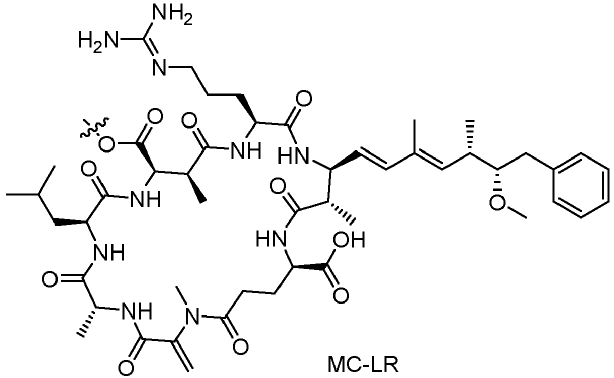 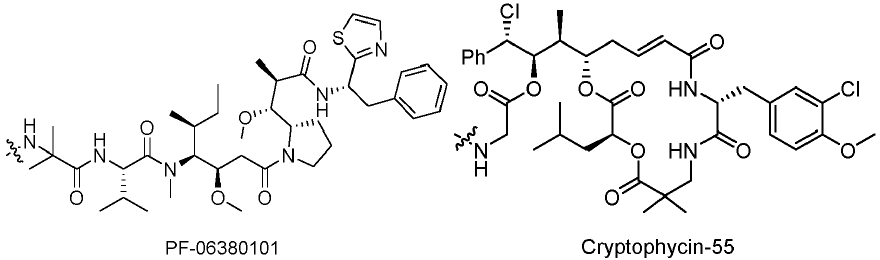 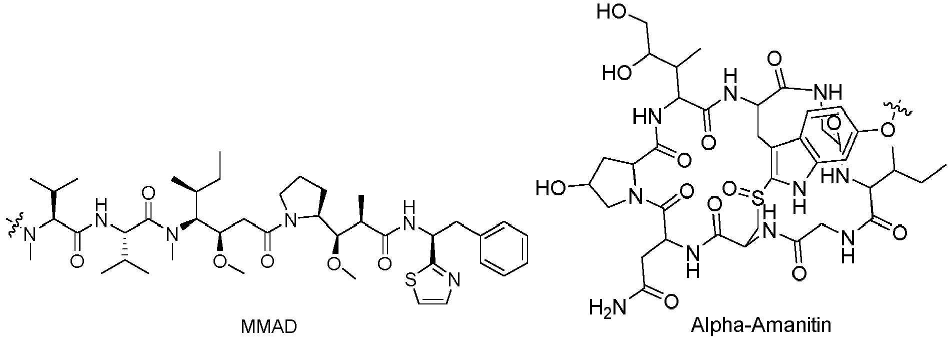 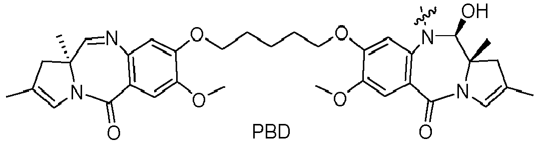 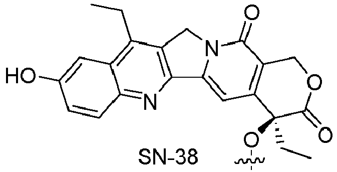 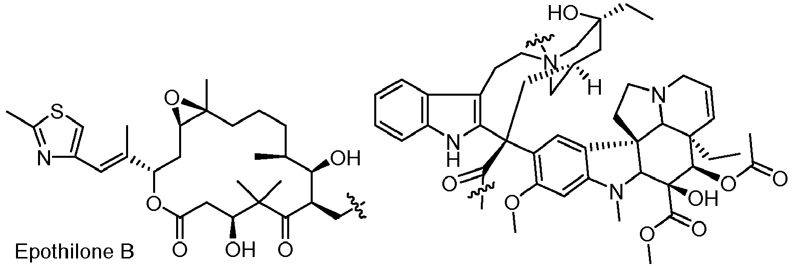 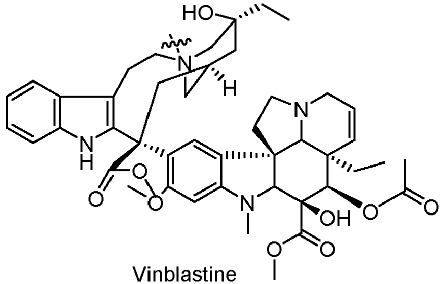 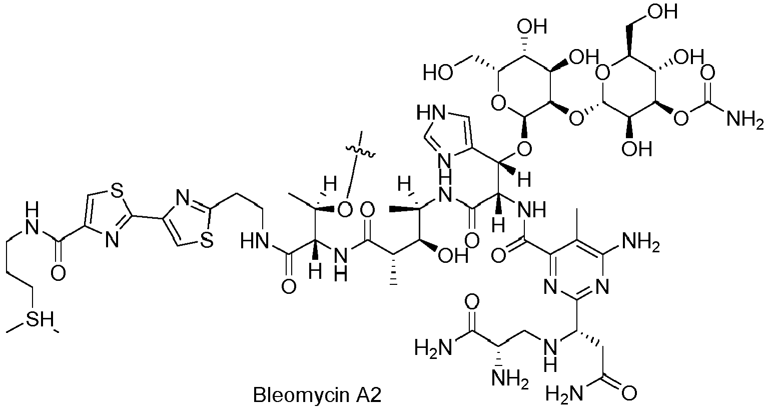 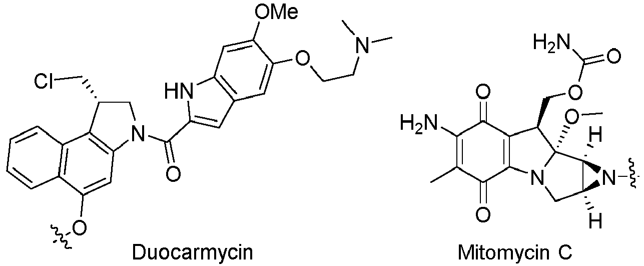 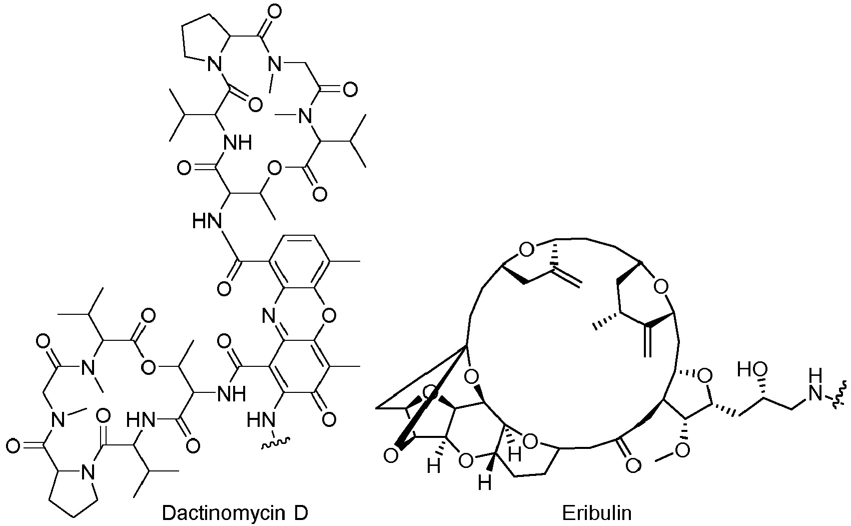 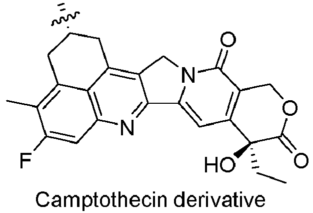 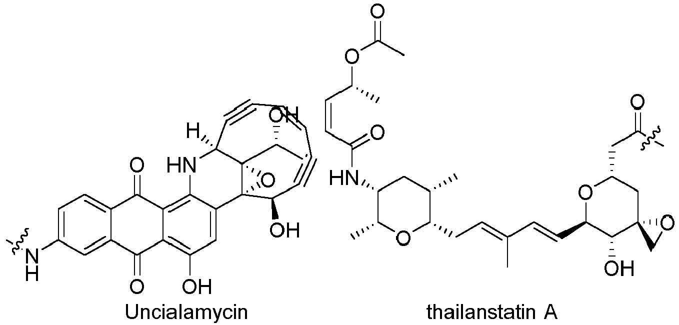 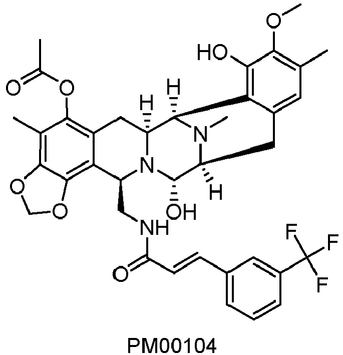 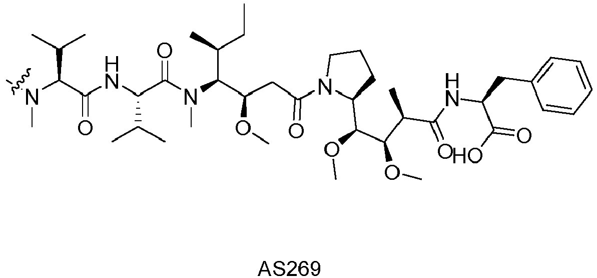 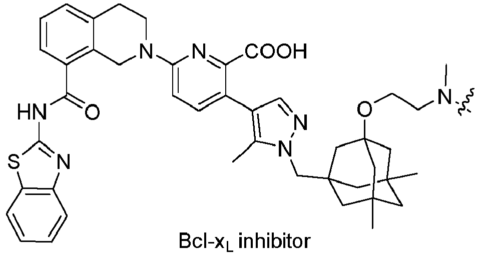 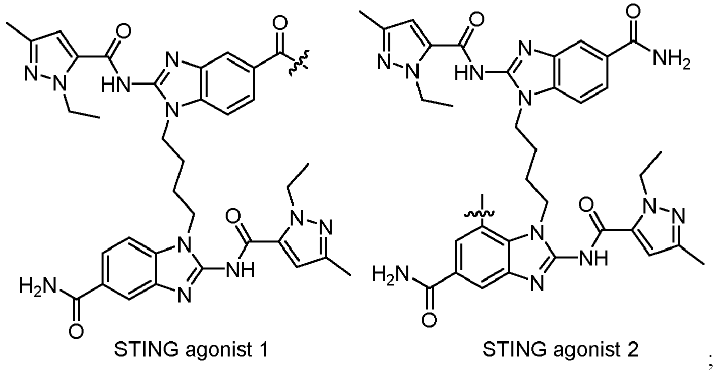 or
moiety C is an auristatin, preferably having a structure according to the following formula: 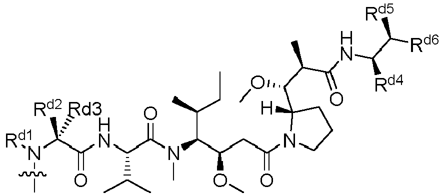 wherein:
R^{1d} is independently H or C₁-C₆ alkyl; preferably H or CH₃;
R^{2d} is independently C₁-C₆ alkyl; preferably CH₃ or iPr;
R^{3d} is independently H or C₁-C₆ alkyl; preferably H or CH₃;
R^{4d} is independently H, C₁-C₆ alkyl, COO(C₁-C₆ alkyl), CON(H or C₁-C₆ alkyl), C₃-C₁₀ aryl or C₃-C₁₀ heteroaryl; preferably H, CH₃, COOH, COOCH₃ or thiazolyl;
R^{5d} is independently H, OH, C₁-C₆ alkyl; preferably H or OH; and
R^{6d} is independently C₃-C₁0 aryl or C₃-C₁0 heteroaryl; preferably optionally substituted phenyl or pyridyl,
wherein preferably, moiety C is derived from MMAE or MMAF;
(f) the immunomodulator agent is selected from molecules known to be able to modulate the immune system, such as ligands of CD3, CD25, TLRs, STING, 4-1BBL, 4-1BB, PD-1, mTor, PDL-1, NKG-2D IMiDs, wherein ligands can be agonists and/or antagonist; or
(g) the protein is selected from cytokines, such as IL2, IL10, IL12, IL15, TNF, Interferon Gamma, or is an antibody.

9. The compound according to any one of the preceding claims, which:
(a) has the structure: 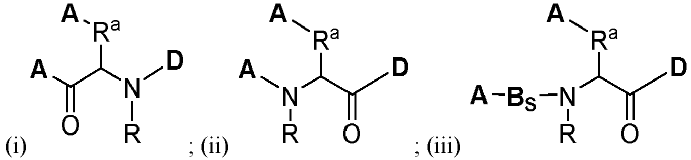 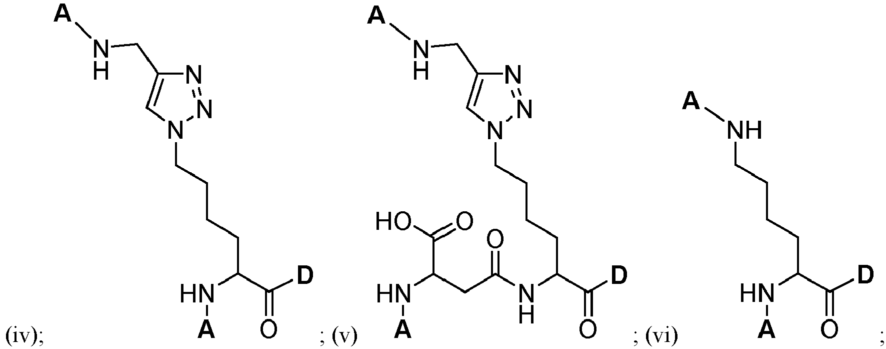 or 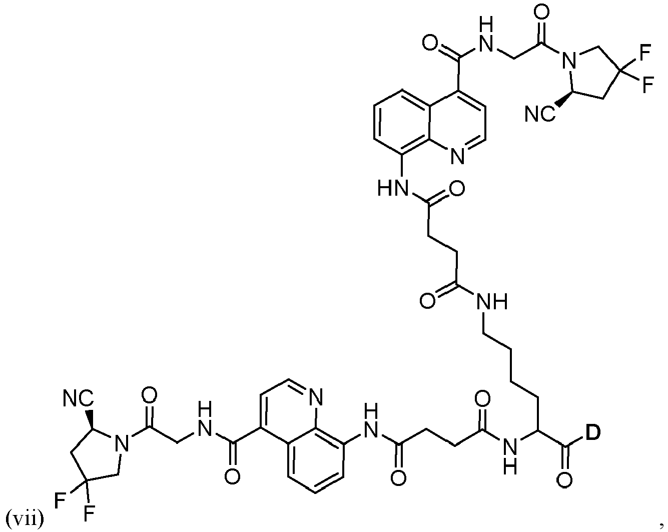 wherein moiety **D** represents **B―C** as defined in any of the preceding claims; or
(b) comprises the structure: 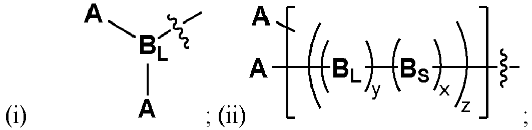 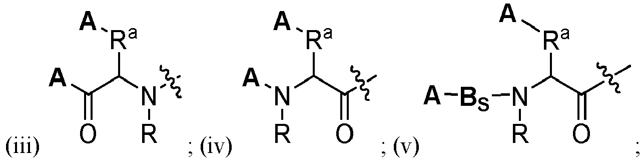 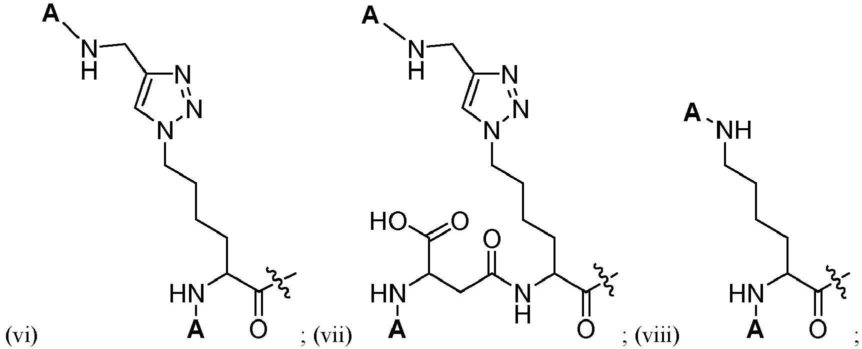 or 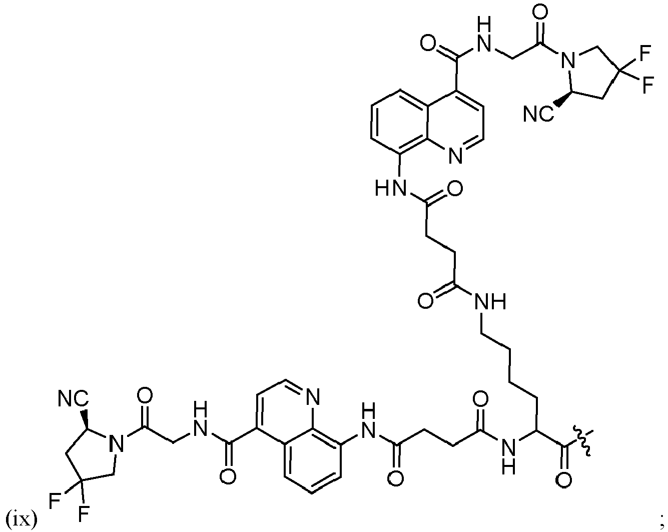 or
(c) has the structure: 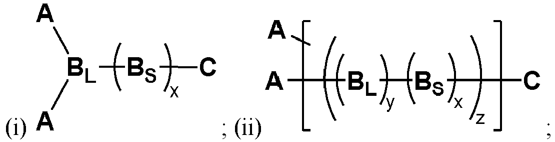 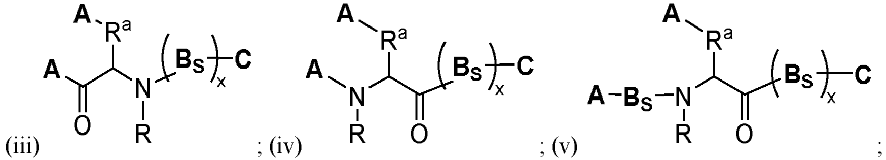 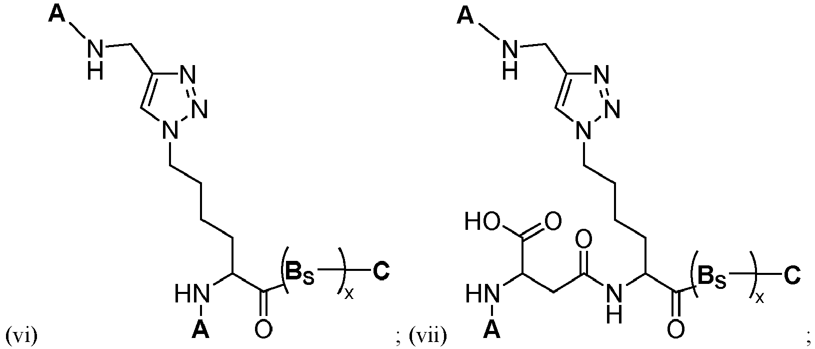 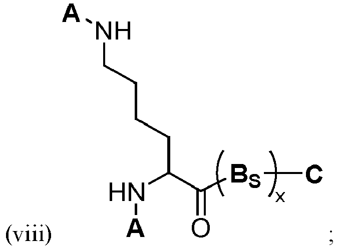 or 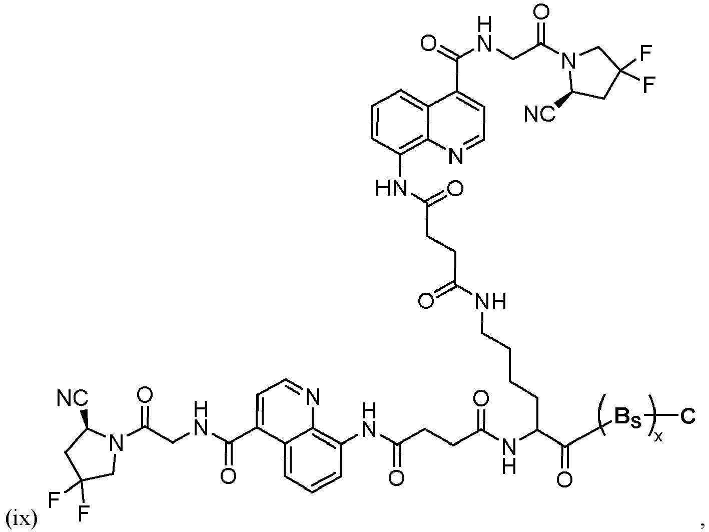 wherein, unless otherwise specified, all groups and variables are defined as in any one of the preceding claims.

10. The compound according to any one of the preceding claims, comprising moiety **D** or **(B_{S})ₓC**, represented by one of the following structures: 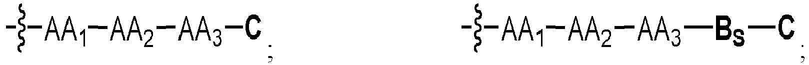 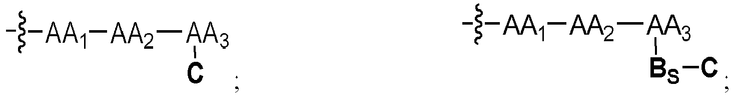 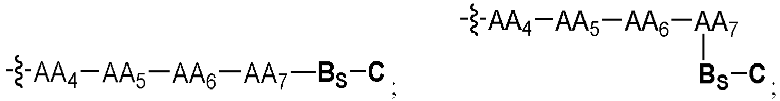 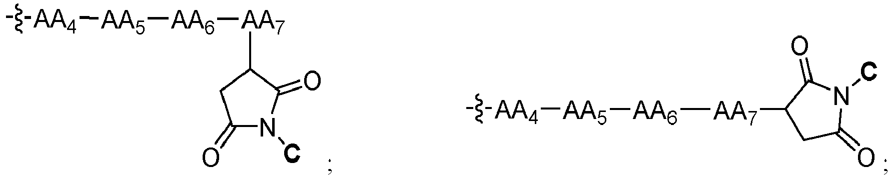 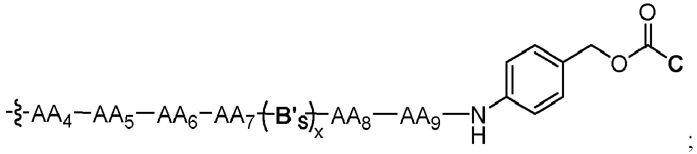 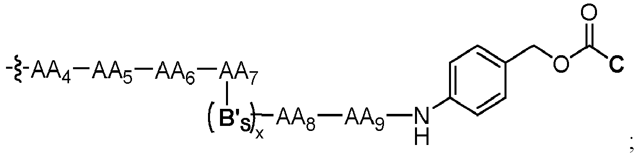 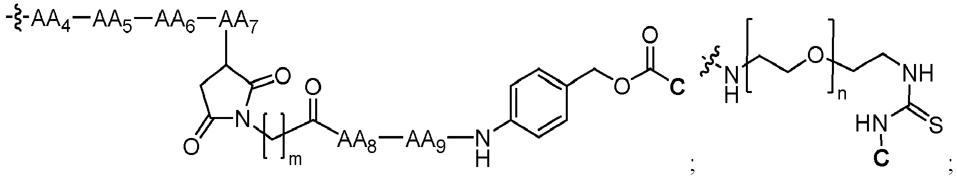 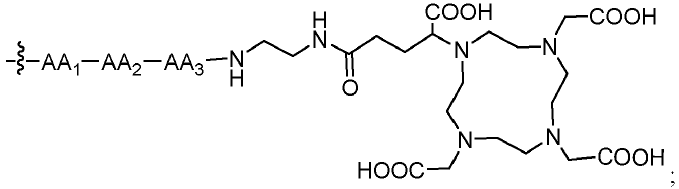 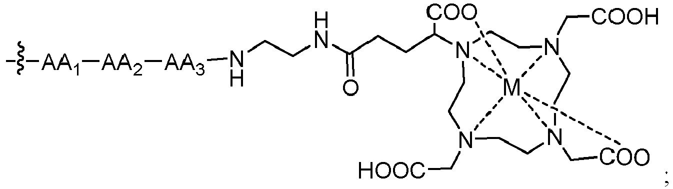 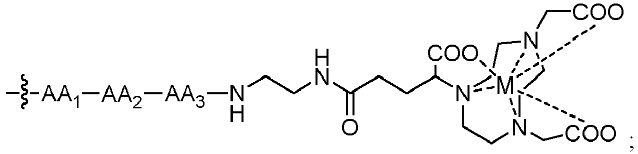 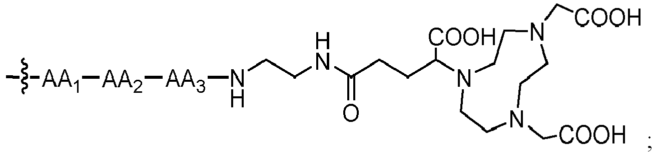 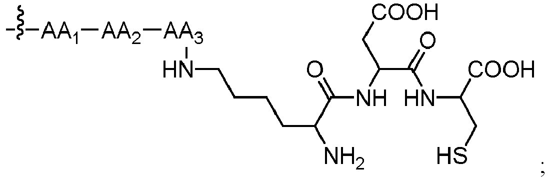 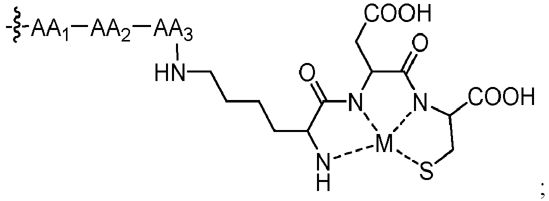 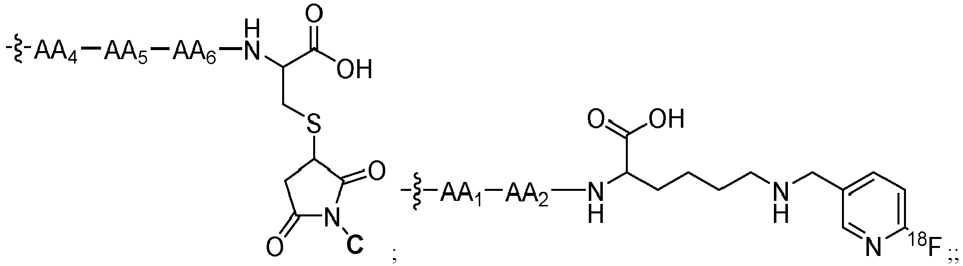 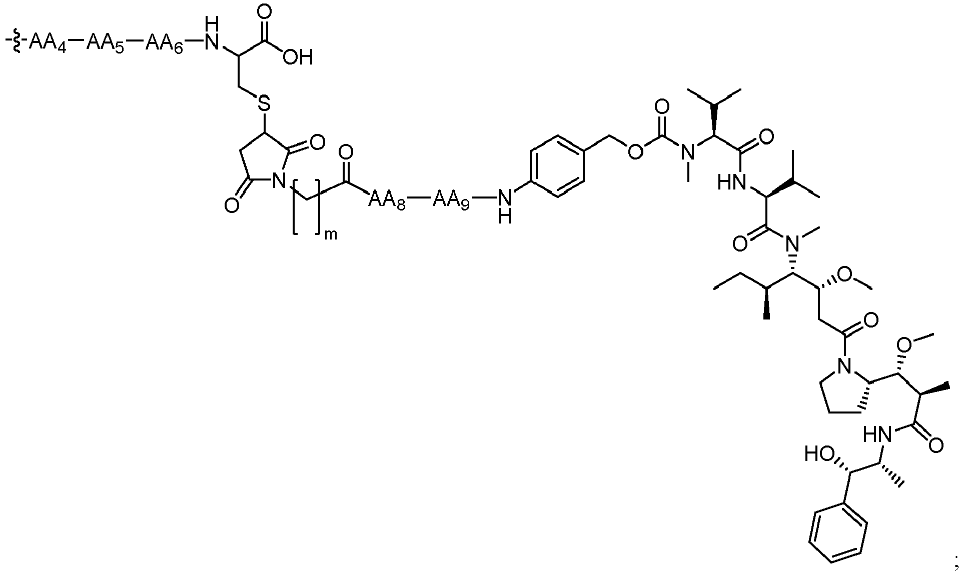 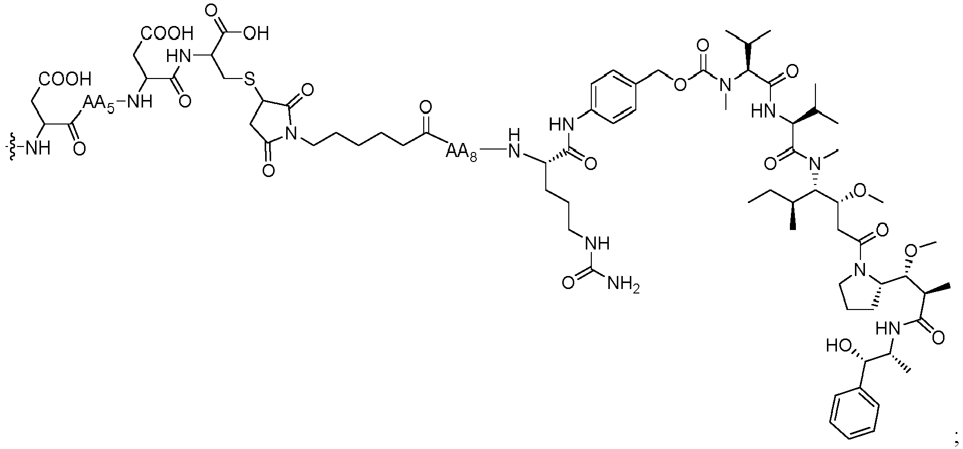 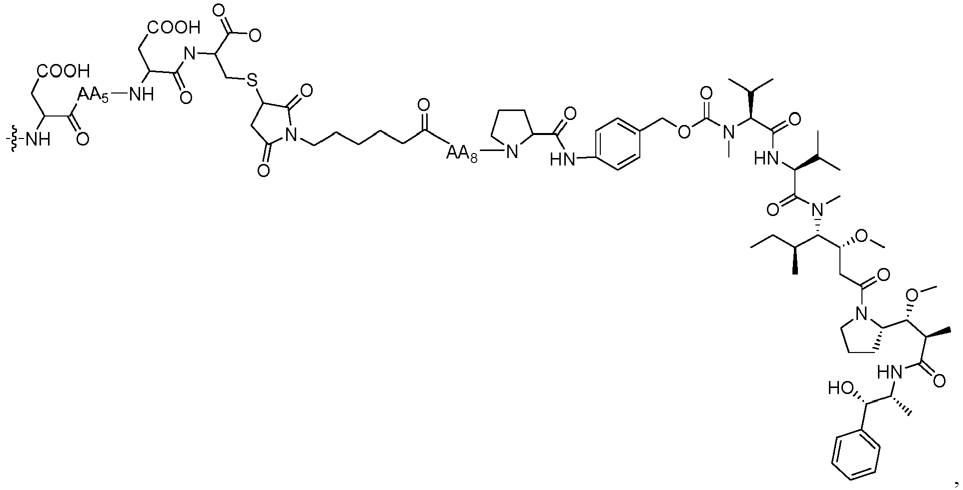
wherein each of AA₁, AA₂, AA₃, AA₄, AA₅, AA₆, AA₇, AA₈, and AA₉ represents a proteinogenic or non-proteinogenic amino acid, or is absent;
preferably wherein: AA₅ is an amino acid with a charged sidechain, and AA₈ is an amino acid with an aliphatic sidechain;
more preferably wherein: AA₁ is selected from Asp and Glu, or is absent; AA₂ is selected from Asp and Glu, or is absent; AA₃ is Lys; AA₄ is selected from Asp and Glu; AA₅ is selected from Lys and Arg; AA6 is selected from Asp and Glu; AA₇ is selected from Cys; and AA₈ is selected from Gly, Ala, and Val; and AA₉ is selected from Pro and citrulline (Cit),
wherein, unless otherwise specified, all groups and variables are defined as in any one of the preceding claims.

11. A compound according to any one of the preceding claims having a structure selected from the conjugates listed in the below table, its individual diastereoisomers, its hydrates, its solvates, its crystal forms, its individual tautomers or a pharmaceutically acceptable salt thereof:
| | |
|---|---|
| **1** | 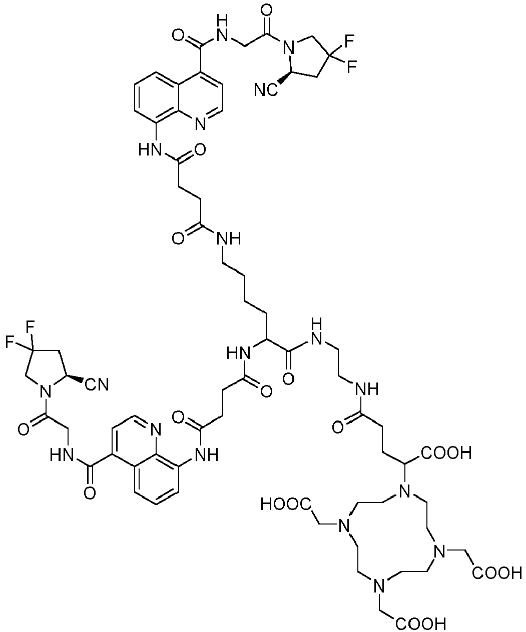 |
| **2** | 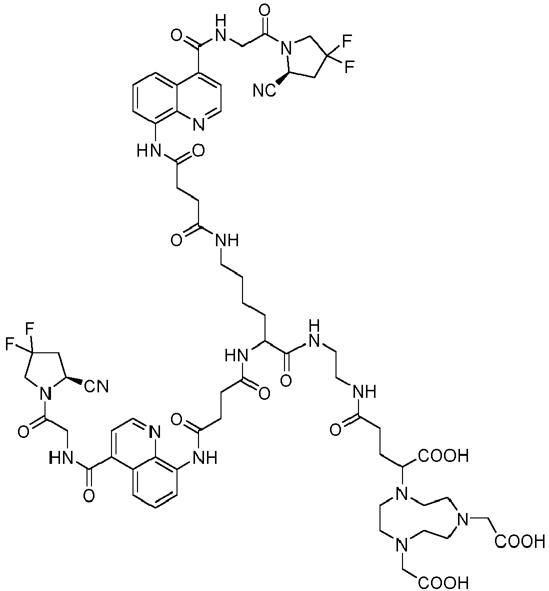 |
| **3** | 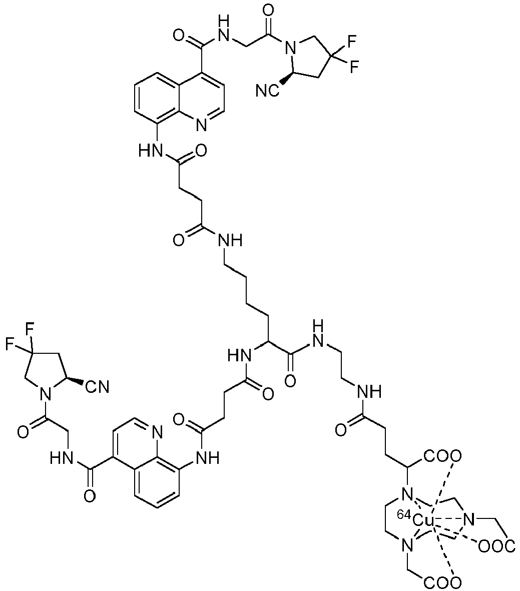 |
| **4** | 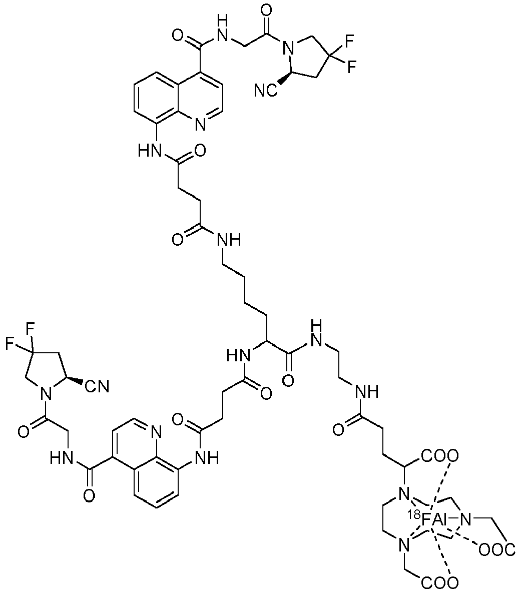 |
| **5** | 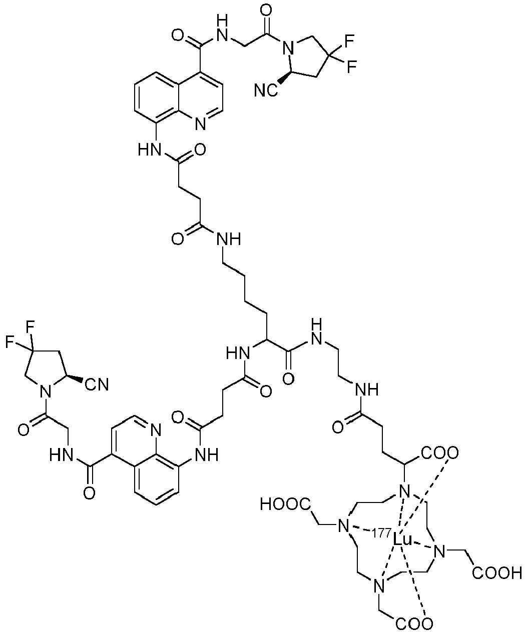 |
| **6** | 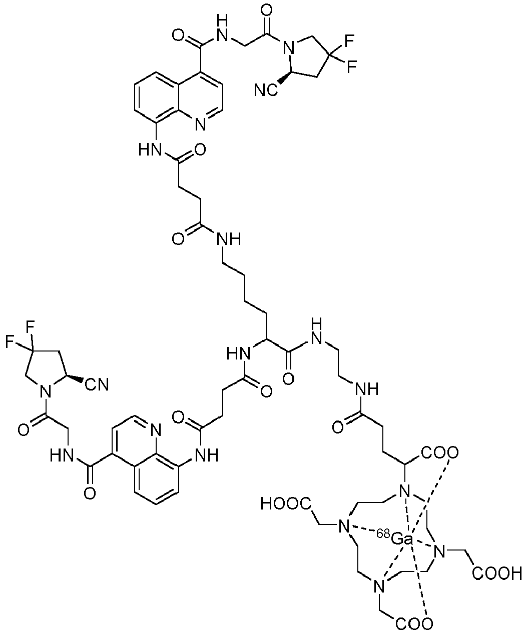 |
| **7** | 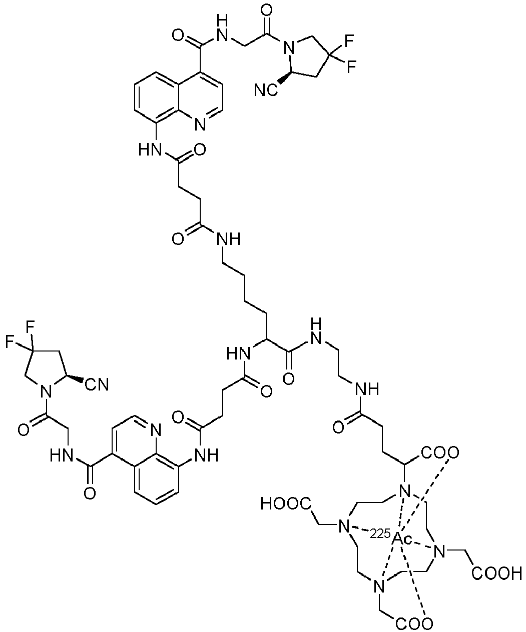 |
| **8** | 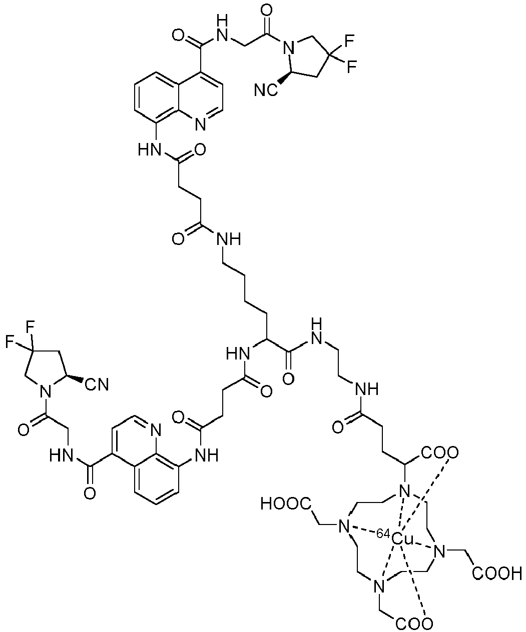 |
| **9** | 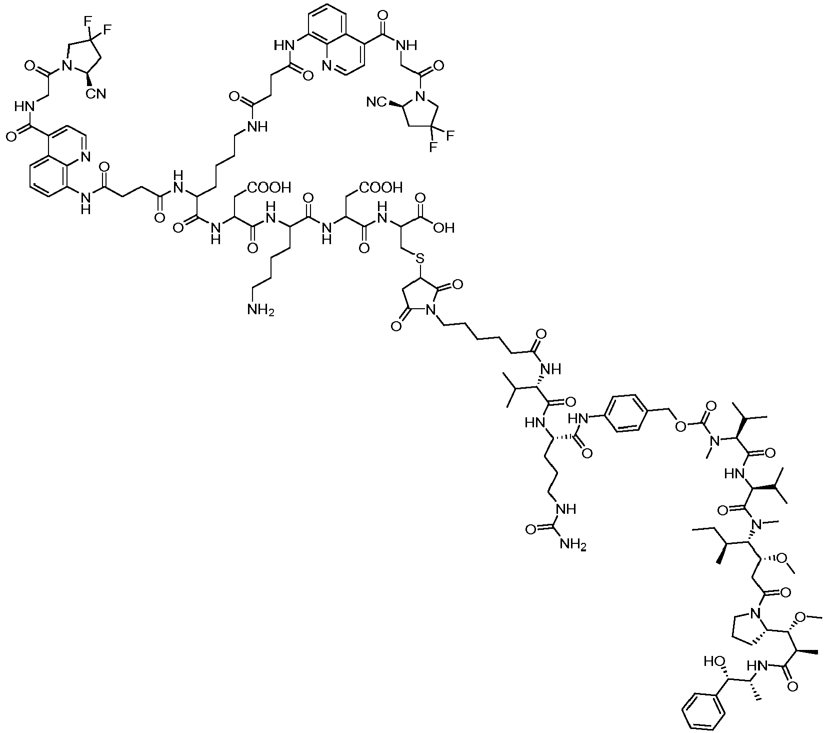 |
| **10** | 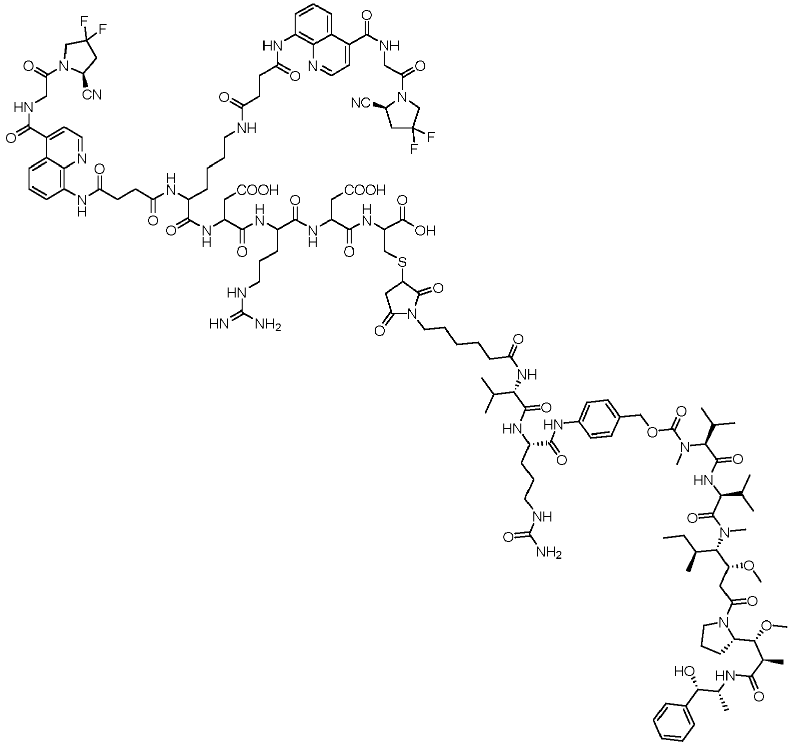 |
| **11** | 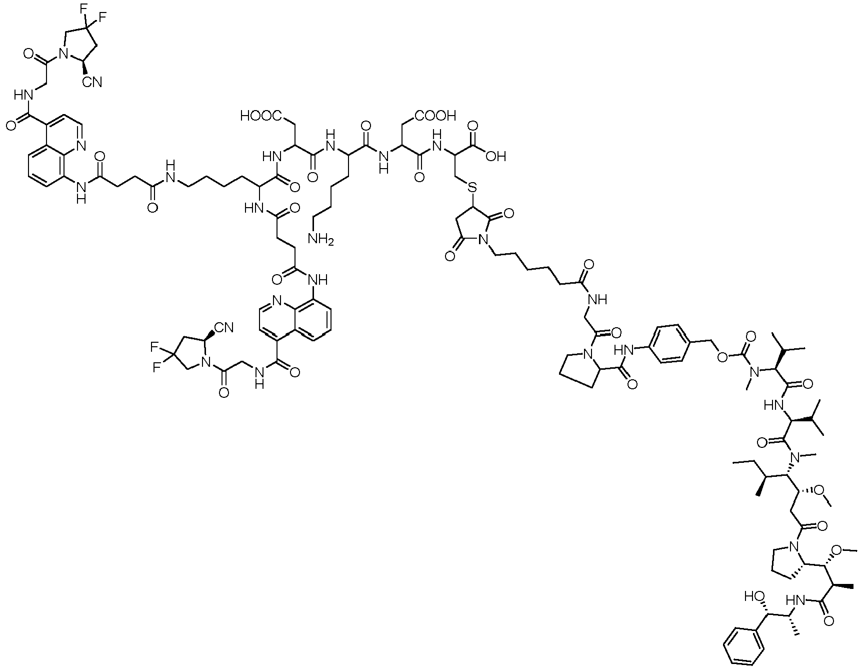 |
| **12** | 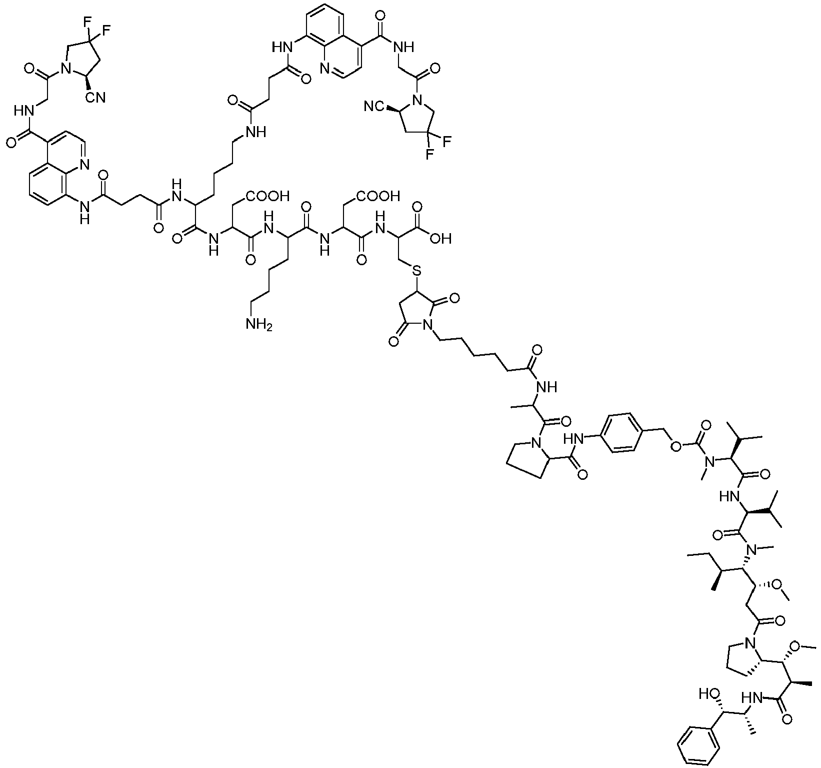 |
| **13** | 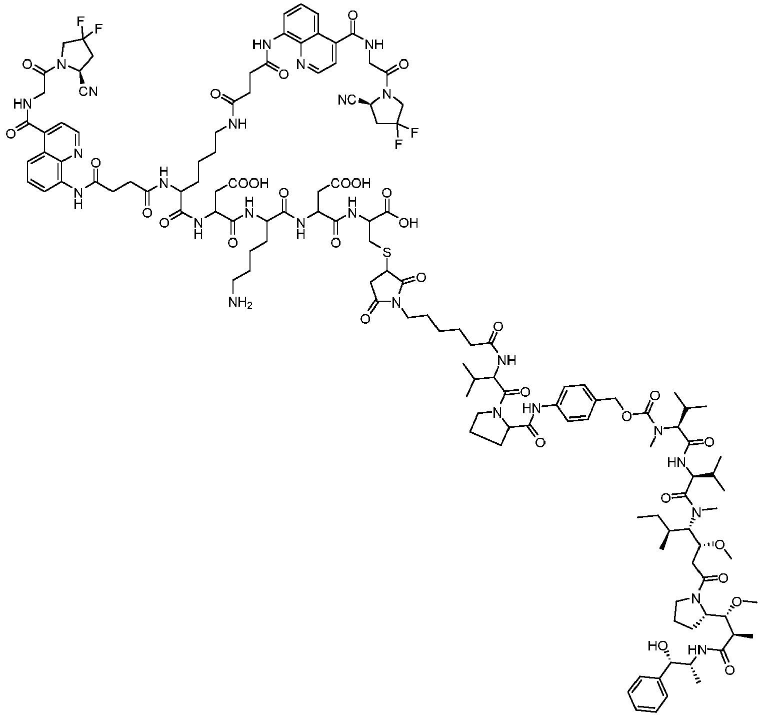 |
| **14** | 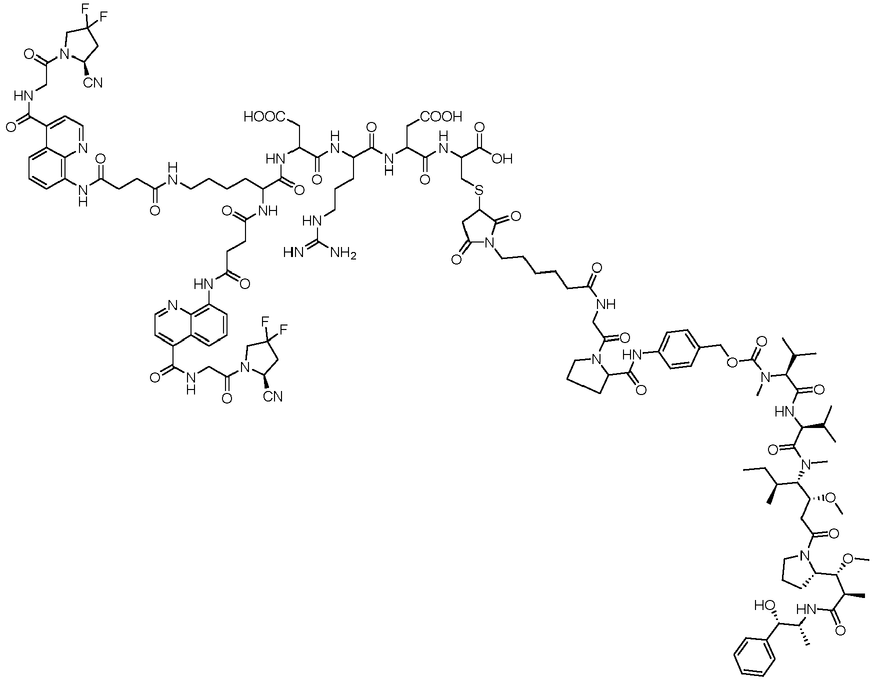 |
| **15** | 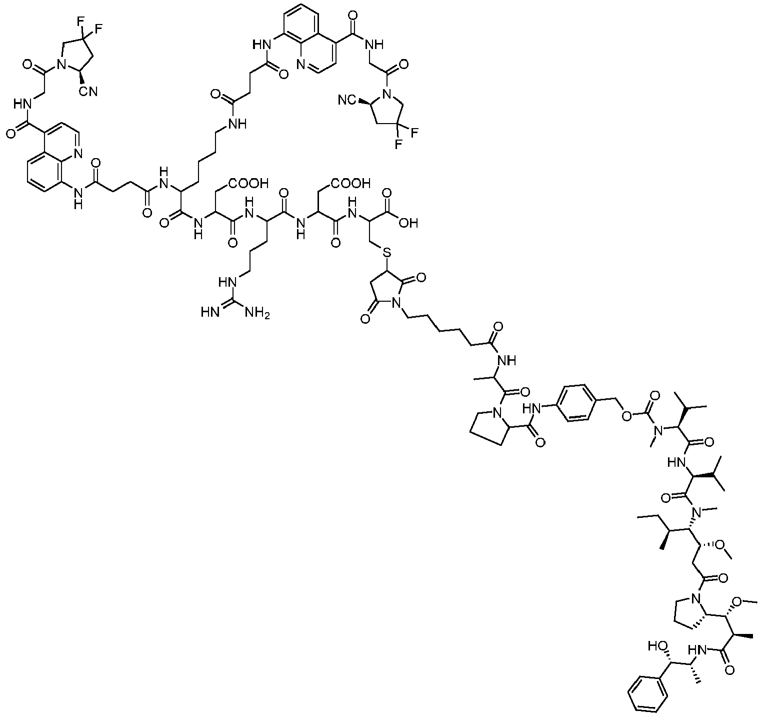 |
| **16** | 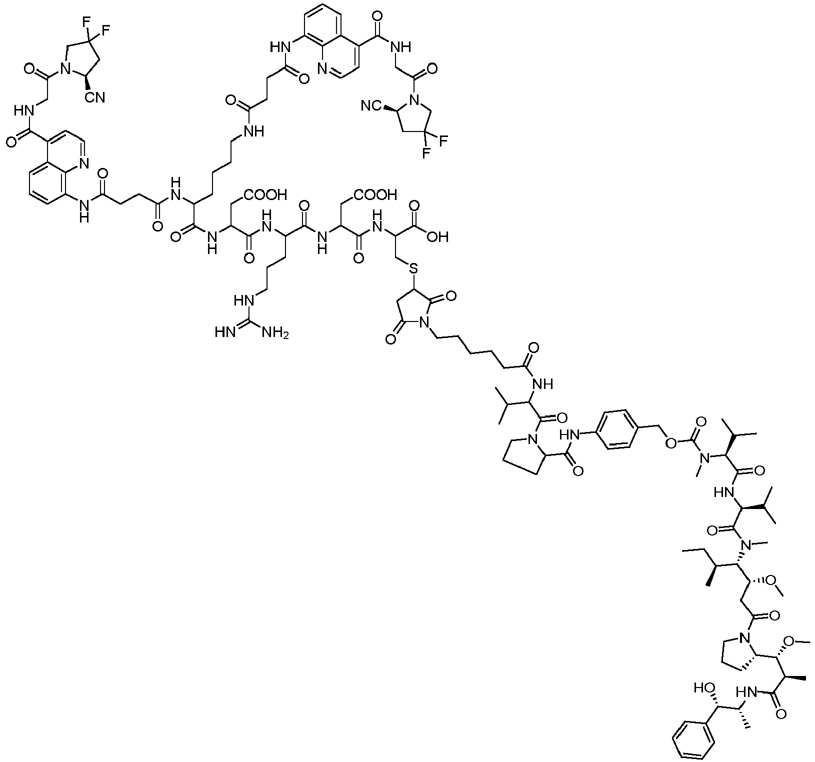 |
| **17** | 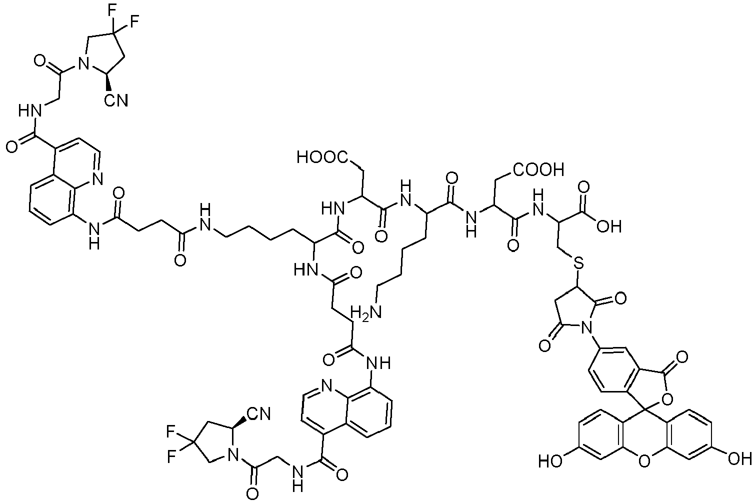 |
| **18** | 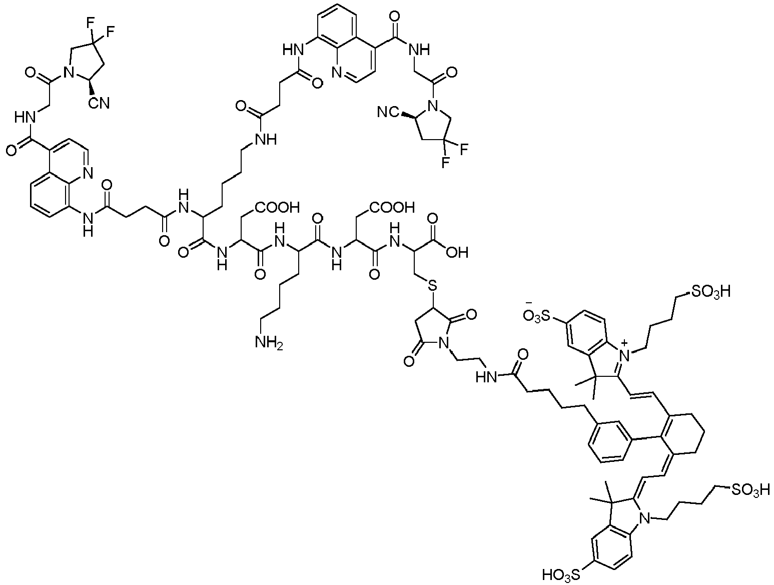 |
| **19** | 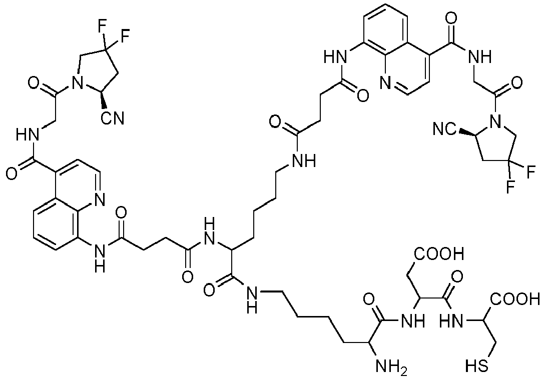 |
| **20** | 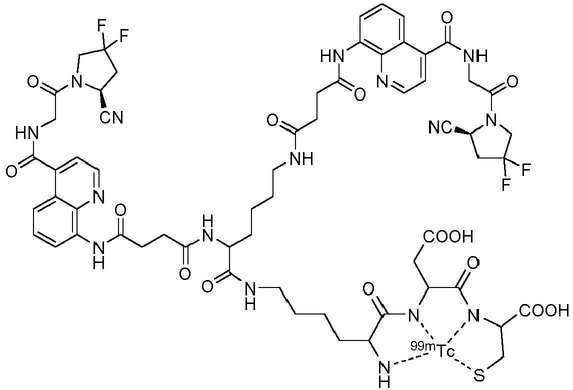 |
| **21** | 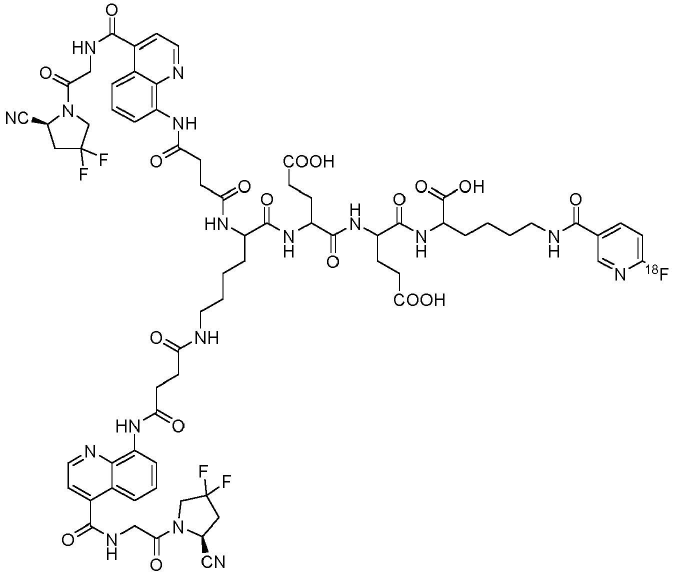 |
| **22** | 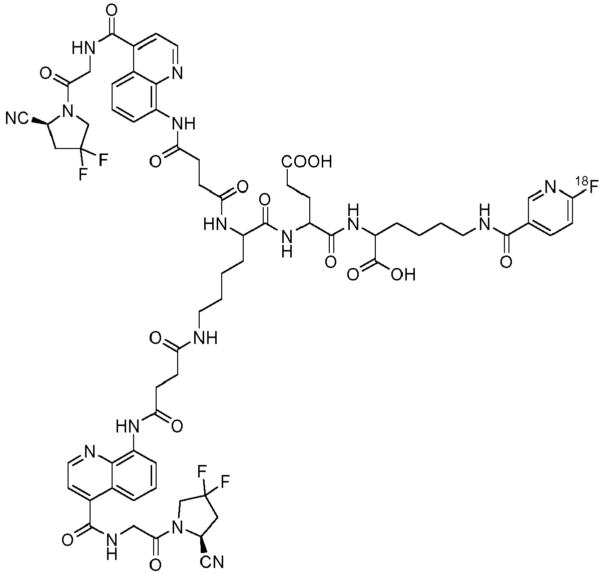 |
| **23** | 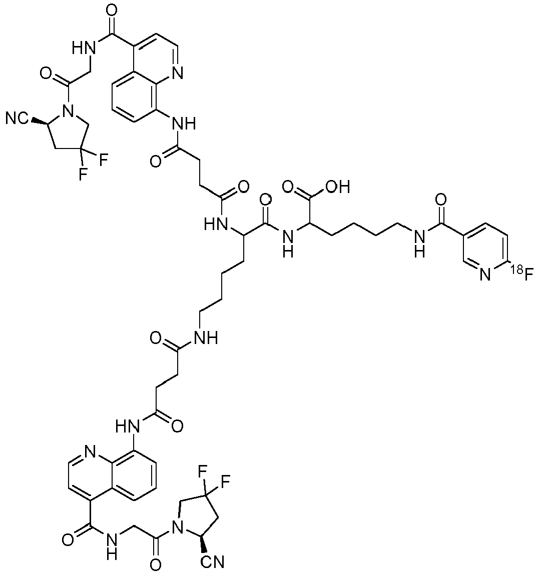 |
| **24** | 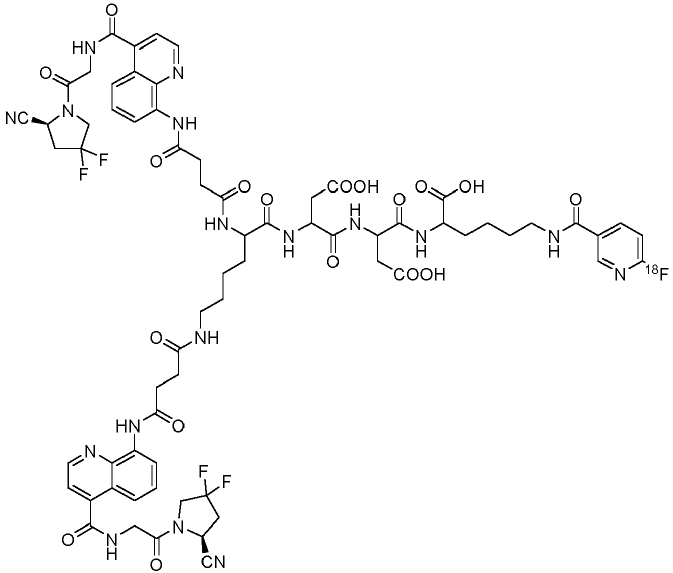 |
| **25** | 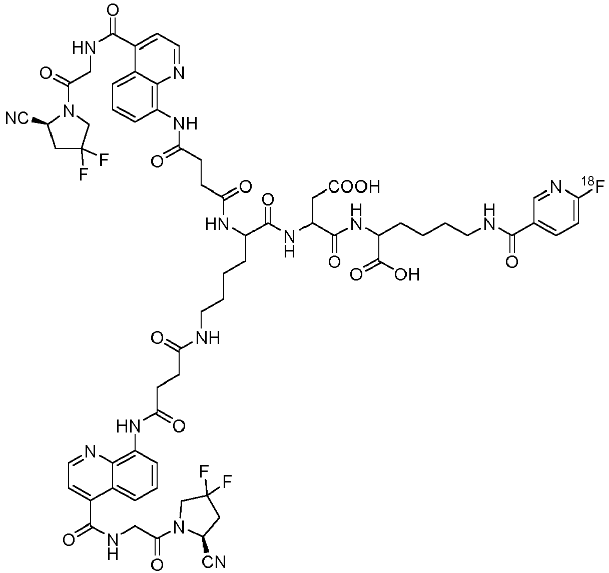 |
| **27** | 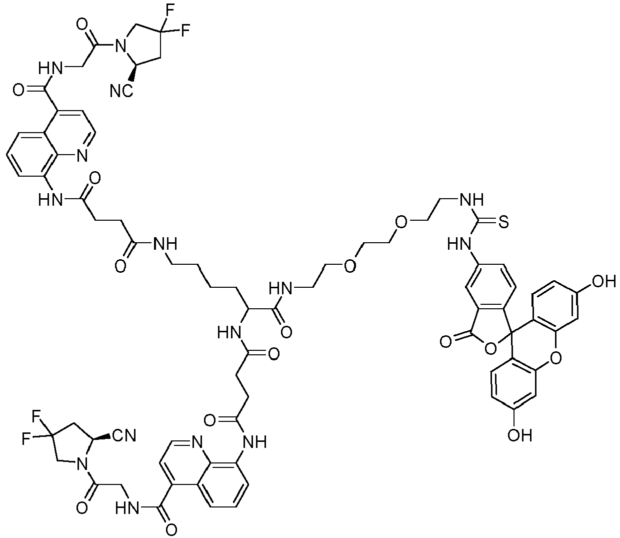 |
| **28** | 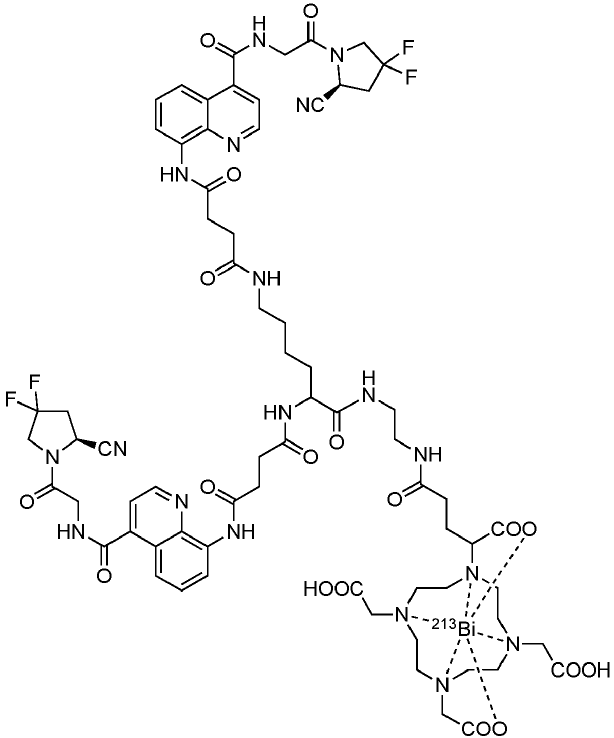 |
| **29** | 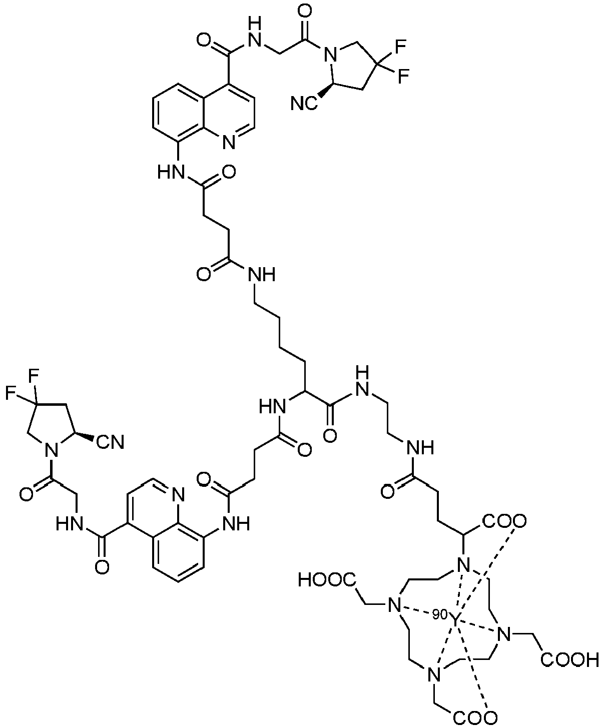 |
| **47** | 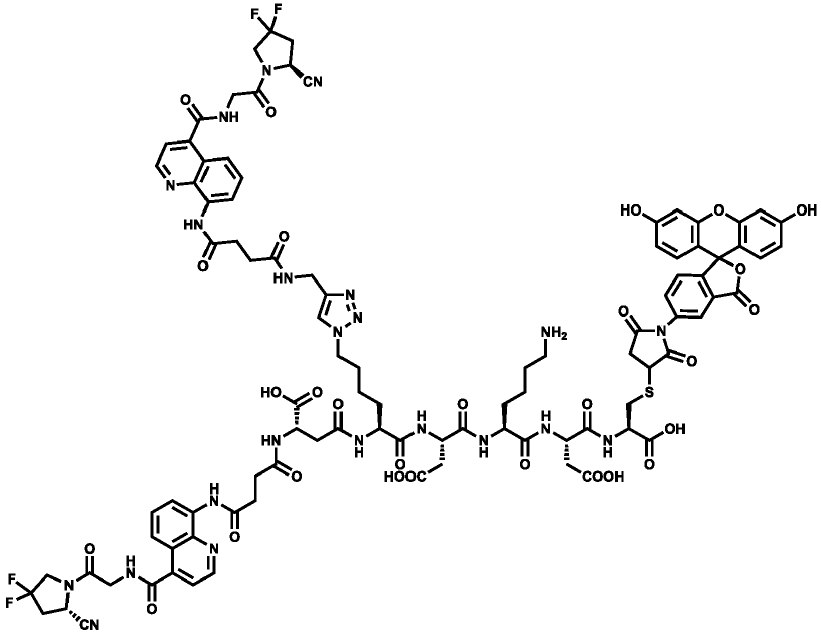 |
| **48** | 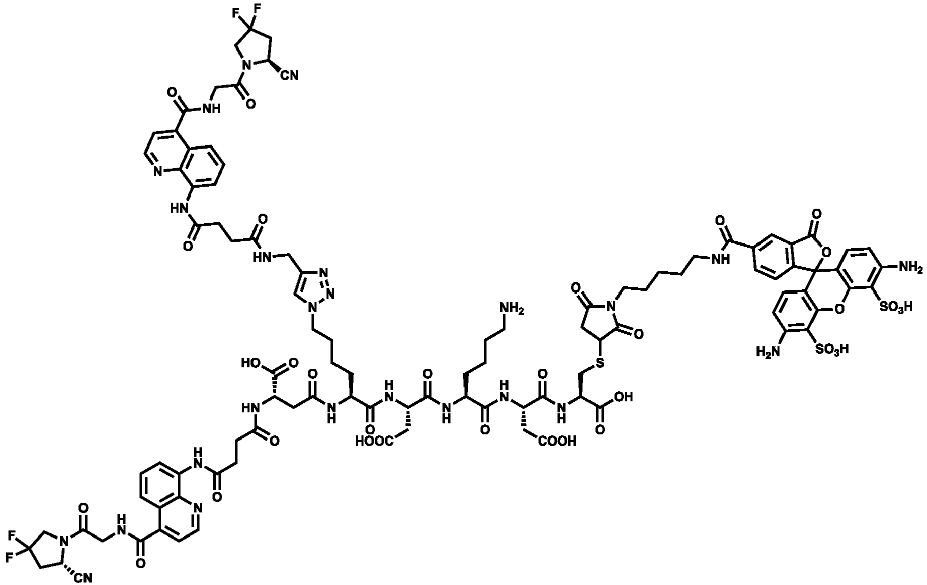 |
| **50** | 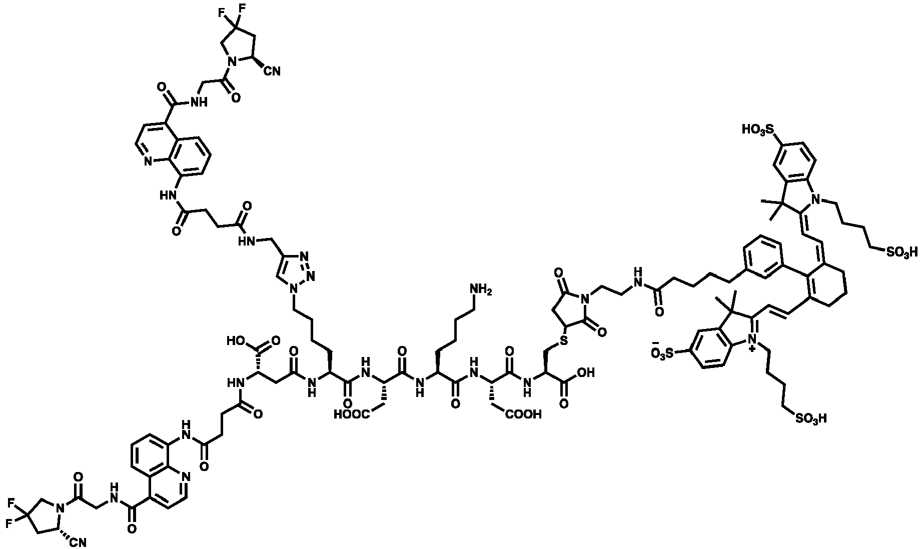 |
| **51** | 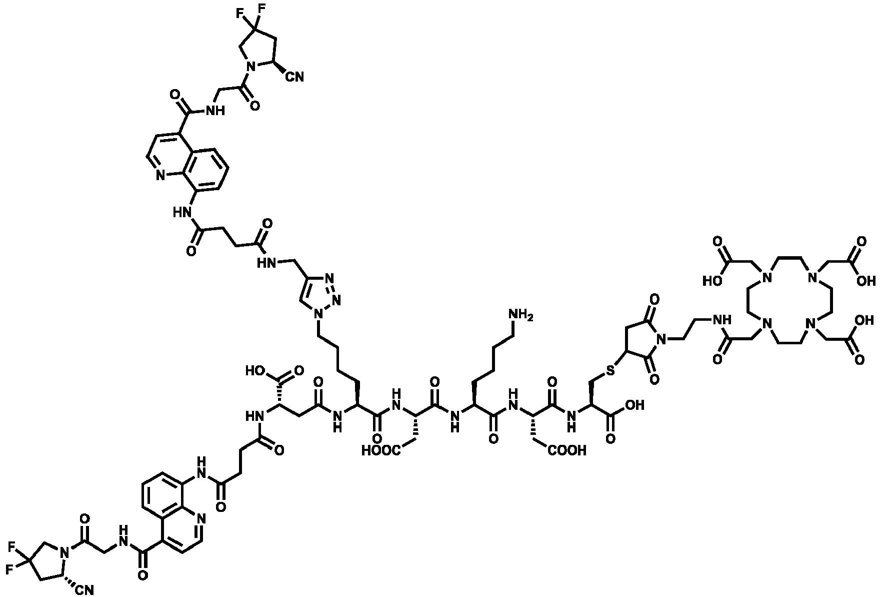 |
| **57** | 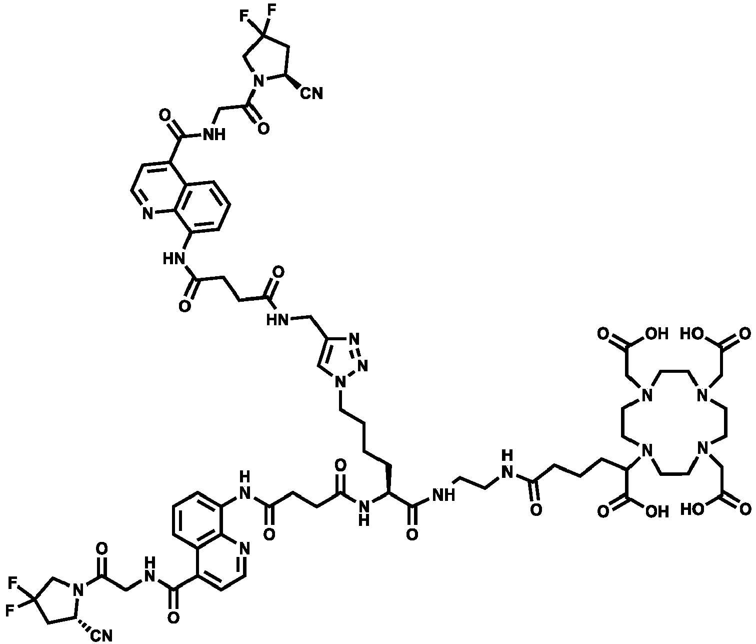 |
| **57'** | 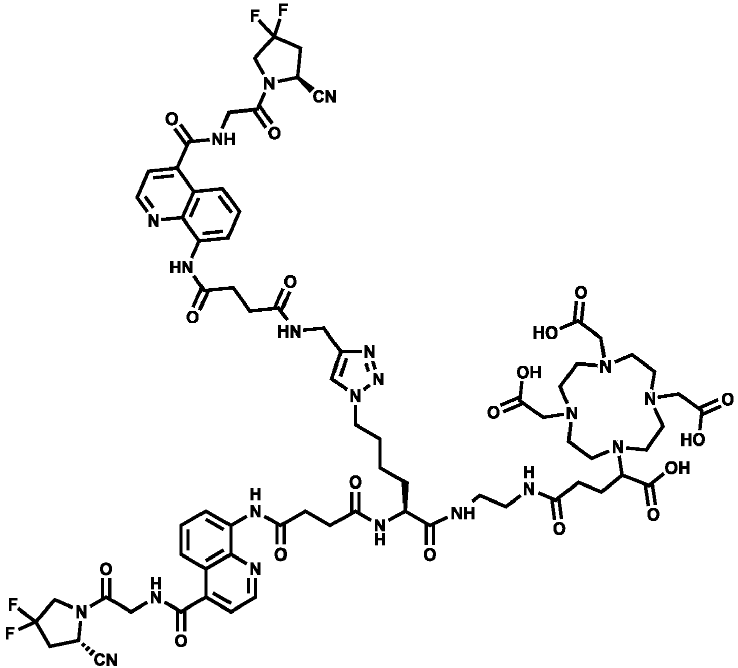 |
| **49** | 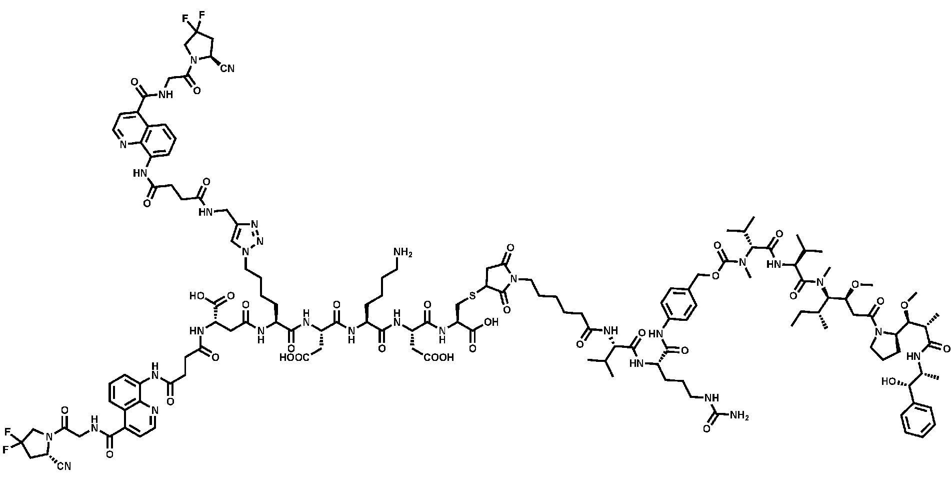 |

12. A pharmaceutical composition comprising the compound according to any one of the preceding claims, and a pharmaceutically acceptable excipient.

13. The compound or the pharmaceutical composition according to any one of the preceding claims for use in:
(a) a method for treatment of the human or animal body by surgery or therapy or a diagnostic method practised on the human or animal body; or
(b) a method for therapy or prophylaxis of a subject suffering from or having risk for a disease or disorder; or
(c) a method for guided surgery practised on a subject suffering from or having risk for a disease or disorder; or
(d) a method for diagnosis of a disease or disorder, the method being practised on the human or animal body and involving a nuclear medicine imaging technique, such as Positron Emission Tomography (PET) or Single Photon Emission Computed Tomography (SPECT); or
(e) a method for targeted delivery of a therapeutic or diagnostic agent to a subject suffering from or having risk for a disease or disorder,
wherein in each of the preceding (b)-(e), said disease or disorder is independently selected from cancer, inflammation, atherosclerosis, fibrosis, tissue remodelling and keloid disorder, preferably wherein the cancer is selected from the group consisting of breast cancer, pancreatic cancer, small intestine cancer, colon cancer, multi-drug resistant colon cancer, rectal cancer, colorectal cancer, metastatic colorectal cancer, lung cancer, non-small cell lung cancer, head and neck cancer, ovarian cancer, hepatocellular cancer, oesophageal cancer, hypopharynx cancer, nasopharynx cancer, larynx cancer, myeloma cells, bladder cancer, cholangiocarcinoma, clear cell renal carcinoma, neuroendocrine tumour, oncogenic osteomalacia, sarcoma, CUP (carcinoma of unknown primary), thymus cancer, desmoid tumours, glioma, astrocytoma, cervix cancer, skin cancer, kidney cancer and prostate cancer;
and wherein in each of the preceding uses or methods, the compound preferably has a prolonged residence at the disease site at a therapeutically or diagnostically relevant level, preferably beyond 1 h, more preferably beyond 6 h post injection.

14. A compound, its individual diastereoisomers, its hydrates, its solvates, its crystal forms, its individual tautomers or a salt thereof, wherein the compound structure comprises:
two moieties A having the following structure: 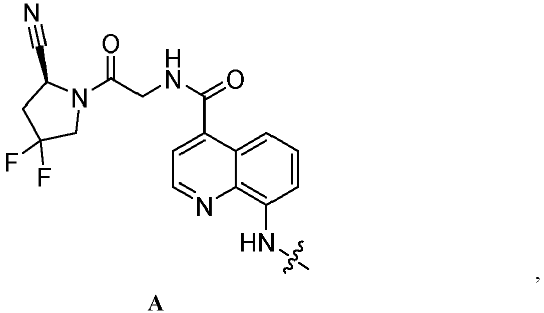
preferably wherein each moiety A has the following structure **A¹** or **A²**, wherein m is 0, 1, 2, 3, 4 or 5: 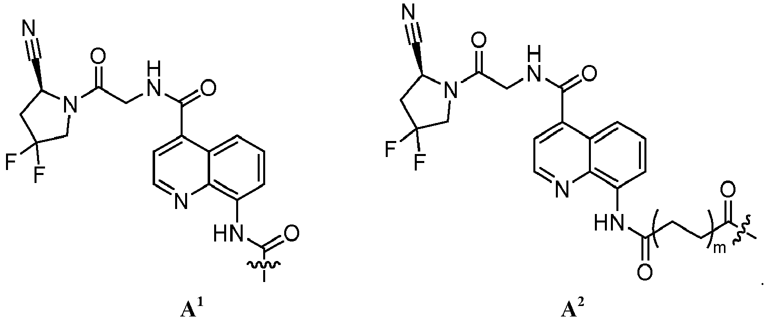
and a reactive moiety **L** capable of reacting and forming a covalent bond with a conjugation partner;
more preferably wherein the compound is represented by the following Formula VI: 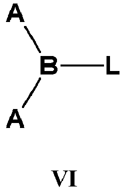
wherein **B** is a covalent bond or a multifunctional moiety covalently attaching the moieties **A** to **L**; and
yet more preferably:
(a) wherein **L** is capable of forming, upon reacting, an amide, ester, carbamate, hydrazone, thiazolidine, methylene alkoxy carbamate, disulphide, alkylene, cycloalkylene, arylalkylene, heteroarylalkylene, heteroalkylene, heterocycloalkylene, alkenylene, cycloalkenylene, arylalkenylene, heteroarylalkenylene, heteroalkenylene, heterocycloalenkylene, alkynylene, heteroalkynylene, arylene, heteroarylene, aminoacyl, oxyalkylene, aminoalkylene, diacid ester, dialkylsiloxane, amide, thioamide, thioether, thioester, ester, carbamate, hydrazone, thiazolidine, methylene alkoxy carbamate, disulfide, vinylene, imine, imidamide, phosphoramide, saccharide, phosphate ester, phosphoramide, carbamate, dipeptide, tripeptide or tetrapeptide linking group; and/or
(b) wherein **B** is as defined in any one of claims 4 to 6; and/or
(c) wherein **L** is selected from: H, NH₂, N₃, COOH, SH, Hal, 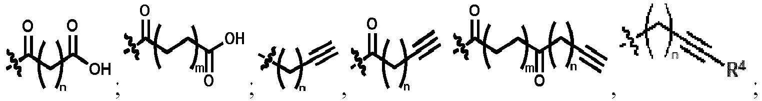 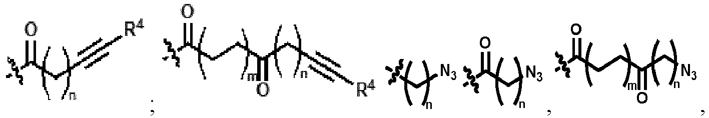 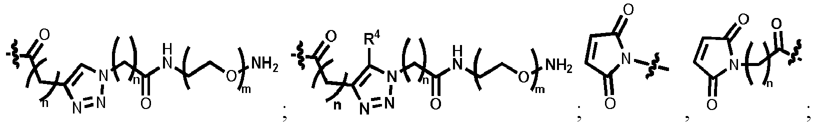 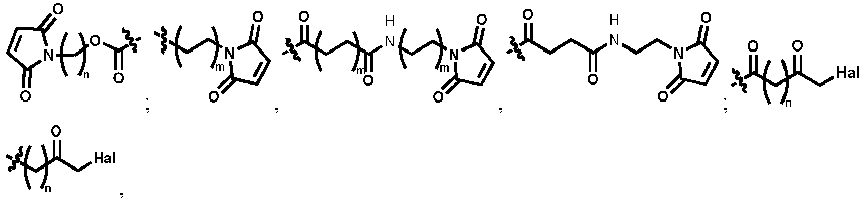 and
wherein each *n* is, independently, 0, 1, 2, 3, 4, 5, 6, 7, 8, 9 or 10;
each m is, independently, 0, 1, 2, 3, 4 or 5;
each Hal is F, Cl, Br or I; and
each R⁴ is, independently selected from carboxy, alkyl, cycloalkyl, aryl and heteroaryl, wherein each of the foregoing is substituted or unsubstituted, halogen, and cyano; and/or
(d) having the structure: 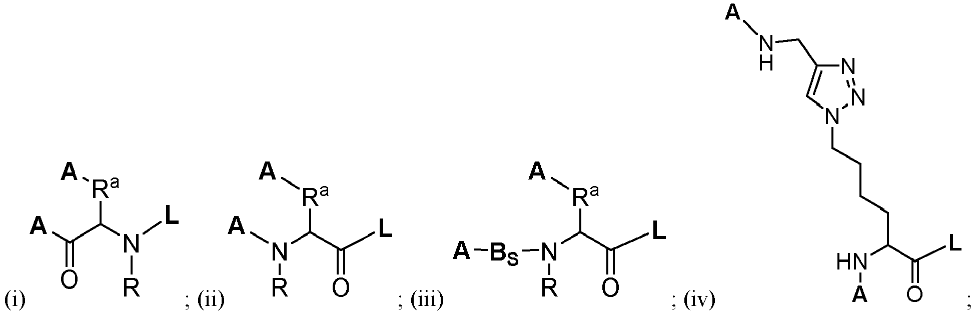 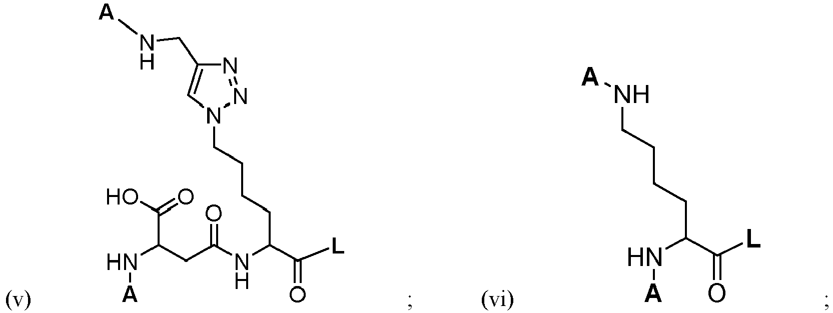 or 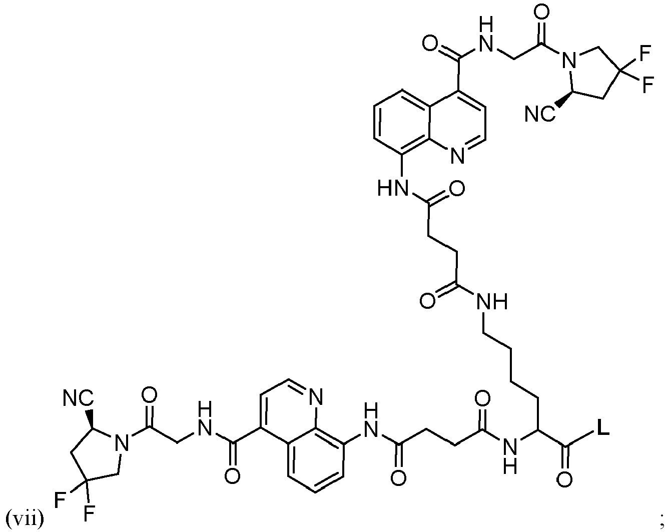 or
(e) which is selected from: 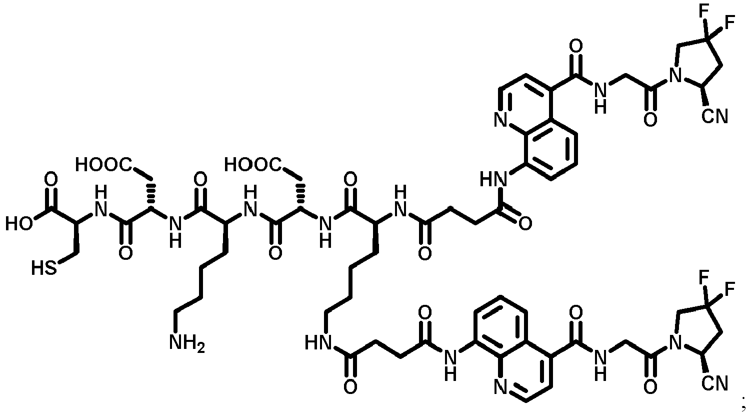 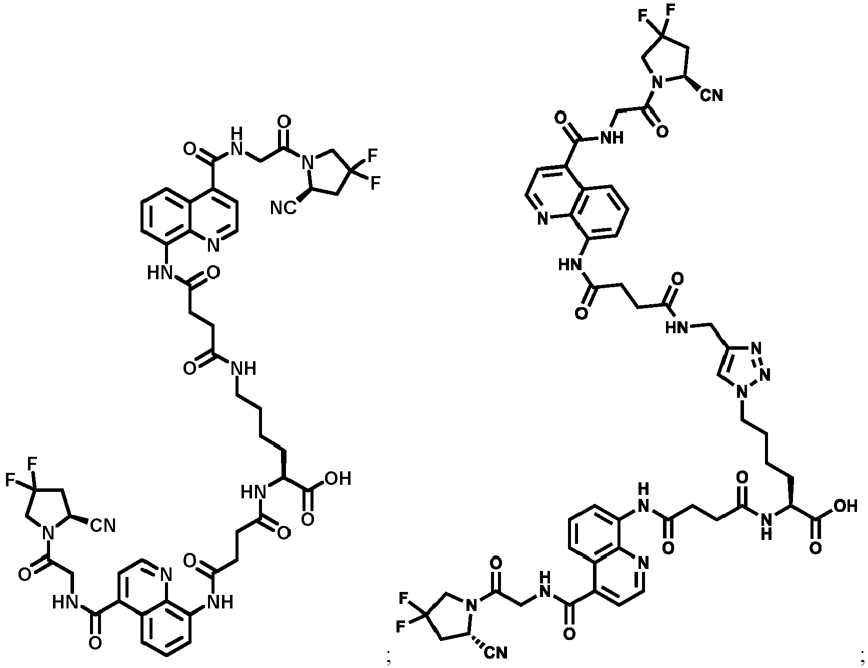 and 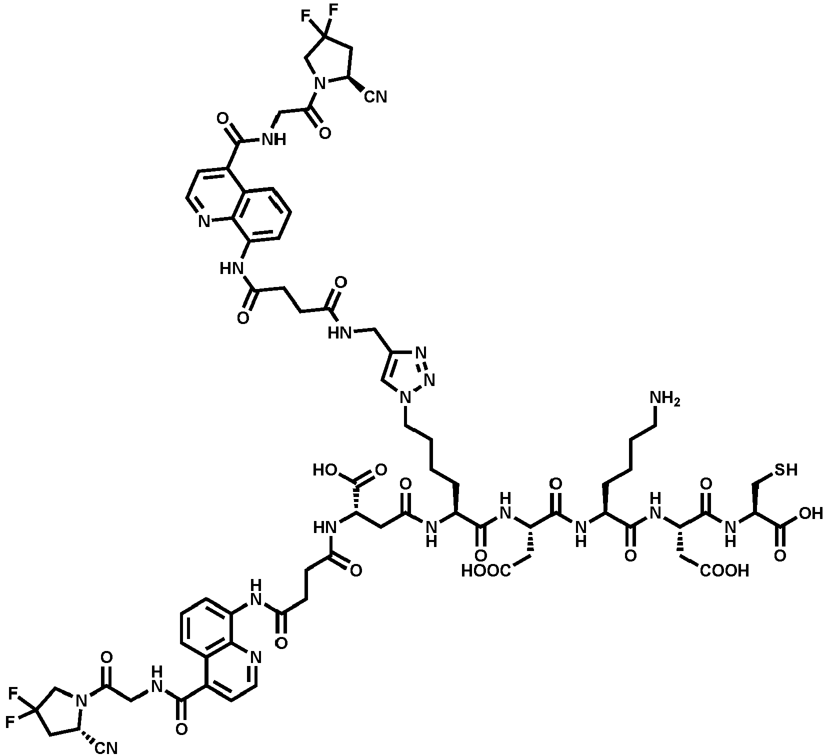 wherein, unless otherwise specified, all groups and variables are defined as in any one of the preceding claims.

15. A method for preparing a conjugate comprising the step of conjugating a compound according to claim 14 with a conjugation partner, wherein preferably:
(a) the compound according to claim 14 is conjugated by reacting with the conjugation partner to form a covalent bond; and/or (b) the conjugate is a compound according to any one of claims 1-11; and/or (c) the conjugation partner is a therapeutic or diagnostic agent; and/or (d) the method further comprises formulating the conjugate as a pharmaceutical composition or as a diagnostic composition.
